# EUROPEAN PATENT APPLICATION

(11) **EP 4 382 661 A1**
(43) Date of publication of application: **12.06.2024**
(21) Application number: 22853057.2
(22) Date of filing: 02.08.2022
(51) Int. Cl.: D06N 3/12, B32B 5/00, C07K 14/435, D06M 15/15

(54) **ARTIFICIAL LEATHER AND METHOD FOR PRODUCING SAME**

(30) Priority: 02.08.2021 JP 2021126890
(71) Applicant: Spiber Inc., Tsuruoka-shi, Yamagata 997-0052 (JP)
(72) Inventor: KAGATA Hideki, Tsuruoka-shi, Yamagata 997-0052 (JP); SAKATA Kazuki, Tsuruoka-shi, Yamagata 997-0052 (JP)
(74) Representative: Handley, Matthew Edward
(86) International application number: PCT/JP2022/029657
(87) International publication number: WO 2023/013638

(57) **Abstract**

The present disclosure relates to a synthetic leather including a base material layer containing a fiber base material, in which at least a part of the synthetic leather contains a polypeptide derivative, the polypeptide derivative contains a block copolymer containing a first segment containing a polypeptide skeleton, and one or a plurality of second segments that are bonded to the first segment, the polypeptide skeleton is a recombinant polypeptide skeleton or a hydrophobic polypeptide skeleton, and the second segment contains a molecular group having a plasticizing function for the polypeptide skeleton.

## Description

### Technical Field

The present disclosure relates to a synthetic leather and a method for producing the same.

### Background Art

Conventionally, a synthetic leather has been used as an alternative to a natural leather. For example, as described in Patent Literature 1, a synthetic leather includes a base fabric composed of a non-woven fabric formed of a synthetic fiber such as polyamide or polyester, and an impregnated body formed of a synthetic resin such as polyurethane impregnated into the base fabric. As the synthetic leather, there is also known a synthetic leather formed by laminating a polyurethane resin or a vinyl chloride resin as a skin layer on a surface of a base fabric such as a woven fabric or a knitted fabric formed of a synthetic fiber such as polyethylene terephthalate (PET) by coating or the like.

Such a synthetic leather can be mass-produced with an artificially stable quality unlike a natural leather having limitations in productivity and quality homogenization, and thus in recent years, the synthetic leather has been used in a wider range of applications such as decorations for clothing, shoes, bags, and the like, and various covers and furniture.

In the conventional synthetic leathers, since all the materials of the synthetic leathers (the base fabric, impregnated body, skin layer, and the like) are often formed of a synthetic resin, hygroscopicity is poor, and moreover, since all the materials are derived from petroleum, the load on the environment may be large.

Under such circumstances, the present applicant proposes, in Patent Literature 2, a synthetic leather including a base material layer containing a fiber base material, in which the fiber base material includes protein fibers and is shrink-proof.

### Citation List

### Patent Literature

Patent Literature 1: JP 10-131059 A
Patent Literature 2: WO 2020/262602 A

### Summary of Invention

### Technical Problem

It has been found that a synthetic leather using a material containing a polypeptide skeleton has an advantageous effect in terms of hygroscopicity, a reduction in environmental load, and the like, but there is still room for improvement in flexibility. An object of the present disclosure is to provide a synthetic leather having excellent flexibility while containing a material containing a polypeptide skeleton, and a method for producing the same.

### Solution to Problem

An aspect of the present disclosure provides a synthetic leather including a base material layer containing a fiber base material, in which at least a part of the synthetic leather contains a polypeptide derivative, the polypeptide derivative contains a block copolymer containing a first segment containing a polypeptide skeleton, and one or a plurality of second segments that are bonded to the first segment, the polypeptide skeleton is a recombinant polypeptide skeleton or a hydrophobic polypeptide skeleton, and the second segment contains a molecular group having a plasticizing function for the polypeptide skeleton.

Since the synthetic leather of the present disclosure contains the polypeptide derivative containing the block copolymer containing the first and second segments, the synthetic leather has excellent flexibility while using a material containing a polypeptide skeleton similarly to proteins. The polypeptide derivative contains a block copolymer in which a first segment and a second segment are directly bonded, and thus, an occurrence of a macro-phase separation as in a case of simply mixing a plasticizer with a protein is reduced.

The first segment may contain a hydrophobic polypeptide skeleton having an average hydropathy index (hydrophobic degree : hydrophobicity index) of 0.22 or more. When the polypeptide skeleton is the hydrophobic polypeptide skeleton as described above, the affinity of the molecular group having a plasticizing function with the first segment is improved, and the flexibility of the synthetic leather is further improved.

A molecular weight of the molecular group having the plasticizing function for the polypeptide skeleton may be 10 or more or 100 or less when a molecular weight of the polypeptide skeleton is 100.

A total molecular weight of the second segment may be 10 or more or 2,000 or less based on a molecular weight of the first segment of 100.

The molecular group having the plasticizing function for the polypeptide skeleton may be polyether, polyester, or polycarbonate.

The polyether may be polyethylene glycol (PEG) or polytetramethylene glycol (PTMG).

The polypeptide skeleton may be a hydrophobic recombinant polypeptide skeleton.

The polypeptide skeleton may include a skeleton derived from a recombinant protein.

The recombinant protein may include a recombinant structural protein.

The recombinant structural protein may include modified fibroin.

The modified fibroin may include modified spider silk fibroin.

The synthetic leather may include at least one of a skin layer bonded to the base material layer, an adhesive layer bonded to the base material layer and the skin layer, and a polymeric substance contained in the base material layer and impregnated into the fiber base material, and at least one of the fiber base material, the skin layer, the adhesive layer, and the polymeric substance may contain a polypeptide derivative.

The synthetic leather may include a porous layer. The porous layer may contain a polypeptide skeleton. In the synthetic leather, the base material layer may be a porous layer.

A molecular weight of the second segment may be a value within a range of 1 to 400 when a molecular weight of the first segment is 100. Thus, in the synthetic leather containing a polypeptide derivative, the flexibility of the entire synthetic leather can be advantageously enhanced while maintaining the properties (for example, high mechanical strength) of the first segment.

The second segment may contain a skeleton derived from at least one selected from the group consisting of polyether, polyester, polycarbonate, polyamide, polyol, polyolefin, polyacetal, polyketal, poly(meth)acrylate, silicone, polyurethane, polyalkyleneimine, a phenolic resin, a urea resin, a melamine resin, and polysaccharides.

The second segment may include at least one selected from the group consisting of a polyether group, a polyester group, a polycarbonate group, a polyamide group, a polyol group, and a modified polysaccharide group.

A plurality of first segments may be contained, and some of the second segments may be bonded to two or more first segments to form a network structure. The network structure is formed, such that toughness of the synthetic leather can be further improved.

The plurality of first segments may be contained, and the first segment and the second segment may be alternately bonded. The first segment and the second segment are alternately bonded, such that the toughness of the synthetic leather can be further improved.

The second segment may contain a plurality of molecular groups, and the plurality of molecular groups may be linked to each other. A designed width of the second segment can be further expanded, and the flexibility of the synthetic leather can be more easily adjusted.

The second segment may further contain a linker, and the molecular group and the polypeptide skeleton may be bonded via the linker.

The linker may include at least one selected from the group consisting of a structural unit represented by the following Formula (1), structural units represented by the following General Formulas (2a) to (6), a structural unit represented by the following Formula (7), structural units represented by the following General Formulas (8a) to (9), structural units represented by the following General Formulas (10) to (11b), and structural units represented by the following General Formulas (13) to (16).

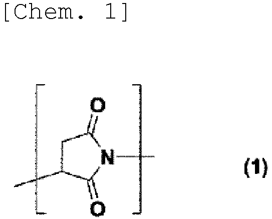

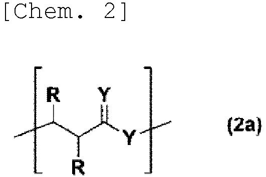

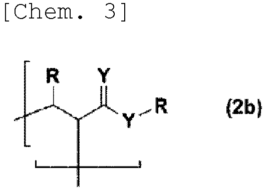

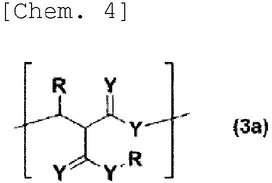

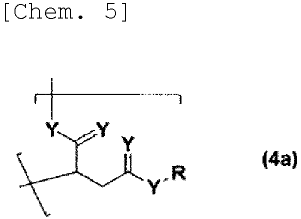

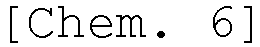

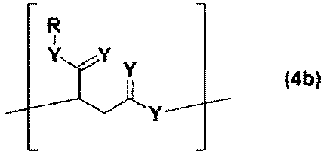

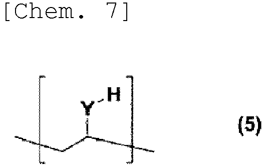

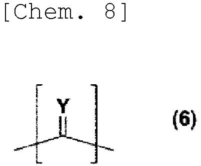

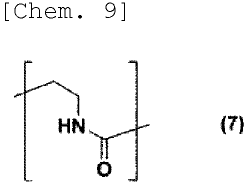

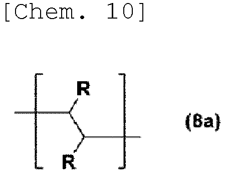

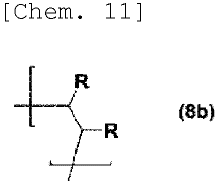

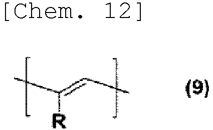

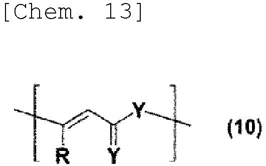

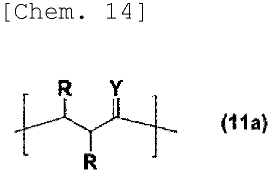

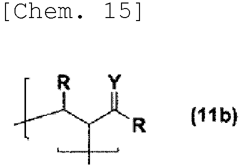

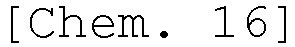

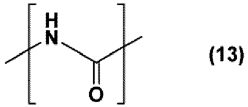

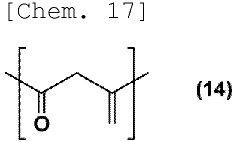

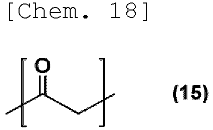

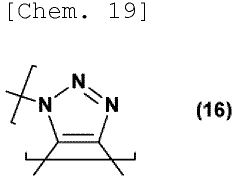

In General Formulas (2a), (2b), (3a), (4a), (4b), (5), (6), (10), (11a), and (11b), Y's each independently represent an oxygen atom, a sulfur atom, or NR¹, and R¹ represents hydrogen, a hydrocarbon group, an aromatic group, a carbonyl group, or a sulfonyl group, and in General Formulas (2a), (2b), (3a), (4a), (4b), (8a), (8b), (9), (10), (11a), and (11b), R's each independently represent hydrogen, a hydrocarbon group, or an aromatic group.

The linker may be at least one selected from the group consisting of a structural unit represented by Formula (1) and structural units represented by General Formulas (2a), (3a), (4a), (4b), and (10).

The first segment may be bonded to the second segment by at least one functional group selected from the group consisting of a thiol group, an amino group, and a hydroxy group in the polypeptide skeleton.

Another aspect of the present disclosure provides a method for producing a synthetic leather including a base material layer containing a fiber base material. The method includes (1) a step of forming a skin layer on the base material layer with an adhesive layer interposed therebetween, in which at least one of the base material layer, the adhesive layer, and the skin layer contains a polypeptide derivative; (2) a step of forming a skin layer on the base material layer without interposing an adhesive layer, in which at least one of the base material layer and the skin layer contains a polypeptide derivative; or (3) a step of forming a base material layer containing a fiber base material and a polymeric substance impregnated into the fiber base material by impregnating the fiber base material with an impregnation solution containing a polymeric substance and then solidifying the polymeric substance, in which at least one of the fiber base material and the polymeric substance contains a polypeptide derivative. In the method, the polypeptide derivative contains a block copolymer containing a first segment containing a polypeptide skeleton, and one or a plurality of second segments that are bonded to the first segment, the polypeptide skeleton is a recombinant polypeptide skeleton or a hydrophobic polypeptide skeleton, and the second segment contains a molecular group having a plasticizing function for the polypeptide skeleton.

The method for producing a synthetic leather may include a step of obtaining a polypeptide derivative by reacting a compound containing a polypeptide skeleton with a compound represented by any one of the following General Formulas (1A) to (16A). In the step of obtaining the polypeptide derivative, the polypeptide skeleton may include at least one selected from the group consisting of a thiol group, an amino group, a hydroxy group, a guanidino group, a carboxy group, a phenoxy group, an indole group, an amide group, an azide group, and an alkynyl group. 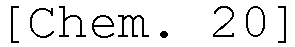 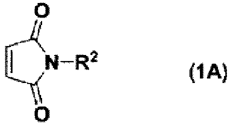 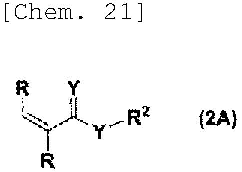 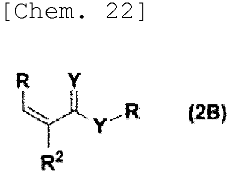 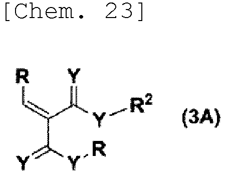 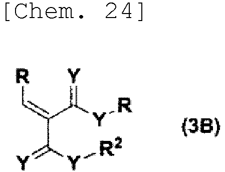 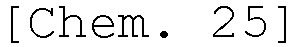 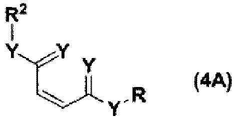 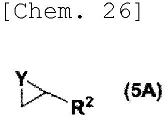 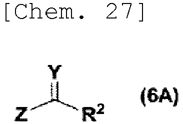 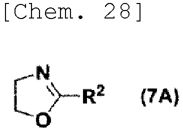 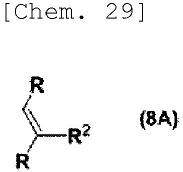 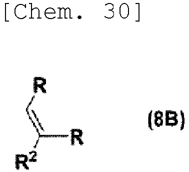 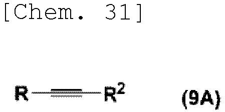 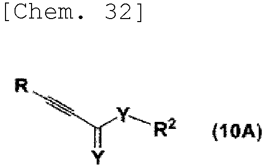 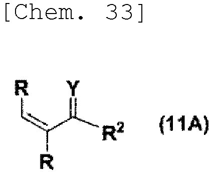 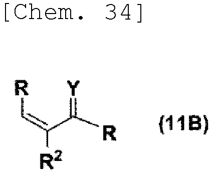 [Chem. 35]

**Z-R²** **(12A)**

[Chem. 36]

**OCN-R²** **(13A)**

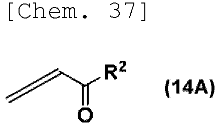

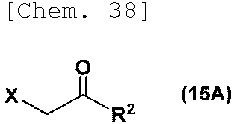

[Chem. 39]

**N₃-R²** **(16A)**

In General Formulas (1A), (2A), (2B), (3A), (3B), (4A), (5A), (6A), (7A), (8A), (8B), (9A), (10A), (11A), (11B), and (12A), R² represents a molecular group having a plasticizing function for the polypeptide skeleton, in General Formulas (2A), (2B), (3A), (3B), (4A), (5A), (6A), (8B), (9A), (10A), (11A), and (11B), Y's each independently represent an oxygen atom, a sulfur atom, or NR¹, and R¹ represents hydrogen, a hydrocarbon group, an aromatic group, a carbonyl group, or a sulfonyl group, in General Formulas (2A), (2B), (3A), (3B), (4A), (5A), (8A), (8B), (9A), (10A), (11A), and (11B), R's each independently represent hydrogen, a hydrocarbon group, or an aromatic group, in General Formulas (6A) and (12A), Z represents a halogen atom, a sulfonic acid ester group, or a fluorine-containing carboxylic acid ester group, and in General Formula (15A), X represents a halogen atom.

In the step of obtaining the polypeptide derivative, a polypeptide derivative is prepared by reacting a compound containing a polypeptide skeleton containing a specific functional group with a specific compound containing a molecular group having a plasticizing function for the polypeptide skeleton and represented by any one of the following General Formulas (1A) to (16A). By doing so, the obtained polypeptide derivative is formed of a material that contains a molecular group having a plasticizing function for the polypeptide skeleton, has a polypeptide skeleton similarly to a protein, and has excellent flexibility as a whole polypeptide derivative. Therefore, a synthetic leather having excellent flexibility can be produced. In the polypeptide derivative, since the first segment and the second segment are bonded, an occurrence of a macro-phase separation as in a case where a plasticizer is simply mixed with a protein is reduced.

The reaction between the compound containing the polypeptide skeleton and the compound represented by any one of the following General Formulas (1A) to (12A) and (14A) may be a Michael addition reaction.

The method for producing a synthetic leather may include a step of obtaining a polypeptide derivative by reacting a compound containing a polypeptide skeleton with a compound containing a molecular group having a plasticizing function for the polypeptide skeleton, and a compound having at least two or more of at least one structural units selected from the group consisting of structural units represented by General Formulas (2-1A) to (2-16A). In the step of obtaining the polypeptide derivative, the polypeptide skeleton may include at least one selected from the group consisting of a thiol group, an amino group, a hydroxy group, a guanidino group, a carboxy group, a phenoxy group, an indole group, an amide group, an azide group, and an alkynyl group, and the compound containing a molecular group may include at least one selected from the group consisting of a thiol group, an amino group, a hydroxy group, a guanidino group, a carboxy group, a phenoxy group, an indole group, an amide group, an azide group, and an alkynyl group.

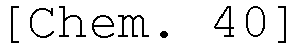

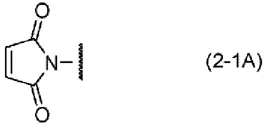

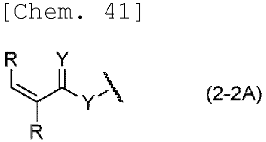

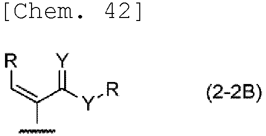

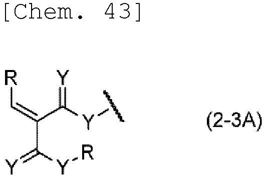

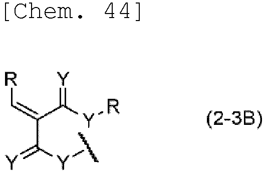

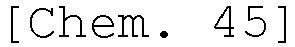

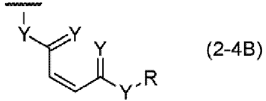

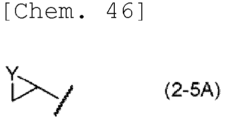

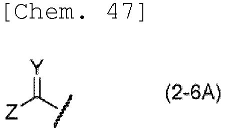

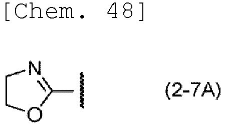

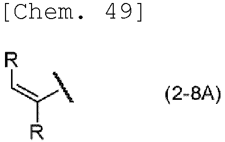

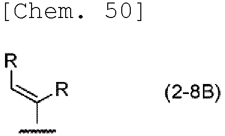

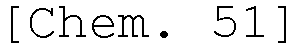

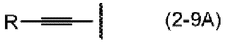

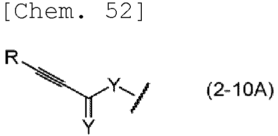

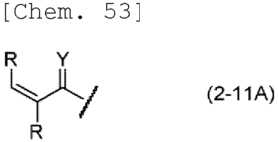

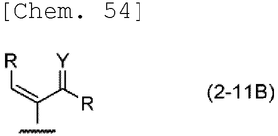

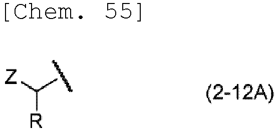

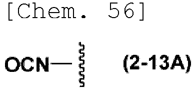

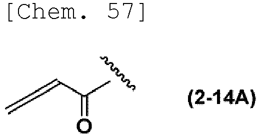

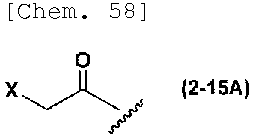

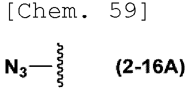

In General Formulas (2-2A), (2-2B), (2-3A), (2-3B), (2-4A), (2-5A), (2-6A), (2-10A), (2-11A), and (2-11B), Y's each independently represent an oxygen atom, a sulfur atom, or NR¹, and R¹ represents hydrogen, a hydrocarbon group, an aromatic group, a carbonyl group, or a sulfonyl group, in General Formulas (2-2A), (2-2B), (2-3A), (2-3B), (2-4A), (2-8A), (2-8B), (2-9A), (2-10A), (2-11A), (2-11B), and (2-12A), R's each independently represent hydrogen, a hydrocarbon group, or an aromatic group, in General Formulas (2-6A) and (2-12A), Z represents a halogen atom, a sulfonic acid ester group, or a fluorine-containing carboxylic acid ester group, and in General Formula (2-15A), X represents a halogen atom.

In the step of obtaining the polypeptide derivative, a polypeptide derivative is prepared by reacting a compound containing a polypeptide skeleton containing a specific functional group with a compound containing a molecular group having a plasticizing function for the polypeptide skeleton and containing a specific functional group, and a compound having at least two or more of at least one structural units selected from the group consisting of structural units represented by General Formulas (2-1A) to (2-16A). By doing so, the obtained polypeptide derivative is formed of a material that contains a molecular group having a plasticizing function for the polypeptide skeleton, has a polypeptide skeleton similarly to a protein, and has excellent flexibility as a whole polypeptide derivative. Therefore, a synthetic leather having excellent flexibility can be produced. In the polypeptide derivative, since the first segment and the second segment are bonded, an occurrence of a macro-phase separation as in a case where a plasticizer is simply mixed with a protein is reduced.

Still another aspect of the present disclosure provides a synthetic leather including a base material layer containing a fiber base material, and at least one of a skin layer bonded to the base material layer, an adhesive layer bonded to the base material layer and the skin layer, and a polymeric substance contained in the base material layer and impregnated into the fiber base material, in which at least one of the fiber base material, the skin layer, the adhesive layer, and the polymeric substance contains a recombinant polypeptide derivative (a polypeptide derivative containing a recombinant polypeptide skeleton) or a hydrophobic polypeptide derivative (a polypeptide derivative containing a hydrophobic polypeptide skeleton).

### Advantageous Effects of Invention

According to the present disclosure, it is possible to provide a synthetic leather having excellent flexibility while containing a material containing a polypeptide skeleton, and a method for producing the same.

### Brief Description of Drawings

Fig. 1 is a schematic cross-sectional view of a synthetic leather according to an embodiment.
Fig. 2 is a schematic cross-sectional view of a synthetic leather according to an embodiment.
Fig. 3 is a schematic cross-sectional view of a synthetic leather according to an embodiment.
Fig. 4 is a schematic cross-sectional view of a synthetic leather according to an embodiment.
Fig. 5 is a schematic view illustrating an example of a domain sequence of modified fibroin.
Fig. 6 is a diagram illustrating a distribution of values of z/w (%) in naturally derived fibroin.
Fig. 7 is a diagram illustrating a distribution of values of x/y (%) in naturally derived fibroin.
Fig. 8 is a schematic view illustrating an example of a domain sequence of modified fibroin.
Fig. 9 is a schematic view illustrating an example of a domain sequence of fibroin.
Fig. 10 is a photograph showing a comparison test result of bending amounts of synthetic leathers, in which the upper part of the photograph shows the result of Comparative Example A, and the lower part of the photograph shows the result of Example B.
Fig. 11 is a photograph showing a comparison test result of bending amounts of synthetic leathers of Examples, in which the upper part of the photograph shows the result of Example D, and the lower part of the photograph shows the result of Example B.
Fig. 12 is a photograph showing a comparison test result of a bending amount of a synthetic leather of Example C.
Fig. 13 is a view illustrating measurement results of SDS-PAGE for polypeptide derivatives prepared in Examples.
Fig. 14 is a view illustrating measurement results of SDS-PAGE for polypeptide derivatives prepared in Examples.
Fig. 15 is a schematic cross-sectional view of a pressure molding machine used in Examples.
Fig. 16 is a schematic view illustrating a method of using the pressure molding machine illustrated in Fig. 15, in which (a), (b), and (c) are schematic cross-sectional views of the pressure molding machine before introduction of a composition, after the introduction of the composition, and in a state where the composition is heated and pressurized, respectively.
Fig. 17 is a photograph showing an appearance of a test molded body molded using the polypeptide derivative of one of Examples.
Fig. 18 is a photograph showing an appearance of a test molded body molded using a mixture of one of Comparative Examples.
Fig. 19 is a photograph showing an appearance of a test molded body molded using a mixture of one of Comparative Examples.
Fig. 20 is a photograph showing an appearance of fibers prepared in Example 12.
Fig. 21 is a graph showing results of GPC measurement in Reference Example 2.
Fig. 22 is a photograph showing results of contact angle measurement in Reference Example 3.
Fig. 23 illustrates optical micrographs of cast films produced in Examples, in which Figs. 23(a) and 23(b) are optical micrographs showing a polypeptide derivative film and a modified fibroin + PEG mixture film enlarged at a magnification of 100 times, respectively.
Fig. 24 illustrates scanning electron micrographs of cast films produced in Examples, in which Figs. 24(a) and 24(b) are scanning micrographs showing a polypeptide derivative film and a modified fibroin + PEG mixture film enlarged at a magnification of 1,000 times, respectively.
Fig. 25 is a graph showing results of GPC measurement of polypeptide derivatives prepared in Examples and modified fibroin as a raw material thereof.

### Description of Embodiments

Hereinafter, modes for carrying out the present disclosure will be described in detail with reference to the drawings in some cases. However, the following embodiments are examples for describing the present disclosure, and are not intended to limit the present disclosure to the following contents.

The materials exemplified in the present specification can be used alone or in combination of two or more thereof unless otherwise specified. Contents of the respective components in a composition mean the total amount of a plurality of substances present in the composition, unless otherwise specified, when a plurality of substances corresponding to the respective components in the composition are present.

In the present specification, the polypeptide derivative refers to a composition containing a block copolymer in which a first segment and a second segment are bonded. The polypeptide derivative may contain an unreacted first segment (a substance for forming a first segment) and/or an unreacted second segment (a substance for forming a second segment). The polypeptide derivative in the present specification is in a uniform state without phase separation in macroscopic observation. The macroscopic observation is performed at a magnification of about 100 to 3,000 times by, for example, an optical microscope, a scanning electron microscope, or the like. Examples of a method of confirming the uniform state without phase separation by macroscopic observation include a method of confirming by forming a cast film formed of a substance to be observed and observing the cast film with an optical microscope or a scanning electron microscope at a magnification of about 100 to 3,000 times. Specifically, the state of being uniform without phase separation can be confirmed by the methods described in Examples described below. In the polypeptide derivative in the present specification, phase separation can be observed when observed microscopically (for example, on a submicron scale using an atomic force microscope).

Unlike a synthetic leather containing a mixture of a first segment and a second segment, and a composition in which one of the first segment and the second segment is dispersed in the other one, a synthetic leather containing such a polypeptide derivative (for example, a synthetic leather containing such a polypeptide derivative in the base material, the skin layer, the adhesive layer, and the like) is advantageous in that physical properties and mechanical properties are uniform in the entire polypeptide derivative-containing moiety (for example, the base material, the skin layer, the adhesive layer, or the like), and can advantageously suppress, for example, an occurrence of cracks. Not only transparency can be secured, but also moldability of the polypeptide derivative-containing moiety can be effectively enhanced at the time of producing the synthetic leather. A content of the block copolymer in which the first segment and the second segment are bonded with respect to the entire polypeptide derivative, the block copolymer being contained in the polypeptide derivative, is not particularly limited, and may be, for example, 30% or more, 40% or more, 50% or more, 60% or more, 70% or more, or 80% or more on a weight basis. However, it is desirable that the polypeptide derivative contains, as a main component, a block copolymer in which a first segment and a second segment are covalently bonded. Therefore, the content of the block copolymer contained in the polypeptide derivative with respect to the entire polypeptide derivative is preferably more than 50%, more preferably 60% or more, still more preferably 70% or more, further still more preferably 80% or more, and most preferably 85% or more on a weight basis. In the synthetic leather containing a polypeptide derivative containing the block copolymer in a proportion of more than 50%, the effect of improving flexibility and toughness obtained by containing the polypeptide derivative can be achieved at a higher level.

### [Synthetic Leather]

The synthetic leather according to the present embodiment includes a base material layer containing a fiber base material, and at least a part of the synthetic leather contains a polypeptide derivative. The synthetic leather may further include a layer other than the base material layer (another layer). The polypeptide derivative is contained in the base material layer or another layer. Since the synthetic leather according to the present embodiment contains a polypeptide derivative, the synthetic leather has excellent flexibility.

The synthetic leather is also called an artificial leather, and has a natural leather-like appearance and function. The synthetic leather can be obtained by, for example, using a woven fabric, a knitted fabric, a non-woven fabric, or the like as a fiber base material, and impregnating or coating a base material layer containing the fiber base material with a resin (polymer), or forming a resin (polymer) layer with or without an adhesive layer interposed therebetween. In the present specification, the synthetic leather also includes a so-called artificial leather (a synthetic leather using a specific non-woven fabric for a base material layer (obtained by impregnating a base material mainly including a fiber layer having a random three-dimensional stereoscopic structure with polyurethane or a polymeric substance having flexibility similar thereto)). In the synthetic leather according to the present embodiment, at least a part of the material constituting the synthetic leather (the base material layer, the adhesive layer, the resin (polymer) layer, or the like) contains a polypeptide derivative.

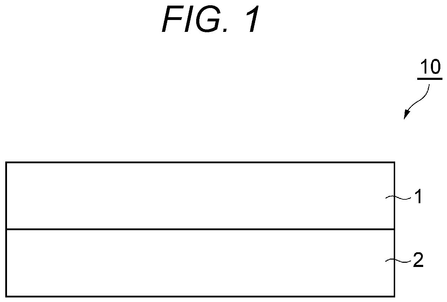

Fig. 1 is a schematic cross-sectional view of a synthetic leather according to an embodiment. A synthetic leather 10 illustrated in Fig. 1 includes a base material layer 2 and a skin layer 1 bonded to the base material layer 2. In the synthetic leather 10 illustrated Fig. 1, the skin layer 1 also functions as a shape retaining layer for retaining the shape of the synthetic leather.

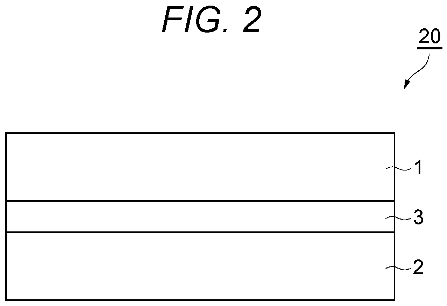

Fig. 2 is a schematic cross-sectional view of a synthetic leather according to another embodiment. A synthetic leather 20 illustrated in Fig. 2 includes a base material layer 2, a skin layer 1, and an adhesive layer 3 bonded to the base material layer 2 and the skin layer 1. In the synthetic leather 20 illustrated Fig. 2, the skin layer 1 also functions as a shape retaining layer for retaining the shape of the synthetic leather. The adhesive layer 3 is a layer formed in a case where the base material layer 2 and the skin layer 1 are bonded with an adhesive (for example, a polyurethane-based adhesive) (for example, in a case where a synthetic leather is produced by a dry method), and is not essential.

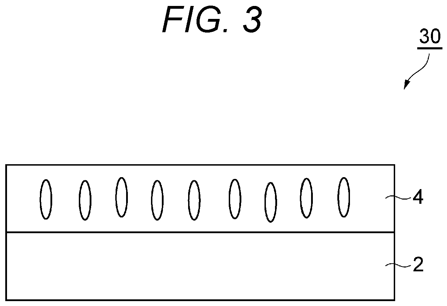

Fig. 3 is a schematic cross-sectional view of a synthetic leather according to another embodiment. A synthetic leather 30 illustrated in Fig. 3 includes a base material layer 2 and a porous layer 4 bonded to the base material layer 2. In the synthetic leather 30 illustrated in Fig. 3, the porous layer 4 functions as a skin layer. The porous layer 4 can be formed, for example, by producing a synthetic leather by a wet method. In the synthetic leather 30 illustrated in Fig. 3, a surface of the porous layer 4 (a surface opposite to a surface bonded to the base material layer 2) may be subjected to a surface processing such as embossing processing, buff suede processing, gravure printing, or film lamination processing.

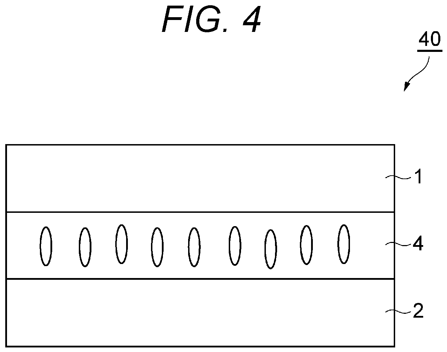

Fig. 4 is a schematic cross-sectional view of a synthetic leather according to another embodiment. A synthetic leather 40 illustrated in Fig. 4 includes a base material layer 2, a porous layer 4 bonded to the base material layer 2, and a skin layer 1 bonded to a surface of the porous layer 4 opposite to the base material layer 2. The synthetic leather 40 illustrated in Fig. 4 can be produced, for example, by subjecting a surface of the porous layer 4 of the synthetic leather 30 illustrated in Fig. 3 (a surface opposite to a surface bonded to the base material layer 2) to surface processing such as gravure printing or film lamination processing. In the synthetic leather 40 illustrated Fig. 4, the skin layer 1 and the porous layer 4 also function as shape retaining layers for retaining the shape of the synthetic leather. In the synthetic leather 40 illustrated in Fig. 4, the porous layer 4 and the skin layer 1 may be bonded with an adhesive layer (not illustrated) interposed therebetween, or the porous layer 4 and the base material layer 2 may be bonded with an adhesive layer (not illustrated) interposed therebetween.

### <Base Material Layer>

The base material layer contains a fiber base material. The fiber base material may be, for example, a knitted and woven fabric or a non-woven fabric. The knitted and woven fabric or the non-woven fabric as the fiber base material may be formed of synthetic fibers such as polyester, polyamide, polyacrylonitrile, polyolefin, and polyvinyl alcohol, natural fibers such as cotton, linen, and silk, generated fibers such as rayon, cotton wool, acetate, and collagen, single or blended fibers such as artificial protein fibers such as artificial casein, collagen, and fibroin, or multicomponent fibers modified into ultrafine fibers obtained by dissolving at least one component or splitting bicomponent fibers, and may be fibers of a polypeptide derivative. The base material layer or a polypeptide derivative fiber contained therein may contain known additives as necessary. Examples of the additives include a colorant, a smoothing agent, an antioxidant, an ultraviolet absorber, a dye, a filler, a crosslinking agent, a matting agent, and a leveling agent.

The knitted and woven fabric is a generic term of a knitted fabric and a woven fabric. The knitted fabric may be any of a knitted fabric having a weft knitting pattern such as flat knitting or circular knitting (simply referred to as a "weft knitted fabric") and a knitted fabric having a warp knitting pattern such as tricot or raschel (simply referred to as a "warp knitted fabric"). The woven fabric may be a woven fabric having any texture of a plain weave texture, a twill weave texture, and a satin weave texture. The knitted and woven fabric may be an unprocessed knitted and woven fabric itself obtained by knitting or weaving, or may be a knitted and woven fabric subjected to processing such as water repellent processing after knitting or weaving.

The knitted and woven fabric can be obtained by knitting or weaving raw material yarns. As a knitting method and a weaving method, known methods can be used. As a knitting machine to be used, for example, a circular knitting machine, a warp knitting machine, a flat knitting machine, or the like can be used, and a circular knitting machine is preferably used from the viewpoint of productivity. Examples of the flat knitting machine include a mold knitting machine and a seamless knitting machine, and in particular, it is more preferable to use a seamless knitting machine because a knitted fabric can be produced in a form of a final product. Examples of a weaving machine to be used include a shuttle weaving machine and a shuttle-less weaving machine such as a gripper weaving machine, a rapier weaving machine, or an air jet weaving machine.

The raw material yarn may be a single yarn, a composite yarn (for example, a blended yarn, a mixed yarn, a covering yarn, or the like), or a combination thereof. The single yarn and the composite yarn may be spun yarns in which short fibers are twisted, or may be filament yarns in which long fibers are twisted. Examples of the fiber contained in the raw material yarn include synthetic fibers such as nylon, polyester, polyamide, polyacrylonitrile, polyolefin, polyvinyl alcohol, polyethylene terephthalate, and polytetrafluoroethylene, regenerated fibers such as cupro, rayon, and lyocell, natural fibers such as cotton, linen, and silk, protein fibers, and fibers of a polypeptide derivative.

A non-woven fabric can be produced by a known production method using, for example, the fiber described above. Specifically, a non-woven fabric can be obtained, for example, by forming a web (including a single layer web and a laminated web) using the fiber described above by a dry method, a wet method, an air-laid method, and the like, and then bonding fibers of the web by a chemical bond method (an immersion method, a spray method, or the like), a needle punch method, and the like. The non-woven fabric can also be formed by spinning by an electrospinning method.

The non-woven fabric is appropriately set so that a numerical range of a fiber density (basis weight), a porosity, a bulk density, or the like is, for example, within a range that can sufficiently secure waterproofness and moisture permeability. These basis weight, porosity, bulk density, and the like can be adjusted, for example, by increasing and decreasing the amount of fibers constituting the web, and increasing or decreasing the number of laminated layers in the case of the laminated web.

The base material layer may further contain a polymeric substance. In the case where the base material layer further contains a polymeric substance, the base material layer can also be referred to as a layer in which a fiber base material and an impregnated body formed of a polymeric substance impregnated into the fiber base material are integrated, or can also be referred to as a layer in which a fiber base material is embedded in a layer formed of a polymeric substance.

The polymeric substance is generally impregnated into the fiber base material in a solution state and coagulated (solidified) by desolvation, and may be present in gaps between fibers of the fiber base material in a state of being fixed to the fibers. That is, the polymeric substance can constitute a base material layer of a synthetic leather together with the fiber base material as a so-called impregnated body integrated with the fiber base material, and connect the fibers of the fiber base material to each other to retain the shape of the base material layer and impart predetermined strength to the base material layer. Examples of such a polymeric substance include synthetic resins such as polyvinyl chloride, polyolefin, polystyrene, polyurethane, a polyester resin, an epoxy resin, an acrylic polymer, and an acrylonitrile-based polymer, and proteins. Examples of the polyurethane include polyether-based polyurethane, polyester-based polyurethane, and polycarbonate-based polyurethane, and these polyurethanes may be used alone or in combination of two or more thereof. The polyurethane can be synthesized, for example, by reacting a diisocyanate with a polyol to synthesize a prepolymer, and reacting the prepolymer with a chain extender. The diisocyanate may be an aromatic compound or an aliphatic compound. The polyol may be, for example, polyester, polyether, polycarbonate, silicone, fluororesin, or the like. When polyether is used as the polyol, hydrolysis resistance, mold resistance, and cold resistance are excellent, and when polycarbonate is used as the polyol, hydrolysis resistance and mold resistance are excellent. The chain extender may be, for example, a glycol, a diamine, a reaction terminator, or the like. The polymeric substance may contain a polypeptide derivative. In the case where the polymeric substance contains a polypeptide derivative, it is possible to impart excellent flexibility to the synthetic leather. Such a polymeric substance may also contain known additives as necessary. Examples of the additives include a colorant, a smoothing agent, an antioxidant, an ultraviolet absorber, a dye, a filler, a crosslinking agent, a matting agent, and a leveling agent.

The base material layer may be a porous layer. When the base material layer is a porous layer, a more voluminous hand feeling can be realized.

A thickness of the base material layer may be, for example, 100 to 2,000 µm, 100 to 1,000 µm, 500 to 1,000 um, or 500 to 750 µm.

### <Skin Layer>

The skin layer is a layer mainly formed of a resin (polymer). The skin layer may be formed of a porous resin (polymer) (porous layer) or may be formed of a non-porous resin (polymer). The porous layer can be formed, for example, by producing a synthetic leather by a wet method.

Examples of the resin (polymer) forming the skin layer include a polypeptide derivative, a protein, and a synthetic resin. Examples of the synthetic resin include polyvinyl chloride, polyolefin, polystyrene, polyurethane, a polyester resin, an epoxy resin, an acrylic polymer, and an acrylonitrile polymer. As the resin (polymer) forming the skin layer, a composite material containing at least two of a polypeptide derivative, a protein, and a synthetic resin may be used. Examples of the polyurethane include polyether-based polyurethane, polyester-based polyurethane, and polycarbonate-based polyurethane, and these polyurethanes may be used alone or in combination of two or more thereof.

The skin layer may also contain known additives as necessary. Examples of the additives include a colorant, a smoothing agent, an antioxidant, an ultraviolet absorber, a dye, a filler, a crosslinking agent, a matting agent, and a leveling agent.

A thickness of the skin layer is not particularly limited, and may be, for example, 1 to 1,000 µm, 1 to 700 µm, 1 to 500 µm, 1 to 400 µm, 5 to 300 µm, 5 to 200 um, or 10 to 100 µm.

A surface of the skin layer (a surface opposite to a surface bonded to the base material layer) may be subjected to surface processing. A known method can be applied to the surface processing. Examples of the surface processing include embossing processing, buff suede processing, film lamination processing, and gravure processing (gravure printing).

### <Adhesive Layer>

The adhesive layer is formed using an adhesive. Examples of a resin (polymer) used as an adhesive include a polypeptide derivative, a protein, and a synthetic resin. Examples of the synthetic resin include polyolefin, polystyrene, polyurethane, acryl, and an ethylene vinyl acetate copolymer (EVA). As the resin (polymer) forming the adhesive layer, a composite material containing at least two of a polypeptide derivative, a protein, and a synthetic resin may be used. The adhesive layer may also contain known additives as necessary. Examples of the additives include a colorant, a smoothing agent, an antioxidant, an ultraviolet absorber, a dye, a filler, a crosslinking agent, a matting agent, and a leveling agent.

A thickness of the adhesive layer is not particularly limited, and may be, for example, 1 to 500 µm, 5 to 400 µm, 10 to 300 µm, 10 to 200 um, or 10 to 100 µm.

### <Method for Producing Synthetic Leather>

The synthetic leather according to the present embodiment can be produced by a method including the following step (1), step (2), or step (3). The polypeptide derivative is as described above.
(1) A step of forming a skin layer on the base material layer with an adhesive layer interposed therebetween, in which at least one of the base material layer, the adhesive layer, and the skin layer contains a polypeptide derivative
(2) A step of forming a skin layer on the base material layer without interposing an adhesive layer, in which at least one of the base material layer and the skin layer contains a polypeptide derivative
(3) A step of forming a base material layer containing a fiber base material and a polymeric substance impregnated into the fiber base material by impregnating the fiber base material with an impregnation solution containing a polymeric substance and then solidifying the polymeric substance, in which at least one of the fiber base material and the polymeric substance contains a polypeptide derivative

The step (3) can be performed by, for example, a method (wet method) in which the fiber base material is immersed in an impregnation solution or the fiber base material is sprayed with the impregnation solution to bring the fiber base material and the impregnation solution into contact with each other to impregnate the fiber base material with the impregnation solution, and then the fiber base material is brought into contact with (for example, immersed in) a coagulation liquid (coagulation solution) to coagulate components in the impregnation solution, thereby forming a base material layer. For example, a solvent solution mainly containing a polymeric substance is applied to the fiber base material. Next, for example, when the base material layer material is immersed in a coagulation solution mainly containing water, the solvent in the base material layer material is eluted into the coagulation solution, and at the same time, the coagulation solution mainly containing water permeates and diffuses into the base material layer material, and the base material layer material is wet-coagulated. As a result, a porous base material layer can be formed. Depending on the type of the solvent (for example, a solvent having a low boiling point), the base material layer forming step can also be performed by a method in which the fiber base material impregnated with the impregnation solution as described above is not brought into contact with the coagulation solution, and the components in the impregnation solution are coagulated by performing desolvation by volatilizing the solvent of the impregnation solution or the like, thereby forming the base material layer.

The solvent used in the impregnation solution can be appropriately selected depending on the type of the polymeric substance, and examples thereof include organic solvents such as dimethyl sulfoxide (DMSO), N,N-dimethylformamide (DMF), formic acid, alcohol, and hexafluoroisopropanol (HFIP).

The type of the coagulation solution can be appropriately selected according to the types of the fiber base material and the polymeric substance and the type of the solvent of the impregnation solution. For example, in a case where the polymeric substance contains polyurethane, for example, N,N-dimethylformamide can be employed as the solvent of the impregnation solution, and water (hot water) can be employed as the coagulation solution. For example, in a case where the polymeric substance contains a protein, as a solvent of the impregnation solution, for example, dimethyl sulfoxide (DMSO), N,N-dimethylformamide (DMF), formic acid, or hexafluoroisopropanol (HFIP), a solvent obtained by adding an inorganic salt as a dissolution accelerator thereto, a lower alcohol having 1 to 5 carbon atoms such as methanol, ethanol, or 2-propanol, acetone, and water (hot water) can be adopted as the coagulation solution. Various additives may be added to the coagulation solution as necessary.

The synthetic leather can be produced, for example, by either a dry method or a wet method including the step (1) or the step (2). The base material layer in the step (1) and the step (2) may be a base material layer formed by a method including the step (3), or may be a base material layer (for example, a base material layer containing no polypeptide derivative) formed by another method.

In the dry method, for example, a synthetic leather can be produced by a method including a step of applying a skin layer material on release paper, a step of applying an adhesive to a surface of the skin layer material on a side opposite to the release paper, and a step of overlapping a base material layer on a surface of the adhesive on a side opposite to the skin layer material and thermocompression-bonding the base material layer. In a case where the skin layer material can be integrally bonded to the base material layer only by, for example, thermocompression-bonding or the like depending on the types of the skin layer material and the material provided for the base material layer, the adhesive can be omitted.

In the wet method, for example, a synthetic leather can be produced by a method including a step of applying a solution containing a skin layer material onto a base material layer, and a step of immersing the base material layer to which the solution containing the skin layer material is applied in a coagulation solution to form a skin layer. In the wet method, it is also possible to form a porous skin layer. For example, a solvent solution mainly containing a resin (polymer) as a skin layer material is applied to a base fabric. Next, for example, when the skin layer material is immersed in a coagulation solution mainly containing water, the solvent in the skin layer material is eluted into the coagulation solution, and at the same time, the coagulation solution mainly containing water permeates and diffuses into the skin layer material, and the skin layer material is wet-coagulated. As a result, a porous skin layer can be formed. Depending on the type of the solvent of the solution containing the skin layer material (for example, a solvent having a low boiling point or the like), the skin layer material may be coagulated by performing desolvation by volatilizing the solvent of the solution containing the skin layer material or the like without bringing the base fabric to which the solution containing the skin layer material is applied into contact with the coagulation solution to form the skin layer. After the skin layer is formed, washing and drying may be performed as necessary.

The type of the coagulation solution can be appropriately selected according to the skin layer material (polymer) and the type of solvent in which the skin layer material is dissolved or dispersed. For example, in a case where the skin layer material (polymer) is polyurethane, for example, dimethylformamide can be employed as the solvent and water (hot water) can be employed as the coagulation solution. For example, in a case where the skin layer material (polymer) is a protein, as a solvent, for example, dimethyl sulfoxide (DMSO), N,N-dimethylformamide (DMF), formic acid, or hexafluoroisopropanol (HFIP), a solvent obtained by adding an inorganic salt as a dissolution accelerator thereto, a lower alcohol having 1 to 5 carbon atoms such as methanol, ethanol, or 2-propanol, acetone, and water can be adopted as the coagulation solution. Various additives may be added to the coagulation solution as necessary.

### <Application of Synthetic Leather>

The synthetic leather according to the present embodiment can be used for applications where a conventional synthetic leather (for example, a synthetic leather formed of a synthetic resin) has been used. The synthetic leather according to the present embodiment can be used for, for example, in applications such as decorations for clothing, shoes, bags, and the like, various covers and furniture, and automobile interior materials.

### <Polypeptide Derivative>

The polypeptide derivative contains a block copolymer containing a first segment containing a polypeptide skeleton and one or a plurality of second segments bonded to the first segment. The second segment contains a molecular group having a plasticizing function for the polypeptide skeleton. The number of second segments is one or plural, preferably 2 or more, more preferably 2 to 10, still more preferably 2 to 8, further still more preferably 2 to 6, and most preferably 2 to 4, with respect to one first segment.

The block copolymer contained in the polypeptide derivative only needs to contain the first segment and the second segment, and for example, may be a copolymer in which one or a plurality of second segments are bonded to one first segment, or may be a copolymer in which a plurality of blocks containing the first segment and the second segment bonded to the first segment are linked. The block copolymer contained in the polypeptide derivative may be a polymer (for example, a graft polymer or the like) containing a first segment containing a polypeptide skeleton as a main chain and a second segment as a side chain. The block copolymer may contain, for example, a plurality of first segments, and the first segment and the second segment may be alternately bonded. The first segment and the second segment are alternately bonded, such that, for example, the toughness of the synthetic leather can be further improved.

The block copolymer contained in the polypeptide derivative may contain the plurality of first segments, and some of the second segments may be bonded to two or more first segments to form a network structure. The network structure is formed, such that toughness of the synthetic leather can be further improved.

The first segment and the second segment may be directly bonded, or may be bonded by a structure that allows the first segment and the second segment to be bonded. In any of these bonding forms, the bond between the first segment and the second segment may be a coordinate bond, a bond by an ionic interaction, or a covalent bond. Among them, a covalent bond is desirable.

### <First Segment>

The first segment contains a polypeptide skeleton, and may, for example, consist only of the polypeptide skeleton. The first segment may be bonded to the second segment by a functional group (for example, a thiol group of cysteine or the like) in an amino acid sequence constituting the polypeptide skeleton. In other words, any recombinant polypeptide or hydrophobic polypeptide containing a functional group capable of binding to the second segment can be employed as the polypeptide skeleton. At this time, the number of the functional groups of the polypeptide may be, for example, 1 or more, 2 or more, or 4 or more. The number of the functional groups of the polypeptide may be, for example, 30 or less, 25 or less, 20 or less, 15 or less, 10 or less, or 8 or less. The number of the functional groups of the polypeptide can be adjusted within the above range, and may be, for example, 1 to 30, 1 to 25, 1 to 20, 1 to 15, 1 to 10, 1 to 8, or 2 to 8. The first segment may also be bonded to the second segment by a functional group introduced into a functional group in an amino acid sequence constituting the polypeptide skeleton.

The functional group as described above may be, for example, at least one selected from the group consisting of a thiol group, an amino group, a hydroxy group, a guanidino group, a carboxy group, a phenoxy group, an indole group, an amide group, an azide group, and an alkynyl group, preferably at least one selected from the group consisting of a thiol group, an amino group, a hydroxy group, a guanidino group, a carboxy group, a phenoxy group, an indole group, and an amide group, more preferably at least one selected from the group consisting of a thiol group, an amino group, a hydroxy group, a guanidino group, a carboxy group, a phenoxy group, and an indole group, still more preferably at least one selected from the group consisting of a thiol group, an amino group, a hydroxy group, a guanidino group, a carboxy group, and a phenoxy group, still more preferably at least one selected from the group consisting of a thiol group, an amino group, a hydroxy group, a guanidino group, and a carboxy group, still more preferably at least one selected from the group consisting of a thiol group, an amino group, a hydroxy group, and a guanidino group, particularly preferably at least one selected from the group consisting of a thiol group, an amino group, and a hydroxy group, particularly preferably at least one selected from the group consisting of a thiol group and an amino group, and most preferably a thiol group. The amino group may be, for example, an amino group of lysine. The hydroxy group may be, for example, a hydroxy group of serine, a hydroxy group of threonine, or the like. The guanidino group may be, for example, a guanidino group of arginine. The carboxy group may be, for example, a carboxy group of glutamic acid, a carboxy group of aspartic acid, or the like. The phenoxy group may be, for example, a phenoxy group of tyrosine. The indole group may be, for example, an indole group of tryptophan. The amide group may be, for example, an amide group of glutamine, an amide group of asparagine, or the like. The alkynyl group may be an alkynyl group introduced by reacting halogenated acetylene with a thiol group of cysteine.

A molecular weight of the first segment is, for example, preferably 200 to 1,000,000, more preferably 300 to 900,000, still more preferably 400 to 800,000, still more preferably 500 to 700,000, still more preferably 600 to 600,000, still more preferably 1,000 to 600,000, still more preferably 3,000 to 600,000, still more preferably 5,000 to 600,000, still more preferably 10,000 to 600,000, and still more preferably 5,000 to 100,000.

When the molecular weight of the first segment is 200 or more, the first segment that functions as a hard segment has a sufficient size with respect to the second segment (soft segment) containing a molecular group having a plasticizing function. In such a case, the rigidity of the synthetic leather molded using the polypeptide derivative is sufficiently increased, and the synthetic leather is more easily used as a material of the synthetic leather. When the molecular weight of the first segment is 1,000,000 or less, the reactivity of the linking reaction is reduced, such that the reaction can be completed in a time when the first segment can be chemically produced as a material in a commercial manner, and as a result, localization of an unreacted second segment remaining in the material of the synthetic leather may be easily suppressed.

The molecular weight of the first segment and a molecular weight of the polypeptide skeleton contained in the first segment may be, for example, 1,000 or more, 2,000 or more, 3,000 or more, 4,000 or more, 5,000 or more, 6,000 or more, 7,000 or more, 8,000 or more, 9,000 or more, 10,000 or more, 20,000 or more, 30,000 or more, 40,000 or more, 50,000 or more, 60,000 or more, 70,000 or more, 80,000 or more, 90,000 or more, or 100,000 or more. The molecular weight of the first segment and the molecular weight of the polypeptide skeleton contained in the first segment may be 400,000 or less, less than 360,000, 300,000 or less, or 200,000 or less.

The solubility of each of the first segment and the polypeptide skeleton in the solvent tends to increase as the molecular weight of each of the first segment and the polypeptide skeleton decreases. Therefore, in a case where the molecular weight of the polypeptide skeleton and/or the first segment is, for example, 200,000 or less or 100,000 or less, when a compound containing a polypeptide skeleton is dissolved in a solvent and is reacted with a compound containing a molecular group having a plasticizing function for the polypeptide skeleton to obtain a polypeptide derivative, it can be desired to improve generation efficiency of the polypeptide derivative to be targeted. In a synthetic leather molded using the polypeptide derivative obtained as described above, improvement of flexibility can be expected while securing a certain degree of strength. When the molecular weight of the polypeptide skeleton and/or the first segment is, for example, 200,000 or less or 100,000 or less, it is expected that the amount of the second segment used can be suppressed as much as possible in order to enhance the flexibility of the polypeptide derivative. The molecular weight of the first segment and the molecular weight of the polypeptide skeleton are weight average molecular weights.

In the present specification, the molecular weight is a value measured by sodium dodecyl sulfatepolyacrylamide gel electrophoresis (SDS-PAGE). The electrophoresis is performed in the following procedure. That is, first, 200 µL of 2 M lithium chloride DMSO (manufactured by FUJIFILM Wako Pure Chemical Corporation) is added to 2 mg of a powder sample, and the mixture is stirred while being heated at 80°C for 60 minutes and further at 95°C for 10 minutes to dissolve the sample. Thereafter, the sample is diluted to 50 times with a 10 M urea solution, further diluted to 2 times with a sample buffer (manufactured by FUJIFILM Wako Pure Chemical Corporation), and heated at 95°C for 5 minutes to denaturalize the protein. Next, a gel for SDS-PAGE (manufactured by Bio-lad) is attached to an electrophoresis device (manufactured by Bio-lad), the device is filled with an SDS buffer, and the electrophoresis device is connected to a power supply device (manufactured by Bio craft). 10 µL of the denatured sample is added to each well of the gel for SDS-PAGE, and a current of 30 mA/1 sheet is allowed to flow for 30 minutes. After completion of the electrophoresis, the gel for SDS-PAGE is taken out from the device, immersed in Oriole fluorescent gel stain (manufactured by Bio-lad), and shaken for 1 hour. Thereafter, the gel is placed on a UV sample tray (manufactured by Bio-lad), and a stained image is acquired with a Gel Doc EZ gel imaging device (manufactured by Bio-lad) .

The number of amino acid residues constituting the polypeptide skeleton may be 50 or more. The number of amino acid residues may be, for example, 100 or more, 150 or more, 200 or more, 250 or more, 300 or more, 350 or more, 400 or more, 450 or more, or 500 or more. The number of amino acid residues may be, for example, 5,000 or less, 4,500 or less, 4,000 or lee, 3,500 or less, 3,000 or less, 2,500 or less, 2,000 or less, 1,500 or less, or 1,000 or less. As the number of amino acid residues is smaller, the solubility in the solvent tends to increase. Therefore, in a case where the number of amino acid residues of the polypeptide according to the present embodiment is, for example, 5,000 or less or 2,500 or less, when a compound containing a polypeptide skeleton is dissolved in a solvent and is reacted with a compound containing a molecular group having a plasticizing function for the polypeptide skeleton to obtain a polypeptide derivative, it can be desired to improve generation efficiency of the polypeptide derivative to be targeted. In a synthetic leather obtained by using the polypeptide derivative obtained as described above, improvement of flexibility can be expected while securing a certain degree of strength.

The polypeptide skeleton is a recombinant polypeptide skeleton or a hydrophobic polypeptide skeleton, and may be a hydrophobic recombinant polypeptide skeleton.

The recombinant polypeptide represents a polypeptide produced by genetic recombination. In a case where the polypeptide skeleton is a recombinant polypeptide skeleton, it is easy to change the design of the amino acid sequence, and thus it is easy to control the properties, physical properties, and the like of the polypeptide derivative. In a case where the polypeptide skeleton is a recombinant polypeptide skeleton, since a uniform molecular design is always possible, a polypeptide skeleton according to a purpose can be stably obtained. This advantageously stabilizes the qualities of the polypeptide derivative and the synthetic leather to be targeted.

In a case where the polypeptide skeleton is a hydrophobic polypeptide skeleton, the affinity of the molecular group having a plasticizing function with the first segment is improved, and a synthetic leather having more excellent flexibility can be produced. In addition, the water resistance of such a synthetic leather is improved, and for example, in a case where the synthetic leather is used as a general-purpose material for industrial purposes, the use life can be advantageously extended. In the polypeptide derivative, for example, the hydrophobicity or hydrophilicity of the entire polypeptide derivative can be arbitrarily adjusted by controlling the hydrophobicity or hydrophilicity of the molecular group having a plasticizing function for the polypeptide skeleton contained in the second segment. In a case where the polypeptide skeleton is a hydrophobic polypeptide skeleton, the entire polypeptide derivative can be shifted to be hydrophobic as compared with a case where the polypeptide skeleton is a hydrophilic polypeptide skeleton, and thus, the hydrophobicity or hydrophilicity of the entire polypeptide derivative can be controlled over a wider range.

The hydrophobicity of the hydrophobic polypeptide skeleton can be determined using a value of the average hydropathy index (hydrophobic degree : hydrophobicity index) described below as an index. The value of the average hydropathy index of the hydrophobic polypeptide skeleton may be, for example, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit value thereof is not particularly limited, and may be, for example, 1.00 or less or 0.7 or less.

The hydrophobic polypeptide skeleton preferably has low solubility in a lithium bromide aqueous solution (concentration: 9 M) at 60°C. This solubility can be evaluated using a compound (polypeptide) corresponding to the hydrophobic polypeptide skeleton or a polypeptide obtained by decomposing a polypeptide derivative and isolating only a hydrophobic polypeptide skeleton. In a case where the polypeptide is dissolved in a lithium bromide aqueous solution (concentration: 9 M) at 60°C, a maximum concentration may be, for example, less than 30 mass%, less than 25 mass%, less than 20 mass%, less than 15 mass%, less than 10 mass%, less than 5 mass%, or less than 1 mass%. The hydrophobic polypeptide skeleton may be completely insoluble in a lithium bromide aqueous solution (concentration: 9 M) at 60°C.

The hydrophobic polypeptide skeleton preferably has a large water contact angle. The water contact angle can be evaluated using a compound (polypeptide) corresponding to the hydrophobic polypeptide skeleton on a base material or a membrane formed of a polypeptide obtained by decomposing a polypeptide derivative and isolating only a hydrophobic polypeptide skeleton. The hydrophobic polypeptide skeleton is preferably a polypeptide constituting a membrane having a contact angle of 55° or more 5 seconds after adding dropwise water to the membrane. The contact angle may be, for example, 60° or more, 65° or more, or 70° or more.

The hydrophobic polypeptide skeleton preferably has excellent hot water resistance. The hot water resistance can be evaluated using a compound (polypeptide) corresponding to the hydrophobic polypeptide skeleton or a polypeptide obtained by decomposing a polypeptide derivative and isolating only a hydrophobic polypeptide skeleton. The hydrophobic polypeptide skeleton is preferably a polypeptide that is not decomposed even when a dispersion in which a content of the polypeptide is 5 mass% is prepared, the dispersion containing the polypeptide and water, and the dispersion is heated at 100°C for 5 hours.

The polypeptide skeleton may include a skeleton derived from a protein, or may be composed only of a skeleton derived from a protein. The protein may be a recombinant protein or modified fibroin. When the polypeptide skeleton includes a skeleton derived from a recombinant protein, the number and position of functional groups in the amino acid sequence constituting the polypeptide skeleton can be arbitrarily adjusted. Therefore, the structure and properties of the polypeptide derivative can be designed.

Examples of the recombinant protein include any protein that can be produced in an industrial scale, and examples thereof include a protein that can be used for industrial purposes. The phrase "can be used for industrial purposes" means, for example, that it can be used for various general-purpose materials used indoors or outdoors.

The recombinant protein may be a protein having a domain sequence different from an amino acid sequence of a naturally derived protein, or may be a protein having a domain sequence identical to an amino acid sequence of a naturally derived protein. The recombinant protein may be a protein obtained by using an amino acid sequence of a naturally derived protein as it is, a protein in which an amino acid sequence is modified based on an amino acid sequence of a naturally derived protein (for example, a protein in which an amino acid sequence is modified by modifying a cloned gene sequence of a naturally derived protein), or a protein artificially designed and synthesized independently of a naturally derived protein (for example, a protein having a desired amino acid sequence by chemically synthesizing a nucleic acid encoding a designed amino acid sequence).

Unlike a natural protein, in the case of the recombinant protein, an amino acid sequence can be freely designed. Therefore, in a case where a skeleton derived from the recombinant protein is contained in a polypeptide derivative, the function, properties, physical properties, and the like of the synthetic leather can be arbitrarily controlled by appropriately designing the amino acid sequence of the recombinant protein. In a case where a skeleton derived from a recombinant protein is contained in the polypeptide derivative, since a uniform molecular design is always possible, a protein that has high homology with a target protein and is suitable for a purpose can be stably obtained. As a result, the quality of the target polypeptide derivative, and ultimately, the synthetic leather obtained using the same can be advantageously stabilized.

The polypeptide skeleton may include a skeleton derived from a recombinant structural protein. The structural protein means a protein related to a structure of a living body, a protein included in a structure created by a living body, or a protein derived from these proteins. The structural protein also refers to a protein that selfaggregates under predetermined conditions to form a structure such as a fiber, a film, a resin, a gel, a micelle, or a nanoparticle. Specific examples of the structural protein include spider silk, silkworm silk, keratin, collagen, elastin, resilin, and proteins derived from them. The recombinant structural protein is a structural protein produced microbially by genetic recombination. The recombinant structural protein may have an improved amino acid sequence from the viewpoint of moldability and/or productivity.

When a protein is artificially molded, an amino acid having a smaller side chain is more likely to be hydrogenbonded, and a molded body having higher strength is more likely to be obtained. Since the alanine residues and the glycine residues are amino acids having nonpolar side chains, the alanine residues and the glycine residues are arranged to face inward in a folding process during the polypeptide production and tend to have α-helix structure or β-sheet structure. Therefore, it is desirable that a proportion of amino acids such as a glycine residue, an alanine residue, and a serine residue is high. From the viewpoint of improving the strength, a content of the alanine residues may be, for example, 10 to 40%, 12 to 40%, 15 to 40%, 18 to 40%, 20 to 40%, or 22 to 40%. From the viewpoint of improving the strength, a content of the glycine residues may be, for example, 10 to 55%, 11 to 55%, 13 to 55%, 15 to 55%, 18 to 55%, 20 to 55%, 22 to 55%, or 25 to 55%.

In the present specification, the "content of the alanine residues" is a value represented by the following equation. Content of alanine residues = (Number of alanine residues contained in polypeptide/Number of all amino acid residues of polypeptide) × 100(%)

The content of the glycine residues, the content of the serine residues, the content of the threonine residues, the content of the proline residues, and the content of the tyrosine residues have the same meanings as those obtained by replacing the alanine residue with the glycine residue, the serine residue, the threonine residue, the proline residue, and the tyrosine residue, respectively, in the above equation.

From the viewpoint of improving processability, it is important to inhibit strong hydrogen bonding between molecules at the time of processing, and from this viewpoint, it is desirable that an amino acid having a large side chain or an amino acid having flexibility is uniformly contained in the entire sequence to a certain extent. Specifically, a motif containing a tyrosine residue, a threonine residue, or a proline residue may be repeated and inserted in a cycle. For example, a total content of the proline residues, the threonine residues, and the tyrosine residues in arbitrary 20 consecutive amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. For example, the total content of the proline residues, the threonine residues, and the tyrosine residues in arbitrary 20 consecutive amino acid residues may be 50% or less, 40% or less, 30% or less, or 20% or less.

A total of the content of the serine residues, the content of the threonine residues, and the content of the tyrosine residues in the protein may be 4% or more, 4.5% or more, 5% or more, 5.5% or more, 6% or more, 6.5% or more, or 7% or more. The total of the content of the serine residues, the content of the threonine residues, and the content of the tyrosine residues may be, for example, 35% or less, 33% or less, 30% or less, 25% or less, or 20% or less.

The protein may have a repetitive sequence. That is, the protein may have a plurality of amino acid sequences (repetitive sequence units) with high sequence identity. The number of amino acid residues of the repetitive sequence units is preferably 6 to 200. A sequence identity between the repetitive sequence units may be, for example, 85% or higher, 90% or higher, 95% or higher, 96% or higher, 97% or higher, 98% or higher, or 99% or higher. A hydrophobic degree (hydropathy index) of the repetitive sequence unit may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit value of the hydrophobic degree of the repetitive sequence unit is not particularly limited, and may be, for example, 1.0 or less or 0.7 or less.

The protein may contain an (A)ₙ motif. Herein, the (A)ₙ motif means an amino acid sequence mainly containing an alanine residue. The number of amino acid residues in the (A)ₙ motif may be 2 to 27 and may be an integer of 2 to 20, 2 to 16, or 2 to 12. A proportion of the number of alanine residues to the number of all amino acid residues in the (A)ₙ motif may be 40% or more and may also be 60% or more, 70% or more, 80% or more, 83% or more, 85% or more, 86% or more, 90% or more, 95% or more, or 100% (which means that the (A)ₙ motif consists of alanine residues).

The protein preferably has an amino acid sequence corresponding to an amino acid sequence in which a cysteine residue is inserted at a position adjacent to a glycine residue, a serine residue, or an alanine residue, and more preferably has an amino acid sequence corresponding to an amino acid sequence in which a cysteine residue is inserted at a position adjacent to a glycine residue. The cysteine residue has a mercapto group (also referred to as a thiol group or a sulfhydryl group), and the selectivity of the mercapto group can facilitate binding to the second segment. Therefore, depending on the insertion position of the cysteine residue in the protein, the binding position of the second segment to the polypeptide skeleton of the first segment can be set to any position. The cysteine residue may be located between the glycine residue, the serine residue, or the alanine residue and the glycine residue, the serine residue, or the alanine residue, or may be located between the serine residue and the glycine residue.

The protein preferably has an amino acid sequence corresponding to an amino acid sequence in which a cysteine residue is inserted at a position adjacent to a hydrophobic amino acid residue. In this case, the hydrophobic amino acid residues are immobilized by a hydrophobic interaction between molecules. The cysteine residue may be located adjacent to the hydrophobic amino acid residue, may be located between the hydrophobic amino acid residue and an amino acid residue other than the hydrophobic amino acid residue, may be located between the hydrophobic amino acid residue and the glycine residue, the serine residue, or the alanine residue, or may be located between the hydrophobic amino acid residue and the glycine residue. The hydrophobic amino acid residue may be one selected from the group consisting of an isoleucine residue, a valine residue, a leucine residue, a phenylalanine residue, a methionine residue, and an alanine residue.

As the protein, fibroin is preferable. An example of the fibroin includes naturally derived fibroin. Examples of the naturally derived fibroin include fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and a hornet silk protein secreted by larvae of Vespa simillima xanthoptera.

More specific examples of the fibroin produced by insects include a silkworm fibroin L chain (GenBank Accession No. M76430 (base sequence), AAA27840.1 (amino acid sequence)) .

Examples of the fibroin produced by spiders include spider silk proteins produced by spiders belonging to the genus *Araneus,* such as *Araneus ventricosus, Araneus diadematus, Araneus pinguis, Araneus pentagrammicus,* and *Araneus nojimai,* spiders belonging to the genus *Neoscona,* such as *Neoscona scylla, Neoscona nautica, Neoscona adianta,* and *Neoscona scylloides,* spiders belonging to the genus *Pronus,* such as *Pronous minutus,* spiders belonging to the genus *Cyrtarachne,* such as *Cyrtarachne bufo* and *Cyrtarachne inaequalis,* spiders belonging to the genus *Gasteracantha,* such as *Gasteracantha kuhlii* and *Gasteracantha mammosa,* spiders belonging to the genus *Ordgarius,* such as *Ordgarius hobsoni* and *Ordgarius sexspinosus,* spiders belonging to the genus *Argiope,* such as *Argiope amoena, Argiope minuta,* and *Argiope bruennichi,* spiders belonging to the genus *Arachnura,* such as *Arachnura logio,* spiders belonging to the genus *Acusilas,* such as Acusilas coccineus, spiders belonging to the genus *Cytophora,* such as *Cyrtophora moluccensis, Cyrtophora exanthematica,* and *Cyrtophora unicolor,* spiders belonging to the genus *Poltys,* such as *Poltys illepidus,* spiders belonging to the genus *Cyclosa,* such as *Cyclosa octotuberculata, Cyclosa sedeculata, Cyclosa vallata,* and *Cyclosa atrata,* and spiders belonging to the genus *Chorizopes,* such as *Chorizopes nipponicus,* and spider silk proteins produced by spiders belonging to the family *Tetragnathidae,* such as spiders belonging to the genus *Tetragnatha,* such as *Tetragnatha praedonia, Tetragnatha maxillosa, Tetragnatha extensa,* and *Tetragnatha squamata,* spiders belonging to the genus *Leucauge,* such as *Leucauge magnifica, Leucauge blanda,* and *Leucauge subblanda,* spiders belonging to the genus *Nephila,* such as *Nephila clavata* and *Nephila pilipes,* spiders belonging to the genus *Menosira,* such as *Menosira ornata,* spiders belonging to the genus *Dyschiriognatha,* such as *Dyschiriognatha tenera,* spiders belonging to the genus *Latrodectus,* such as *Latrodectus mactans, Latrodectus hasseltii, Latrodectus geometricus,* and *Latrodectus tredecimguttatus,* and spiders belonging to the genus *Euprosthenops.* Examples of the spider silk protein include dragline silk proteins such as MaSps (MaSp1 and MaSp2) and ADFs (ADF3 and ADF4) and MiSps (MiSp1 and MiSp2).

More specific examples of the fibroin produced by spiders include fibroin-3 (adf-3) [derived from *Araneus diadematus*] (GenBank Accession Number AAC47010 (amino acid sequence), U47855 (base sequence)), fibroin-4 (adf-4) [derived from *Araneus diadematus*] (GenBank Accession Number AAC47011 (amino acid sequence), U47856 (base sequence)), dragline silk protein spidroin 1 [derived from *Nephila clavipes*] (GenBank Accession Number AAC04504 (amino acid sequence), U37520 (base sequence)), major ampullate spidroin 1 [derived from *Latrodectus hesperus*] (GenBank Accession Number ABR68856 (amino acid sequence), EF595246 (base sequence)), dragline silk protein spidroin 2 [derived from *Nephila clavata*] (GenBank Accession Number AAL32472 (amino acid sequence), AF441245 (base sequence)), major anpullate spidroin 1 [derived from *Euprosthenops australis*] (GenBank Accession Number CAJ00428 (amino acid sequence), AJ973155 (base sequence)) and major ampullate spidroin 2 [*Euprosthenops australis*] (GenBank Accession Number CAM32249.1 (amino acid sequence), AM490169 (base sequence)), minor ampullate silk protein 1 [*Nephila clavipes*] (GenBank Accession Number AAC14589.1 (amino acid sequence), minor ampullate silk protein 2 [*Nephila clavipes*] (GenBank Accession Number AAC14591.1 (amino acid sequence)), and minor ampullate spidroin-like protein [*Nephilengys cruentata*] (GenBank Accession Number ABR37278.1 (amino acid sequence)).

More specific examples of the naturally derived fibroin include fibroin having sequence information registered in NCBI GenBank. Among sequence information registered in NCBI GenBank, for example, the sequence information of the fibroin can be checked by extracting sequences with a keyword of spidroin, ampullate, fibroin, "silk and polypeptide", or "silk and protein" in DEFINITION from sequences containing INV as DIVISION, by extracting sequences having a specific product character string from CDS, and by extracting sequences having a character string specific to TISSUE TYPE from SOURCE.

The protein may be modified fibroin. The "modified fibroin" in the present specification represents artificially produced fibroin (artificial fibroin). The modified fibroin may be fibroin having an amino acid sequence different from or identical to an amino acid sequence of naturally derived fibroin. When the protein includes modified fibroin, fibrillation of the polypeptide derivative is facilitated. Thus, it is possible to obtain advantages such as improvement of a fiber forming ability when the polypeptide derivative is used as a molding material.

The modified fibroin may be a fibrous protein having a structure similar to that of naturally derived fibroin, or may be fibroin having a sequence similar to a repetitive sequence of naturally derived fibroin. The "sequence similar to the repetitive sequence of fibroin" may actually be a sequence of naturally derived fibroin, or may be a sequence similar thereto.

The "modified fibroin" may be fibroin whose amino acid sequence is modified based on naturally derived fibroin (for example, fibroin whose amino acid sequence is modified by altering a cloned gene sequence of naturally derived fibroin) or fibroin obtained by artificially designing an amino acid sequence independently of naturally derived fibroin (for example, fibroin having a desired amino acid sequence by chemically synthesizing a nucleic acid encoding the designed amino acid sequence), as long as it has the amino acid sequence specified in the present disclosure. A modified amino acid sequence of modified fibroin is also included in modified fibroin when the amino acid sequence thereof is different from the amino acid sequence of naturally derived fibroin. Examples of the modified fibroin include modified silk fibroin (fibroin obtained by modifying an amino acid sequence of a silk protein produced by silkworms), and modified spider silk fibroin (fibroin obtained by modifying an amino acid sequence of a spider silk protein produced by spiders). The modified fibroin preferably includes modified spider silk fibroin, and more preferably consists of modified spider silk fibroin.

Specific examples of the modified fibroin according to the present embodiment include modified fibroin derived from a major dragline silk protein produced in a major ampullate gland of a spider (first modified fibroin), modified fibroin having a domain sequence in which the content of glycine residues is reduced (second modified fibroin), modified fibroin having a domain sequence in which the content of an (A)ₙ motif is reduced (third modified fibroin), modified fibroin in which the content of glycine residues and the content of an (A)ₙ motif are reduced (fourth modified fibroin), modified fibroin having a domain sequence including a region locally having a high hydrophobicity index (fifth modified fibroin), and modified fibroin having a domain sequence in which the content of glutamine residues is reduced (sixth modified fibroin).

Examples of the first modified fibroin include a protein having a domain sequence represented by Formula 1: [(A)ₙ motif-REP]ₘ. In the first modified fibroin, the number of amino acid residues in the (A)ₙ motif is preferably an integer of 3 to 20, more preferably an integer of 4 to 20, still more preferably an integer of 8 to 20, even still more preferably an integer of 10 to 20, still further preferably an integer of 4 to 16, particularly preferably an integer of 8 to 16, and most preferably an integer of 10 to 16. The number of amino acid residues constituting the REP in Formula 1 in the first modified fibroin is preferably 10 to 200 residues, more preferably 10 to 150 residues, even more preferably 20 to 100 residues, and still more preferably 20 to 75 residues. In the first modified fibroin, the total number of glycine residues, serine residues, and alanine residues included in the amino acid sequence represented by Formula 1: [(A)ₙ motif-REP]ₘ is preferably 40% or more, more preferably 60% or more, and still more preferably 70% or more, with respect to the total number of amino acid residues.

The first modified fibroin may be a polypeptide having an amino acid sequence unit represented by Formula 1: [(A)ₙ motif-REP]ₘ, and having a C-terminal sequence which is an amino acid sequence set forth in any one of SEQ ID NOs: 1 to 3 or a C-terminal sequence which is an amino acid sequence having 90% or more homology with the amino acid sequence set forth in any one of SEQ ID NOs: 1 to 3.

The amino acid sequence set forth in SEQ ID NO: 1 is identical to an amino acid sequence consisting of 50 amino acid residues at the C-terminus of the amino acid sequence of ADF3 (GI: 1263287, NCBI), the amino acid sequence set forth in SEQ ID NO: 2 is identical to an amino acid sequence obtained by removing 20 amino acid residues from the C-terminus of the amino acid sequence set forth in SEQ ID NO: 1, and the amino acid sequence set forth in SEQ ID NO: 3 is identical to an amino acid sequence obtained by removing 29 residues from the C-terminus of the amino acid sequence set forth in SEQ ID NO: 1.

More specific examples of the first modified fibroin include modified fibroin having (1-i) an amino acid sequence set forth in SEQ ID NO: 4 (recombinant spider silk protein ADF3KaiLargeNRSH1) or (1-ii) an amino acid sequence having a sequence identity of 90% or higher with the amino acid sequence set forth in SEQ ID NO: 4. The sequence identity is preferably 95% or higher.

The amino acid sequence set forth in SEQ ID NO: 4 is obtained by the following mutation so that in the amino acid sequence of ADF3 having the N-terminus to which an amino acid sequence including a start codon, a His10 tag, and a human rhinovirus 3C protease (HRV3C protease) recognition site (SEQ ID NO: 5) are added, the first to thirteenth repetitive regions are roughly doubled and the translation ends at the 1,154th amino acid residue. The C-terminal amino acid sequence of the amino acid sequence set forth in SEQ ID NO: 4 is identical to the amino acid sequence set forth in SEQ ID NO: 3.

The modified fibroin of (1-i) may consist of the amino acid sequence set forth in SEQ ID NO: 4.

The domain sequence of the second modified fibroin has an amino acid sequence in which the content of glycine residues is reduced compared to the naturally derived fibroin. The second modified fibroin can be defined as fibroin having an amino acid sequence corresponding to an amino acid sequence in which at least one or a plurality of glycine residues in the REP are substituted by other amino acid residues, compared to the naturally derived fibroin.

The domain sequence of the second modified fibroin may have an amino acid sequence corresponding to an amino acid sequence in which one glycine residue in at least one or the plurality of motif sequences is substituted with another amino acid residue, in at least one motif sequence selected from GGX and GPGXX (where G represents a glycine residue, P represents a proline residue, and X represents an amino acid residue other than glycine) in the REP, as compared with naturally derived fibroin.

In the second modified fibroin, a ratio of the motif sequence described above in which a glycine residue is substituted by another amino acid residue to the total motif sequences may be 10% or more.

The second modified fibroin contains a domain sequence represented by Formula 1: [(A)ₙ motif-REP]ₘ, and may have an amino acid sequence in which z/w is 30% or more, 40% or more, 50% or more, or 50.9% or more in a case where the total number of amino acid residues in the amino acid sequence consisting of XGX (where X represents an amino acid residue other than glycine) included in all REPs in a sequence excluding the sequence from the (A)ₙ motif located at the most C-terminal side to the C-terminus of the domain sequence from the domain sequence is defined as z, and the total number of amino acid residues in the sequence excluding the sequence from the (A)ₙ motif located at the most C-terminal side to the C-terminus of the domain sequence from the domain sequence is defined as w. The number of alanine residues with respect to the total number of amino acid residues in the (A)ₙ motif is 83% or more, preferably 86% or more, more preferably 90% or more, still more preferably 95% or more, and even still more preferably 100% (meaning that the (A)ₙ motif consists of only alanine residues).

It is preferable that a content ratio of the amino acid sequence consisting of XGX in the second modified fibroin is increased by substituting one glycine residue in the GGX motif with another amino acid residue. A content ratio of the amino acid sequence consisting of GGX in the domain sequence of the second modified fibroin is preferably 30% or less, more preferably 20% or less, even more preferably 10% or less, still more preferably 6% or less, still even more preferably 4% or less, and particularly preferably 2% or less. The content ratio of the amino acid sequence consisting of GGX in the domain sequence is calculated by a method similar to the following method for calculating a content ratio of the amino acid sequence consisting of XGX (z/w).

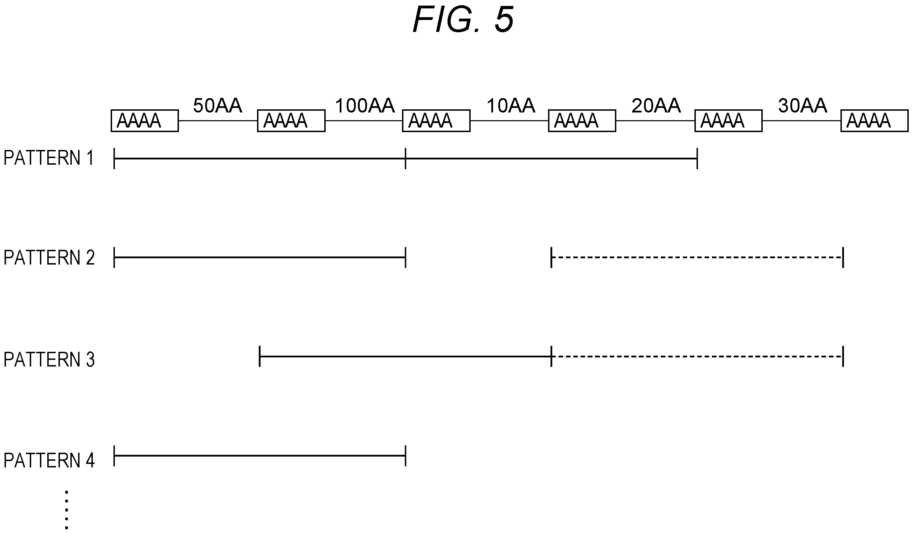

The method for calculating z/w will be described in more detail. First, the amino acid sequence consisting of XGX is extracted from all REPs included in a sequence excluding the sequence from the (A)ₙ motif located at the most C-terminal side to the C-terminus of the domain sequence from the domain sequence in the fibroin having a domain sequence represented by Formula 1: [(A)ₙ motif-REP]ₘ (modified fibroin or naturally derived fibroin). A total number of amino acid residues constituting XGX is denoted by z. For example, when 50 amino acid sequences consisting of XGX are extracted (no overlap), z is 50 × 3 = 150. For example, in an amino acid sequence consisting of XGXGX, one X (X in the center) is included in two XGX's, and the overlapping portion is subtracted to calculate z (that is, there are 5 amino acid residues in XGXGX). w is a total number of amino acid residues contained in a sequence excluding the sequence from the (A)ₙ motif located at the most C-terminal side to the C-terminus of the domain sequence from the domain sequence. For example, in the case of the domain sequence illustrated in Fig. 5, w is 4 + 50 + 4 + 100 + 4 + 10 + 4 + 20 + 4 + 30 = 230 (excluding the (A)ₙ motif located at the most C-terminal side). Next, z is divided by w to calculate z/w (%).

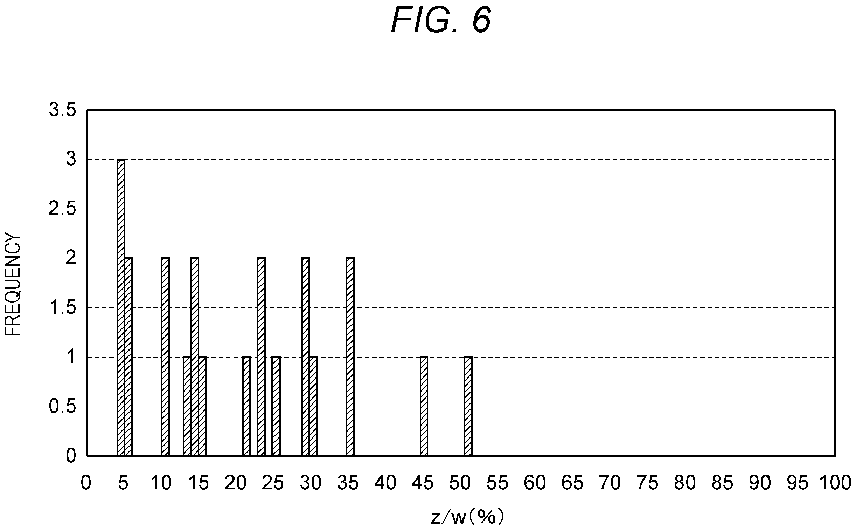

Here, z/w in the naturally derived fibroin will be described. First, fibroin whose amino acid sequence information was registered in NCBI GenBank was verified using the method exemplified above, and as a result, 663 types of fibroin (among these, 415 types were fibroin derived from spiders) were extracted. z/w was calculated by the calculation method described above from the amino acid sequences of the naturally derived fibroins which contain a domain sequence represented by Formula 1: [(A)ₙ motif-REP]ₘ and in which the content ratio of the amino acid sequence consisting of GGX in the fibroin is 6% or less, among all the extracted fibroins. The results are illustrated in Fig. 6. In Fig. 6, the horizontal axis represents z/w (%), and the vertical axis represents a frequency. As is clear from Fig. 6, the values of z/w in the naturally derived fibroin are all less than 50.9% (the largest value is 50.86%).

z/w in the second modified fibroin is preferably 50.9% or more, more preferably 56.1% or more, even more preferably 58.7% or more, still more preferably 70% or more, and still even more preferably 80% or more. An upper limit of z/w is not particularly limited, and may be, for example, 95% or less.

The second modified fibroin can be obtained by, for example, performing modification so that at least a part of base sequences encoding glycine residues in a cloned gene sequence for the naturally derived fibroin are substituted so as to encode another amino acid residue. In this case, one glycine residue in a GGX motif and GPGXX motif may be selected as a glycine residue to be modified or may be substituted so that z/w becomes 50.9% or more. It is possible to obtain the second modified fibroin by, for example, designing an amino acid sequence satisfying the above aspect from the amino acid sequence of the naturally derived fibroin and chemically synthesizing a nucleic acid encoding the designed amino acid sequence. In any case, in addition to the modification corresponding to a substitution of a glycine residue in the REP in the amino acid sequence of the naturally derived fibroin with another amino acid residue, further amino acid sequence modification may be performed, which corresponds to a substitution, a deletion, an insertion, and/or an addition of one or a plurality of amino acid residues.

Another amino acid residue above is not particularly limited as long as it is an amino acid residue other than a glycine residue, and the amino acid residue is preferably a hydrophobic amino acid residue such as a valine (V) residue, a leucine (L) residue, an isoleucine (I) residue, a methionine (M) residue, a proline (P) residue, a phenylalanine (F) residue, and a tryptophan (W) residue, and a hydrophilic amino acid residue such as a glutamine (Q) residue, an asparagine (N) residue, a serine (S) residue, a lysine (K) residue, and a glutamic acid (E) residue, more preferably a valine (V) residue, a leucine (L) residue, an isoleucine (I) residue, a phenylalanine (F) residue, and a glutamine (Q) residue, and even more preferably a glutamine (Q) residue.

More specific examples of the second modified fibroin include modified fibroin having (2-i) an amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799) or (2-ii) an amino acid sequence having a sequence identity of 90% or higher with the amino acid sequence set forth in SEQ ID NO: 6, SEQ ID NO: 7, SEQ ID NO: 8, or SEQ ID NO: 9.

The modified fibroin of (2-i) will be described. The amino acid sequence set forth in SEQ ID NO: 6 is obtained by substituting all GGX's in the REP in an amino acid sequence set forth in SEQ ID NO: 10 (Met-PRT313), which corresponds to the naturally derived fibroin, with GQX's. The amino acid sequence set forth in SEQ ID NO: 7 is obtained by deleting every other two (A)ₙ motifs from the N-terminal side to the C-terminal side from the amino acid sequence set forth in SEQ ID NO: 6 and further inserting one [(A)ₙ motif-REP] before the C-terminal sequence. The amino acid sequence set forth in SEQ ID NO: 8 is obtained by inserting two alanine residues on the C-terminal side of each (A)ₙ motif of the amino acid sequence set forth in SEQ ID NO: 7 and further substituting a part of glutamine (Q) residues with a serine (S) residue to delete a part of amino acids on the C-terminal side so as to be almost the same as the molecular weight of SEQ ID NO: 7. The amino acid sequence set forth in SEQ ID NO: 9 is obtained by adding a predetermined hinge sequence and a His tag sequence to the C-terminus of a sequence obtained by repeating a region of 20 domain sequences (where several amino acid residues on the C-terminal side of the region are substituted) present in the amino acid sequence set forth in SEQ ID NO: 7 four times.

A value of z/w in the amino acid sequence set forth in SEQ ID NO: 10 (corresponding to naturally derived fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. The values of x/y of the amino acid sequences set forth in SEQ ID NO: 10, SEQ ID NO: 6, SEQ ID NO: 7, SEQ ID NO: 8, and SEQ ID NO: 9 at a Giza ratio (described below) of 1:1.8 to 11.3 are 15.0%, 15.0%, 93.4%, 92.7%, and 89.8%, respectively.

The modified fibroin of (2-i) may consist of the amino acid sequence set forth in SEQ ID NO: 6, SEQ ID NO: 7, SEQ ID NO: 8, or SEQ ID NO: 9.

The modified fibroin of (2-ii) contains an amino acid sequence having a sequence identity of 90% or higher with the amino acid sequence set forth in SEQ ID NO: 6, SEQ ID NO: 7, SEQ ID NO: 8, or SEQ ID NO: 9. The modified fibroin of (2-ii) is also a protein having a domain sequence represented by Formula 1: [(A)ₙ motif-REP]ₘ. The sequence identity is preferably 95% or higher.

It is preferable that the modified fibroin of (2-ii) has 90% or higher sequence identity with the amino acid sequence set forth in SEQ ID NO: 6, SEQ ID NO: 7, SEQ ID NO: 8, or SEQ ID NO: 9, and z/w is 50.9% or more in a case where the total number of amino acid residues in the amino acid sequence consisting of XGX (where X represents an amino acid residue other than glycine) included in the REP is defined as z, and the total number of amino acid residues of the REP in the domain sequence is defined as w.

The second modified fibroin may have a tag sequence at one or both of the N-terminus and the C-terminus thereof. By having a tag sequence, isolation, immobilization, detection, visualization, and the like of the modified fibroin become possible.

Examples of the tag sequence include an affinity tag using specific affinity (binding properties or affinity) to another molecule. A specific example of the affinity tag includes a histidine tag (His tag). The His-tag is a short peptide in which about 4 to 10 histidine residues are lined up and can be used for isolating modified fibroin by chelating metal chromatography, since it has a property of specifically binding to metal ions such as nickel. Specific examples of the tag sequence include an amino acid sequence set forth in SEQ ID NO: 11 (an amino acid sequence including a His-tag sequence and a hinge sequence).

As the tag sequence, it is possible to employ a tag sequence such as glutathione-S-transferase (GST) that specifically binds to glutathione or a maltose binding protein (MBP) that specifically binds to maltose.

As the tag sequence, an "epitope tag" utilizing an antigen-antibody reaction can be employed. Adding a peptide (epitope) exhibiting antigenicity as a tag sequence allows binding of an antibody to the epitope. Examples of the epitope tag include an HA (a peptide sequence of influenza virus hemagglutinin) tag, a myc tag, a FLAG tag, and the like. The use of the epitope tag allows purification of the modified fibroin to be easily performed with high specificity.

It is possible to use a tag sequence which is cleaved with a specific protease as the tag sequence. By treating a protein adsorbed via the tag sequence with a protease, the modified fibroin from which the tag sequence is cleaved can be recovered.

More specific examples of the modified fibroin having a tag sequence include modified fibroin having (2-iii) an amino acid sequence set forth in SEQ ID NO: 12 (PRT380), SEQ ID NO: 13 (PRT410), SEQ ID NO: 14 (PRT525), or SEQ ID NO: 15 (PRT799) or (2-iv) an amino acid sequence having a sequence identity of 90% or higher with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are obtained by adding the amino acid sequence set forth in SEQ ID NO: 11 (which has a His-tag sequence and a hinge sequence) to the N-termini of the amino acid sequences set forth in SEQ ID NO: 10, SEQ ID NO: 6, SEQ ID NO: 7, SEQ ID NO: 8, and SEQ ID NO: 9, respectively.

The modified fibroin of (2-iii) may consist of the amino acid sequence set forth in SEQ ID NO: 12, SEQ ID NO: 13, SEQ ID NO: 14, or SEQ ID NO: 15.

The modified fibroin of (2-iv) contains an amino acid sequence having a sequence identity of 90% or higher with the amino acid sequence set forth in SEQ ID NO: 12, SEQ ID NO: 13, SEQ ID NO: 14, or SEQ ID NO: 15. The modified fibroin of (2-iv) is also a protein having a domain sequence represented by Formula 1: [(A)ₙ motif-REP]ₘ. The sequence identity is preferably 95% or higher.

It is preferable that the modified fibroin of (2-iv) has 90% or higher sequence identity with the amino acid sequence set forth in SEQ ID NO: 12, SEQ ID NO: 13, SEQ ID NO: 14, or SEQ ID NO: 15, and z/w is 50.9% or more in a case where the total number of amino acid residues in the amino acid sequence consisting of XGX (where X represents the amino acid residue other than glycine) in the REP is defined as z, and the total number of amino acid residues in the REP in the domain sequence is defined as w.

The second modified fibroin may contain a secretory signal for releasing a protein produced in an artificial protein production system to the outside of a host. A sequence of the secretory signal is designed appropriately depending on the type of the host.

The domain sequence of the third modified fibroin has an amino acid sequence in which the content of the (A)ₙ motif is reduced, as compared with naturally derived fibroin. It can be said that the domain sequence of the third modified fibroin has an amino acid sequence corresponding to an amino acid sequence in which at least one or a plurality of (A)ₙ motifs are deleted, as compared with naturally derived fibroin.

The third modified fibroin may have an amino acid sequence corresponding to an amino acid sequence in which 10 to 40% of the (A)ₙ motifs are deleted from naturally derived fibroin.

The domain sequence of the third modified fibroin may have an amino acid sequence corresponding to an amino acid sequence in which at least one (A)ₙ motif of every one to three (A)ₙ motifs is deleted from the N-terminal side to the C-terminal side, as compared with naturally derived fibroin.

The domain sequence of the third modified fibroin may have an amino acid sequence corresponding to an amino acid sequence in which deletion of at least two consecutive (A)ₙ motifs and deletion of one (A)ₙ motif are repeated in this order from the N-terminal side to the C-terminal side, as compared with naturally derived fibroin.

The third modified fibroin may have a domain sequence having an amino acid sequence corresponding to an amino acid sequence in which at least (A)ₙ motif every other two positions is deleted from the N-terminal side to the C-terminal side.

The third modified fibroin contains a domain sequence represented by Formula 1: [(A)ₙ motif-REP]ₘ, and may have an amino acid sequence in which x/y is 20% or more, 30% or more, 40% or more, or 50% or more in a case where the numbers of amino acid residues in REPs of two adjacent [(A)ₙ motif-REP] units are sequentially compared from the N-terminal side to the C-terminal side, and the number of amino acid residues in one REP having a smaller number of amino acid residues is defined as 1, the maximum value of the total value of the number of amino acid residues in the two adjacent [(A)ₙ motif-REP] units where the ratio of the number of amino acid residues in the other REPs is 1.8 to 11.3 is defined as x, and the total number of amino acid residues in the domain sequence is defined as y. The number of alanine residues with respect to the total number of amino acid residues in the (A)ₙ motif is 83% or more, preferably 86% or more, more preferably 90% or more, still more preferably 95% or more, and even still more preferably 100% (meaning that the (A)ₙ motif consists of only alanine residues).

A method of calculating x/y will be described in more detail with reference to Fig. 5. Fig. 5 illustrates a domain sequence excluding the N-terminal sequence and the C-terminal sequence from the modified fibroin. The domain sequence has a sequence of (A)ₙ motif-first REP (50 amino acid residues)-(A)ₙ motif-second REP (100 amino acid residues)-(A)ₙ motif-third REP (10 amino acid residues)-(A)ₙ motif-fourth REP (20 amino acid residues)-(A)ₙ motiffifth REP (30 amino acid residues)-(A)ₙ motif from the N-terminal side (left side).

The two adjacent [(A)ₙ motif-REP] units are sequentially selected from the N-terminal side to the C-terminal side so as not to overlap. In this case, an unselected [(A)ₙ motif-REP] unit may exist. Fig. 5 illustrates a pattern 1 (a comparison between a first REP and a second REP and a comparison between a third REP and a fourth REP), a pattern 2 (a comparison between a first REP and a second REP and a comparison between a fourth REP and a fifth REP), a pattern 3 (a comparison between a second REP and a third REP and a comparison between a fourth REP and a fifth REP), and a pattern 4 (a comparison between a first REP and a second REP). Note that [(A)ₙ motif-REP] units may be selected in other ways.

Subsequently, the number of amino acid residues of each REP in the selected two adjacent [(A)ₙ motif-REP] units is compared for each pattern. The comparison is performed by setting the smaller number of amino acid residues as 1 and determining the ratio of the number of amino acid residues in the other REPs. For example, comparing the first REP (50 amino acid residues) and the second REP (100 amino acid residues), a ratio of amino acid residues in the second REP is 100/50 = 2, defining the first REP having fewer amino acid residues as 1.
Similarly, comparing the fourth REP (20 amino acid residues) and the fifth REP (30 amino acid residues), a ratio of amino acid residues in the fifth REP is 30/20 = 1.5, defining the fourth REP having fewer amino acid residues as 1.

In Fig. 5, a set of [(A)ₙ motif-REP] units in which the ratio of the number of amino acid residues in the other REPs when one REP having a smaller number of amino acid residues is 1 is 1.8 to 11.3 is indicated by a solid line. In the present specification, the ratio is referred to as a Giza ratio. A set of [(A)ₙ motif-REP] units in which the ratio of the number of amino acid residues of the other REPs is less than 1.8 or more than 11.3 in a case where one REP having a smaller number of amino acid residues is defined as 1 is indicated by a broken line.

In each pattern, the number of all amino acid residues of two adjacent [(A)ₙ motif-REP] units indicated by solid lines (including not only the number of amino acid residues of the REP but also the number of amino acid residues of the (A)ₙ motif) is combined. The total values of the patterns are compared, and one that has the highest value (the maximum total value) is defined as x. In the example illustrated in Fig. 5, the total value of the pattern 1 is the maximum.

Then, x is divided by the total number of amino acid residues y of the domain sequence to calculate x/y (%).

x/y in the third modified fibroin is preferably 50% or more, more preferably 60% or more, even more preferably 65% or more, still more preferably 70% or more, still even more preferably 75% or more, and particularly preferably 80% or more. An upper limit of x/y is not particularly limited, and may be, for example, 100% or less. With a Giza ratio of 1 : 1.9 to 11.3, x/y is preferably 89.6% or more. With a Giza ratio of 1 : 1.8 to 3.4, x/y is preferably 77.1% or more. When a Giza ratio is 1 : 1.9 to 8.4, x/y is preferably 75.9% or more. With a Giza ratio of 1 : 1.9 to 4.1, x/y is preferably 64.2% or more.

In a case where the third modified fibroin is modified fibroin in which at least seven of a plurality of (A)ₙ motifs in the domain sequence consist of only alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited as long as the value is 100% or less.

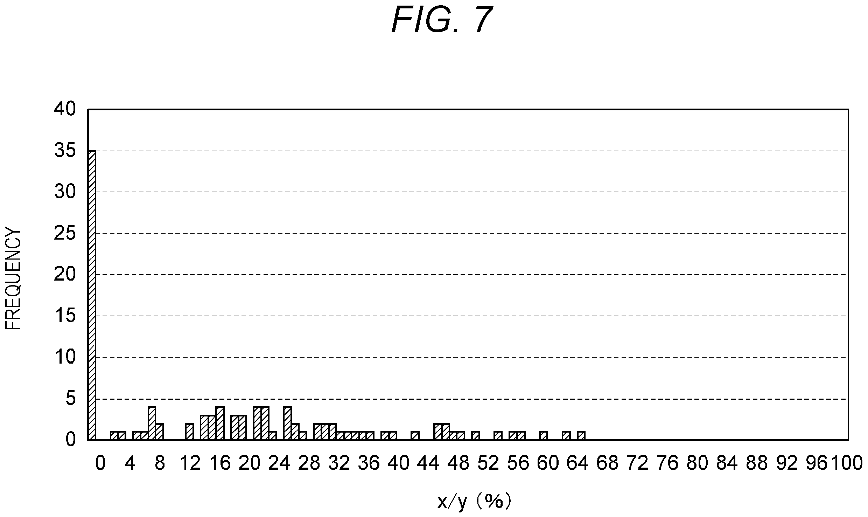

Here, x/y in the naturally derived fibroin will be described. First, fibroin whose amino acid sequence information was registered in NCBI GenBank was verified using the method exemplified above, and as a result, 663 types of fibroin (among these, 415 types were fibroin derived from spiders) were extracted. The values of x/y were calculated by the calculation method described above, from amino acid sequences of naturally derived fibroins consisting of a domain sequence represented by Formula 1: [(A)ₙ motif-REP]ₘ, among all the extracted fibroins. The results in a case where the Giza ratio is 1:1.9 to 4.1 are illustrated in Fig. 7.

In Fig. 7, the horizontal axis represents x/y (%), and the vertical axis represents a frequency. As is clear from Fig. 7, the values of x/y in the naturally derived fibroin are all less than 64.2% (the largest value is 64.14%) .

The third modified fibroin can be obtained from, for example, a cloned gene sequence of naturally derived fibroin, by deleting one or a plurality of sequences encoding an (A)ₙ motif so that x/y is 64.2% or more. For example, the third modified fibroin can also be obtained, from the amino acid sequence of naturally derived fibroin, by designing an amino acid sequence corresponding to an amino acid sequence in which one or a plurality of (A)ₙ motifs are deleted so that x/y is 64.2% or more, and chemically synthesizing a nucleic acid encoding the designed amino acid sequence. In any case, in addition to the modification corresponding to deletion of the (A)ₙ motif from the amino acid sequence of naturally derived fibroin, modification of the amino acid sequence corresponding to substitution, deletion, insertion, and/or addition of one or a plurality of amino acid residues may be performed.

More specific examples of the third modified fibroin include modified fibroin having (3-i) an amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799) or (3-ii) an amino acid sequence having a sequence identity of 90% or higher with the amino acid sequence set forth in SEQ ID NO: 17, SEQ ID NO: 7, SEQ ID NO: 8, or SEQ ID NO: 9.

The modified fibroin of (3-i) will be described. The amino acid sequence set forth in SEQ ID NO: 17 is obtained by deleting every other two (A)ₙ motifs from the N-terminal side to the C-terminal side from the amino acid sequence set forth in SEQ ID NO: 10 (Met-PRT313) corresponding to naturally derived fibroin and further inserting one [(A)ₙ motif-REP] before the C-terminal sequence. The amino acid sequence set forth in SEQ ID NO: 7, SEQ ID NO: 8, or SEQ ID NO: 9 is as described in the second modified fibroin.

A value of x/y in the amino acid sequence set forth in SEQ ID NO: 10 (corresponding to the naturally derived fibroin) at a Giza ratio of 1 : 1.8 to 11.3 is 15.0%. Values of x/y in the amino acid sequence set forth in SEQ ID NO: 17 and the amino acid sequence set forth in SEQ ID NO: 7 are both 93.4%. A value of x/y in the amino acid sequence set forth in SEQ ID NO: 8 is 92.7%. A value of x/y in the amino acid sequence set forth in SEQ ID NO: 9 is 89.8%. Values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin of (3-i) may consist of the amino acid sequence set forth in SEQ ID NO: 17, SEQ ID NO: 7, SEQ ID NO: 8, or SEQ ID NO: 9.

The modified fibroin of (3-ii) contains an amino acid sequence having a sequence identity of 90% or higher with the amino acid sequence set forth in SEQ ID NO: 17, SEQ ID NO: 7, SEQ ID NO: 8, or SEQ ID NO: 9. The modified fibroin of (3-ii) is also a protein having a domain sequence represented by Formula 1: [(A)ₙ motif-REP]ₘ. The sequence identity is preferably 95% or higher.

It is preferable that the modified fibroin of (3-ii) has 90% or higher sequence identity with the amino acid sequence set forth in SEQ ID NO: 17, SEQ ID NO: 7, SEQ ID NO: 8, or SEQ ID NO: 9, and x/y is 64.2% or more in a case where the numbers of amino acid residues in REPs of two adjacent [(A)ₙ motif-REP] units are sequentially compared from the N-terminal side to the C-terminal side, and the number of amino acid residues in one REP having a small number of amino acid residues is defined as 1, the maximum value of the total value of the number of amino acid residues in the two adjacent [(A)ₙ motif-REP] units where the ratio of the number of amino acid residues in the other REPs is 1.8 to 11.3 (the Giza ratio is 1 : 1.8 to 11.3) is defined as x, and the total number of amino acid residues in the domain sequence is defined as y.

The third modified fibroin may have the tag sequence described above at one or both of the N-terminus and the C-terminus thereof.

More specific examples of the modified fibroin having a tag sequence include modified fibroin having (3-iii) an amino acid sequence set forth in SEQ ID NO: 18 (PRT399), SEQ ID NO: 13 (PRT410), SEQ ID NO: 14 (PRT525), or SEQ ID NO: 15 (PRT799) or (3-iv) an amino acid sequence having a sequence identity of 90% or higher with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are obtained by adding the amino acid sequence set forth in SEQ ID NO: 11 (which has a His-tag sequence and a hinge sequence) to the N-termini of the amino acid sequences set forth in SEQ ID NO: 17, SEQ ID NO: 7, SEQ ID NO: 8, and SEQ ID NO: 9, respectively.

The modified fibroin of (3-iii) may consist of the amino acid sequence set forth in SEQ ID NO: 18, SEQ ID NO: 13, SEQ ID NO: 14, or SEQ ID NO: 15.

The modified fibroin of (3-iv) contains an amino acid sequence having a sequence identity of 90% or higher with the amino acid sequence set forth in SEQ ID NO: 18, SEQ ID NO: 13, SEQ ID NO: 14, or SEQ ID NO: 15. The modified fibroin of (3-iv) is also a protein having a domain sequence represented by Formula 1: [(A)ₙ motif-REP]ₘ. The sequence identity is preferably 95% or higher.

It is preferable that the modified fibroin of (3-iv) has 90% or higher sequence identity with the amino acid sequence set forth in SEQ ID NO: 18, SEQ ID NO: 13, SEQ ID NO: 14, or SEQ ID NO: 15, and x/y is 64.2% or more in a case where the number of amino acid residues in REPs in two adjacent [(A)ₙ motif-REP] units are sequentially compared from the N-terminal side to the C-terminal side, and the number of amino acid residues in one REP having a small number of amino acid residues is defined as 1, the maximum value of the total value of the number of amino acid residues in the two adjacent [(A)ₙ motif-REP] units where the ratio of the number of amino acid residues in the other REPs is 1.8 to 11.3 is defined as x, and the total number of amino acid residues in the domain sequence is defined as y.

The third modified fibroin may contain a secretory signal for releasing a protein produced in an artificial protein production system to the outside of a host. A sequence of the secretory signal is designed appropriately depending on the type of the host.

The domain sequence of the fourth modified fibroin has an amino acid sequence in which the content of an (A)ₙ motif and the content of glycine residues are reduced, as compared with naturally derived fibroin. It can be said that the domain sequence of the fourth modified fibroin has an amino acid sequence corresponding to an amino acid sequence in which at least one or a plurality of (A)ₙ motifs are deleted and at least one or a plurality of glycine residues in the REP are substituted with another amino acid residue, as compared with naturally derived fibroin. That is, the fourth modified fibroin is modified fibroin having characteristics of both the second modified fibroin and the third modified fibroin described above. Specific aspects thereof and the like are as in the descriptions for the second modified fibroin and the third modified fibroin.

More specific examples of the fourth modified fibroin include modified fibroin having (4-i) an amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799) or (4-ii) an amino acid sequence having a sequence identity of 90% or higher with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the modified fibroin containing the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The domain sequence of the fifth modified fibroin may have an amino acid sequence containing a region in which a hydrophobicity index is locally high, which corresponds to an amino acid sequence in which one or a plurality of amino acid residues in the REP are substituted by amino acid residues having a high hydrophobicity index, and/or an amino acid sequence in which one or a plurality of amino acid residues having a high hydrophobicity index are inserted into REP, compared to the naturally derived fibroin.

The region locally having a high hydrophobicity index preferably includes two to four consecutive amino acid residues.

The amino acid residue having a high hydrophobicity index is more preferably an amino acid residue selected from isoleucine (I), valine (V), leucine (L), phenylalanine (F), cysteine (C), methionine (M), and alanine (A).

In addition to the modification corresponding to a substitution of one or a plurality of amino acid residues in the REP with amino acid residues having a high hydrophobicity index and/or an insertion of one or a plurality of amino acid residues having a high hydrophobicity index into REP, compared to the naturally derived fibroin, further amino acid sequence modification may be performed on the fifth modified fibroin, which corresponds to a substitution, a deletion, an insertion, and/or an addition of one or a plurality of amino acid residues, compared to the naturally derived fibroin.

The fifth modified fibroin can be obtained from, for example, a cloned gene sequence for the naturally derived fibroin by substituting one or a plurality of hydrophilic amino acid residues (for example, amino acid residues having a negative value of hydrophobicity index) in the REP with hydrophobic amino acid residues (for example, amino acid residues having a positive value of hydrophobicity index) and/or by inserting one or a plurality of hydrophobic amino acid residues into REP. It is possible to obtain the fifth modified fibroin by, for example, designing an amino acid sequence corresponding to an amino acid sequence in which one or a plurality of hydrophilic amino acid residues in the REP in the amino acid sequence of the naturally derived fibroin are substituted by hydrophobic amino acid residues and/or an amino acid sequence in which one or a plurality of hydrophobic amino acid residues are inserted into REP in the amino acid sequence of the naturally derived fibroin and chemically synthesizing a nucleic acid encoding the designed amino acid sequence. In any case, in addition to the modification corresponding to a substitution of one or a plurality of hydrophilic amino acid residues in the REP in the amino acid sequence of the naturally derived fibroin with hydrophobic amino acid residues and/or an insertion of one or a plurality of hydrophobic amino acid residues into REP in the amino acid sequence of the naturally derived fibroin, further amino acid sequence modification may be performed, which corresponds to a substitution, a deletion, an insertion, and/or an addition of one or a plurality of amino acid residues.

The fifth modified fibroin contains a domain sequence represented by Formula 1: [(A)ₙ motif-REP]ₘ, and may have an amino acid sequence in which p/q is 6.2% or more in a case where in all REPs included in a sequence excluding the sequence from the (A)ₙ motif located at the most C-terminal side to the C-terminus of the domain sequence from the domain sequence, the total number of amino acid residues included in a region where the average value of hydrophobicity indices of four consecutive amino acid residues is 2.6 or more is defined as p, and the total number of amino acid residues included in the sequence excluding the sequence from the (A)ₙ motif located at the most C-terminal side to the C-terminus of the domain sequence from the domain sequence is defined as q.

As the hydrophobicity index of an amino acid residue, a known index is used herein (Hydropathy index: Kyte J, & Doolittle R (1982)" A simple method for displaying the hydropathic character of a protein", J. Mol. Biol., 157, pp. 105-132). Specifically, the hydrophobicity indices (hydropathy index, hereinafter also referred to as *"*HI*"*) of amino acids are as represented in Table 1 below.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. In the calculation, a sequence excluding the sequence from the (A)ₙ motif located at the most C-terminal side to the C-terminus of the domain sequence from the domain sequence represented by Formula 1 [(A)ₙ motif-REP]ₘ (hereinafter also referred to as "sequence A") is used. First, in all REPs included in the sequence A, an average hydrophobicity index of four consecutive amino acid residues is calculated. An average hydrophobicity index is determined by dividing a sum of HI of hydrophobicity indices of amino acid residues in four consecutive amino acid residues by 4 (the number of amino acid residues). An average hydrophobicity index is determined for all four consecutive amino acid residues (each amino acid residue is used for calculating an average one to four times). Then, a region where four consecutive amino acid residues have an average hydrophobicity index of 2.6 or more is determined. Even when a certain amino acid residue corresponds to a plurality of *"*four consecutive amino acid residues having an average hydrophobicity index of 2.6 or more*"*, the region is regarded as including one amino acid residue. The total number of amino acid residues included in the region is p. The total number of amino acid residues included in the sequence A is q.

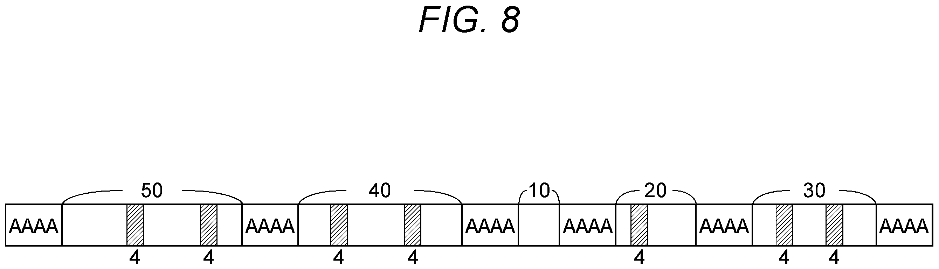

For example, in a case where *"*four consecutive amino acid residues having an average hydrophobicity index of 2.6 or more*"* are extracted from 20 places (no overlap), there are 20 sets of four consecutive amino acid residues (no overlap) in a region where four consecutive amino acid residues have an average hydrophobicity index of 2.6 or more, and thus, *"*p*"* is 20 × 4 = 80. For example, in a case where one amino acid residue overlaps within two sets of *"*four consecutive amino acid residues having an average hydrophobicity index of 2.6 or more*"*, a region where four consecutive amino acid residues have an average hydrophobicity index of 2.6 or more is regarded as including seven amino acid residues (p = 2 × 4 - 1 = 7, The overlapping amino acid residue is deducted, being indicated by *"*-1*"*). For example, in the case of the domain sequence illustrated in Fig. 8, there are seven *"*four consecutive amino acid residues having an average value of the hydrophobicity indices of 2.6 or more*"* without overlapping, and thus p is 7 × 4 = 28. For example, in the case of the domain sequence illustrated in Fig. 8, q is 4 + 50 + 4 + 40 + 4 + 10 + 4 + 20 + 4 + 30 = 170 (the (A)ₙ motif located at the end of the C-terminal side is excluded). Next, p is divided by q to calculate p/q (%). In a case of Fig. 8, 28/170 = 16.47%.

p/q in the fifth modified fibroin is preferably 6.2% or more, more preferably 7% or more, even more preferably 10% or more, still more preferably 20% or more, and still even more preferably 30% or more. An upper limit of p/q is not particularly limited, and may be, for example, 45% or less.

The fifth modified fibroin can be obtained by, for example, modifying a cloned amino acid sequence of the naturally derived fibroin into an amino acid sequence containing a region in which a hydrophobicity index is locally high by substituting one or a plurality of hydrophilic amino acid residues (for example, amino acid residues having a negative value of hydrophobicity index) in the REP with hydrophobic amino acid residues (for example, amino acid residues having a positive value of hydrophobicity index) and/or by inserting one or a plurality of hydrophobic amino acid residues into REP, so that the condition of p/q is satisfied. It is possible to obtain the fifth modified fibroin by, for example, designing an amino acid sequence satisfying the condition of p/q from the amino acid sequence of the naturally derived fibroin and chemically synthesizing a nucleic acid encoding the designed amino acid sequence. In any case, in addition to the modification corresponding to a substitution of one or a plurality of amino acid residues in the REP with amino acid residues having a high hydrophobicity index and/or an insertion of one or a plurality of amino acid residues having a high hydrophobicity index into REP, compared to the naturally derived fibroin, further modification may be performed, which corresponds to a substitution, a deletion, an insertion, and/or an addition of one or a plurality of amino acid residues.

The amino acid residue having a high hydrophobicity index is not particularly limited but is preferably isoleucine (I), valine (V), leucine (L), phenylalanine (F), cysteine (C), methionine (M), and alanine (A). Among these examples, valine (V), leucine (L), and isoleucine (I) are more preferable.

More specific examples of the fifth modified fibroin include modified fibroin having (5-i) an amino acid sequence set forth in SEQ ID NO: 19 (Met-PRT720), SEQ ID NO: 20 (Met-PRT665), or SEQ ID NO: 21 (Met-PRT666) or (5-ii) an amino acid sequence having a sequence identity of 90% or higher with the amino acid sequence set forth in SEQ ID NO: 19, SEQ ID NO: 20, or SEQ ID NO: 21.

The modified fibroin of (5-i) will be described. The amino acid sequence set forth in SEQ ID NO: 19 is obtained by inserting amino acid sequences consisting of three amino acid residues (VLI) at two sites for each REP in the amino acid sequence set forth in SEQ ID NO: 7 (Met-PRT410) excluding the terminal domain sequence on the C-terminal side, substituting a part of glutamine (Q) residues with serine (S) residues, and deleting a part of amino acids on the C-terminal side. The amino acid sequence set forth in SEQ ID NO: 20 is obtained by inserting an amino acid sequence consisting of three amino acid residues (VLI) at one site for each REP in the amino acid sequence set forth in SEQ ID NO: 8 (Met-PRT525). The amino acid sequence set forth in SEQ ID NO: 21 is obtained by inserting amino acid sequences consisting of three amino acid residues (VLI) at two sites for each REP in the amino acid sequence set forth in SEQ ID NO: 8.

The modified fibroin of (5-i) may consist of the amino acid sequence set forth in SEQ ID NO: 19, SEQ ID NO: 20, or SEQ ID NO: 21.

The modified fibroin of (5-ii) contains an amino acid sequence having a sequence identity of 90% or higher with the amino acid sequence set forth in SEQ ID NO: 19, SEQ ID NO: 20, or SEQ ID NO: 21. The modified fibroin of (5-ii) is also a protein having a domain sequence represented by Formula 1: [(A)ₙ motif-REP]ₘ. The sequence identity is preferably 95% or higher.

It is preferable that the modified fibroin of (5-ii) has 90% or higher sequence identity with the amino acid sequence set forth in SEQ ID NO: 19, SEQ ID NO: 20, or SEQ ID NO: 21, and p/q is 6.2% or more in a case where in all REPs included in a sequence excluding the sequence from the (A)ₙ motif located at the most C-terminal side to the C-terminus of the domain sequence from the domain sequence, the total number of amino acid residues included in a region where the average value of hydrophobicity indices of four consecutive amino acid residues is 2.6 or more is defined as p, and the total number of amino acid residues included in the sequence excluding the sequence from the (A)ₙ motif located at the most the C-terminal side to the C-terminus of the domain sequence from the domain sequence is defined as q.

The fifth modified fibroin may have a tag sequence at one or both of the N-terminus and the C-terminus thereof.

More specific examples of the modified fibroin having a tag sequence include modified fibroin having (5-iii) an amino acid sequence set forth in SEQ ID NO: 22 (PRT720), SEQ ID NO: 23 (PRT665), or SEQ ID NO: 24 (PRT666) or (5-iv) an amino acid sequence having a sequence identity of 90% or higher with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are obtained by adding the amino acid sequence set forth in SEQ ID NO: 11 (which has a His-tag sequence and a hinge sequence) to the N-termini of the amino acid sequences set forth in SEQ ID NO: 19, SEQ ID NO: 20, and SEQ ID NO: 21, respectively.

The modified fibroin of (5-iii) may consist of the amino acid sequence set forth in SEQ ID NO: 22, SEQ ID NO: 23, or SEQ ID NO: 24.

The modified fibroin of (5-iv) contains an amino acid sequence having a sequence identity of 90% or higher with the amino acid sequence set forth in SEQ ID NO: 22, SEQ ID NO: 23, or SEQ ID NO: 24. The modified fibroin of (5-iv) is also a protein having a domain sequence represented by Formula 1: [(A)ₙ motif-REP]ₘ. The sequence identity is preferably 95% or higher.

It is preferable that the modified fibroin of (5-iv) has 90% or more sequence identity with the amino acid sequence set forth in SEQ ID NO: 22, SEQ ID NO: 23, or SEQ ID NO: 24, and p/q is 6.2% or more in a case where in all REPs included in a sequence excluding the sequence from the (A)ₙ motif located at the most C-terminal side to the C-terminus of the domain sequence from the domain sequence, the total number of amino acid residues included in a region where the average value of hydrophobicity indices of four consecutive amino acid residues is 2.6 or more is defined as p, and the total number of amino acid residues included in the sequence excluding the sequence from the (A)ₙ motif located at the most C-terminal side to the C-terminus of the domain sequence from the domain sequence is defined as q.

The fifth modified fibroin may contain a secretory signal for releasing a protein produced in an artificial protein production system to the outside of a host. A sequence of the secretory signal is designed appropriately depending on the type of the host.

The sixth modified fibroin has an amino acid sequence in which a content of glutamine residues is reduced, compared to the naturally derived fibroin.

It is preferable that the sixth modified fibroin contains at least one motif selected from a GGX motif and a GPGXX motif in the amino acid sequence of REP.

In a case where the sixth modified fibroin contains the GPGXX motif in the REP, a content rate of the GPGXX motifs is generally 1% or more. The content rate of the GPGXX motif may be 5% or more and is preferably 10% or more. An upper limit of a content rate of the GPGXX motif is not particularly limited and may be 50% or less or 30% or less.

In the present specification, the *"*content rate of the GPGXX motif*"* is a value calculated by the following method.

The content rate of the GPGXX motif in fibroin (modified fibroin or naturally derived fibroin) having a domain sequence represented by Formula 1: [(A)ₙ motif-REP]ₘ or Formula 2: [(A)ₙ motif-REP]ₘ-(A)ₙ motif is calculated as s/t, in a case where the number obtained by tripling the total number of GPGXX motifs in regions of all REPs included in a sequence excluding the sequence from the (A)ₙ motif located at the most C-terminal side to the C-terminus of the domain sequence from the domain sequence (that is, corresponding to the total number of G and P in the GPGXX motifs) is defined as s, and the total number of amino acid residues in all REPs excluding a sequence from the (A)ₙ motif located at the most C-terminal side to the C-terminus of the domain sequence from the domain sequence and further excluding the (A)ₙ motifs is defined as t.

In the calculation of the content rate of the GPGXX motif, the *"*sequence excluding the sequence from the (A)ₙ motif located at the most C-terminal side to the C-terminus of the domain sequence from the domain sequence" is used to exclude the effect occurring due to the fact that the *"*sequence from the (A)ₙ motif located at the most C-terminal side to the C-terminus of the domain sequence*"* (a sequence corresponding to REP) may include a sequence having a low correlation with the sequence characteristic of fibroin, which influences the calculation result of the content rate of the GPGXX motif in a case where m is small (that is, in a case where the domain sequence is short). In a case where the *"*GPGXX motif*"* is located at the C-terminus of an REP, even when *"*XX*"* is *"*AA*"*, for example, it is regarded as the *"*GPGXX motif*"*.

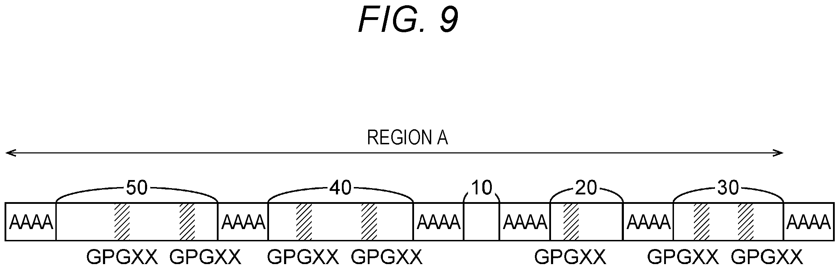

Fig. 9 is a schematic view illustrating an example of a domain sequence of modified fibroin. The calculation method of the content rate of the GPGXX motif will be specifically described with reference to Fig. 9. First, in the domain sequence of the modified fibroin (*"*[(A)ₙ motif-REP]ₘ-(A)ₙ motif*"* type) illustrated in Fig. 9, since all REPs are contained in the *"*sequence excluding the sequence from the (A)ₙ motif located at the most C-terminal side to the C-terminus of the domain sequence from the domain sequence*"* (the sequence indicated by the *"*region A*"* in Fig. 9), the number of GPGXX motifs for calculating s is 7, and s is 7 × 3 = 21. Similarly, all REPs are included in the sequence excluding the sequence from the (A)ₙ motif located at the most C-terminal side to the C-terminus of the domain sequence from the domain sequence*"* (the sequence indicated by the *"*region A*"* in Fig. 9). Thus, the total number t of amino acid residues in all REPs further excluding the (A)ₙ motifs from the sequence is 50 + 40 + 10 + 20 + 30 = 150. Next, s/t (%) can be calculated by dividing s by t, and in the case of the modified fibroin of Fig. 9, s/t (%) is 21/150 = 14.0%.

The content rate of glutamine residues in the sixth modified fibroin is preferably 9% or less, more preferably 7% or less, even more preferably 4% or less, and particularly preferably 0%.

Herein, the *"*content rate of glutamine residues*"* is a value calculated by the following method.

In fibroin (modified fibroin or naturally derived fibroin) having a domain sequence represented by Formula 1: [(A)ₙ motif-REP]ₘ or Formula 2: [(A)ₙ motif-REP]ₘ-(A)ₙ motif, the content rate of the glutamine residues is calculated as u/t, in which a total number of glutamine residues in regions of all REPs contained in a sequence excluding the sequence from the (A)ₙ motif located at the most C-terminal side to the C-terminus of the domain sequence from the domain sequence (sequence corresponding to the *"*region A*"* in Fig. 9) is u, and a total number of amino acid residues in all REPs excluding the sequence from the (A)ₙ motif located at the most C-terminal side to the C-terminus of the domain sequence from the domain sequence and further excluding (A)ₙ motifs is t. In the calculation of the content rate of the glutamine residues, the reason for targeting the *"*sequence excluding the sequence from the (A)ₙ motif located at the most C-terminal side to the C-terminus of the domain sequence from the domain sequence*"* is the same as the reason described above.

The domain sequence of the sixth modified fibroin may have an amino acid sequence corresponding to an amino acid sequence in which one or a plurality of glutamine residues in the REP are deleted or replaced with other amino acid residues, as compared with naturally derived fibroin.

As the *"*other amino acid residues*"*, any amino acid residue other than the glutamine residue can be used, but an amino acid residue having a higher hydrophobicity index than the glutamine residue is preferable. The hydrophobicity index of each amino acid residue is shown in Table 1.

As shown in Table 1, an example of the amino acid residue having a higher hydrophobicity index than that of the glutamine residue includes one selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these examples, the amino acid residue is more preferably one selected from isoleucine (I), valine (V), leucine (L), phenylalanine (F), cysteine (C), methionine (M), and alanine (A), and still more preferably one selected from isoleucine (I), valine (V), leucine (L), and phenylalanine (F).

The sixth modified fibroin may have a hydrophobic degree (hydropathy index) of REP of, for example, -0.80 or more, -0.70, -0.60 or more, -0.50 or more, -0.40 or more, - 0.30 or more, -0.20 or more, -0.10 or more, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit of the hydrophobic degree of each REP is not particularly limited, and may be 1.0 or less or 0.7 or less.

Herein, the *"*hydrophobic degree of REP*"* is a value calculated by the following method.

In fibroin (modified fibroin or naturally derived fibroin) having a domain sequence represented by Formula 1: [(A)ₙ motif-REP]ₘ or Formula 2: [(A)ₙ motif-REP]ₘ-(A)ₙ motif, the hydrophobic degree of REP is calculated as v/t, in which the sum of hydrophobicity indices of the amino acid residues in the regions of all REPs contained in the sequence excluding the sequence from the (A)ₙ motif located at the most C-terminal side to the C-terminus of the domain sequence from the domain sequence (sequence corresponding to the *"*region A*"* in Fig. 9) is v, and the total number of amino acid residues in all REPs excluding the sequence from the (A)ₙ motif located at the most C-terminal side to the C-terminus of the domain sequence from the domain sequence and further excluding (A)ₙ motifs is t. In the calculation of the hydrophobic degree of REP, the reason for targeting the *"*sequence excluding the sequence from the (A)ₙ motif located at the most C-terminal side to the C-terminus of the domain sequence from the domain sequence*"* is the same as the reason described above.

In addition to the modification corresponding to a deletion of one or a plurality of glutamine residues in the REP and/or a substitution of one or a plurality of glutamine residues in the REP with other amino acid residues, compared to the naturally derived fibroin, further amino acid sequence modification may be performed on the domain sequence of the sixth modified fibroin, which corresponds to a substitution, a deletion, an insertion, and/or an addition of one or a plurality of amino acid residues.

The sixth modified fibroin can be obtained from, for example, a cloned gene sequence for the naturally derived fibroin by deleting one or a plurality of glutamine residues in the REP and/or substituting one or a plurality of glutamine residues in the REP with other amino acid residues. It is possible to obtain the sixth modified fibroin by, for example, designing an amino acid sequence corresponding to an amino acid sequence in which one or a plurality of glutamine residues in REP in the amino acid sequence of the naturally derived fibroin are deleted and/or one or a plurality of glutamine residues in REP in the amino acid sequence of the naturally derived fibroin are substituted by other amino acid residues and chemically synthesizing a nucleic acid encoding the designed amino acid sequence.

More specific examples of the sixth modified fibroin include modified fibroin having (6-i) an amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028) or modified fibroin having (6-ii) an amino acid sequence having a sequence identity of 90% or higher with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-i) will be described. The amino acid sequence set forth in SEQ ID NO: 25 is obtained by substituting all QQ's in the amino acid sequence set forth in SEQ ID NO: 7 (Met-PRT410) with VL's. The amino acid sequence set forth in SEQ ID NO: 26 is obtained by substituting all QQ's in the amino acid sequence set forth in SEQ ID NO: 7 with TS's and substituting the remaining Q*'*s with A*'*s. The amino acid sequence set forth in SEQ ID NO: 27 is obtained by substituting all QQ*'*s in the amino acid sequence set forth in SEQ ID NO: 7 with VL*'*s and substituting the remaining Q*'*s with I*'*s. The amino acid sequence set forth in SEQ ID NO: 28 is obtained by substituting all QQ*'*s in the amino acid sequence set forth in SEQ ID NO: 7 with VI*'*s and substituting the remaining Q*'*s with L*'*s. The amino acid sequence set forth in SEQ ID NO: 29 is obtained by substituting all QQ*'*s in the amino acid sequence set forth in SEQ ID NO: 7 with VF*'*s and substituting the remaining Q*'*s with I*'*s.

The amino acid sequence set forth in SEQ ID NO: 30 is obtained by substituting all QQ*'*s in the amino acid sequence set forth in SEQ ID NO: 8 (Met-PRT525) with VL*'*s. The amino acid sequence set forth in SEQ ID NO: 31 is obtained by substituting all QQ*'*s in the amino acid sequence set forth in SEQ ID NO: 8 with VL*'*s and substituting the remaining Q*'*s with I*'*s.

The amino acid sequence set forth in SEQ ID NO: 32 is obtained by substituting all QQ*'*s with VF*'*s in a sequence in which a region of 20 domain sequences existing in the amino acid sequence set forth in SEQ ID NO: 7 (Met-PRT410) is repeated twice and substituting the remaining Q*'*s with I*'*s.

The amino acid sequence set forth in SEQ ID NO: 41 (Met-PRT917) is obtained by substituting all QQ*'*s in the amino acid sequence set forth in SEQ ID NO: 7 with LI*'*s and substituting the remaining Q*'*s with V*'*s. The amino acid sequence set forth in SEQ ID NO: 42 (Met-PRT1028) is obtained by substituting all QQ*'*s in the amino acid sequence set forth in SEQ ID NO: 7 with IF*'*s and substituting the remaining Q*'*s with T*'*s.

The content rate of the glutamine residues in each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 2).

**[Table 2]**

| Modified fibroin | Content rate of glutamine residues | Content rate of GPGXX motifs | Hydrophobic degree of REP |
|---|---|---|---|
| Met-PRT410 (SEQ ID NO: 7) | 17.7% | 27.9% | -0.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT966 (SEQ ID NO: 32) | 0.0% | 28.0% | 0.35 |
| Met-PRT917 (SEQ ID NO: 41) | 0.0% | 27.9% | 0.46 |
| Met-PRT1028 (SEQ ID NO: 42) | 0.0% | 28.1% | 0.05 |

The modified fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-ii) contains an amino acid sequence having a sequence identity of 90% or higher with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The modified fibroin of (6-ii) is also a protein having a domain sequence represented by Formula 1: [(A)ₙ motif-REP]ₘ or Formula 2: [(A)ₙ motif-REP]ₘ-(A)ₙ motif. The sequence identity is preferably 95% or higher.

The content rate of the glutamine residues in the modified fibroin of (6-ii) is preferably 9% or less. Furthermore, the content rate of the GPGXX motifs in the modified fibroin of (6-ii) is preferably 10% or more.

The sixth modified fibroin may have a tag sequence at one or both of the N-terminus and the C-terminus thereof. By having a tag sequence, isolation, immobilization, detection, visualization, and the like of the modified fibroin become possible.

More specific examples of the modified fibroin having a tag sequence include modified fibroin having (6-iii) an amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028) or modified fibroin having (6-iv) an amino acid sequence having a sequence identity of 90% or higher with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 are obtained by adding the amino acid sequence set forth in SEQ ID NO: 11 (which has a His-tag sequence and a hinge sequence) to the N-termini of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42, respectively. Since only the tag sequence is added to the N-termini, the content rates of glutamine residues do not change, and the content rate of glutamine residues in each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is 9% or less (Table 3).

**[Table 3]**

| Modified fibroin | Content rate of glutamine residues | Content rate of GPGXX motifs | Hydrophobic degree of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |
| PRT917 (SEQ ID NO: 43) | 0.0% | 27.9% | 0.46 |
| PRT1028 (SEQ ID NO: 44) | 0.0% | 28.1% | 0.05 |

The modified fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The modified fibroin of (6-iv) contains an amino acid sequence having a sequence identity of 90% or higher with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The modified fibroin of (6-iv) is also a protein having a domain sequence represented by Formula 1: [(A)ₙ motif-REP]ₘ or Formula 2: [(A)ₙ motif-REP]ₘ-(A)ₙ motif. The sequence identity is preferably 95% or higher.

The content rate of glutamine residues in the modified fibroin of (6-iv) is preferably 9% or less. The content rate of the GPGXX motifs in the modified fibroin of (6-iv) is preferably 10% or more.

The sixth modified fibroin may contain a secretory signal for releasing a protein produced in an artificial protein production system to the outside of a host. A sequence of the secretory signal is designed appropriately depending on the type of the host.

The modified fibroin may have at least two characteristics among the characteristics of the first modified fibroin, the second modified fibroin, the third modified fibroin, the fourth modified fibroin, the fifth modified fibroin, and the sixth modified fibroin.

The modified fibroin according to the present embodiment is a protein having a domain sequence represented by Formula 1: [(A)n motif-REP]m or Formula 2: [(A)n motif-REP]m-(A)n motif. Furthermore, an amino acid sequence (N-terminal sequence and C-terminal sequence) may be added to either or both of the N-terminus and the C-terminus of the domain sequence of the modified fibroin. The N-terminal sequence and the C-terminal sequence are typically, but are not limited to, regions not having repeats of fibroin-specific amino acid motifs and having about 100 amino acid residues.

The term *"*domain sequence*"* in the present specification is an amino acid sequence that produces a crystal region (typically, corresponding to the (A)ₙ motif of the amino acid sequence) and an amorphous region (typically, corresponding to REP of the amino acid sequence) specific to fibroin, and means an amino acid sequence represented by Formula 1: [(A)ₙ motif-REP]ₘ or Formula 2: [(A)ₙ motif-REP]ₘ- (A)ₙ motif. Here, the (A)ₙ motif represents an amino acid sequence consisting of 4 to 27 amino acid residues, and the number of alanine residues with respect to the total number of amino acid residues in the (A)ₙ motif is 80% or more. REP represents an amino acid sequence consisting of 10 to 200 amino acid residues. m represents an integer of 10 to 300. m is preferably an integer of 20 to 300, and more preferably an integer of 30 to 300. The plurality of (A)ₙ motifs may be the same amino acid sequence or different amino acid sequences. The plurality of REPs may be identical or different amino acid sequences.

It is preferable that at least seven of the plurality of (A)ₙ motifs in the domain sequence consist of only alanine residues. The phrase *"*consist of only alanine residues*"* means that the (A)ₙ motif has an amino acid sequence represented by (Ala)ₖ (where Ala represents an alanine residue). k preferably represents an integer of 4 to 27, more preferably an integer of 4 to 20, and still more preferably an integer of 4 to 16.

The REP consists of 10 to 200 amino acid residues. One or more of the amino acid residues constituting the REP may be an amino acid residue selected from the group consisting of a glycine residue, a serine residue, and an alanine residue. That is, the REP may have an amino acid residue selected from the group consisting of a glycine residue, a serine residue, and an alanine residue.

One or more of the amino acid residues constituting the REP may be a hydrophobic amino acid residue. That is, the REP preferably has a hydrophobic amino acid residue. The hydrophobic amino acid residue means an amino acid residue having a positive hydrophobicity index. As the hydrophobicity index (the hydropathy index, hereinafter, also referred to as *"*HI*"*) of the amino acid residue, a known index is used (Hydropathy index: Kyte J, & Doolittle R (1982)" A simple method for displaying the hydropathic character of a protein", J. Mol. Biol., 157, pp. 105-132). Examples of the hydrophobic amino acid residue include isoleucine (HI: 4.5), valine (HI: 4.2), leucine (HI: 3.8), phenylalanine (HI: 2.8), methionine (HI: 1.9), and alanine (HI: 1.8).

In the modified fibroin according to the present embodiment, it is preferable that the domain sequence has an amino acid sequence corresponding to an amino acid sequence in which a cysteine residue is inserted into an REP in comparison to naturally derived fibroin.

The domain sequence preferably has an amino acid sequence corresponding to an amino acid sequence in which a cysteine residue is inserted at a position adjacent to a glycine residue, a serine residue, or an alanine residue in an REP, and more preferably has an amino acid sequence corresponding to an amino acid sequence in which a cysteine residue is inserted at a position adjacent to a glycine residue in an REP. The cysteine residue in the REP may be located between the glycine residue, the serine residue, or the alanine residue and the glycine residue, the serine residue, or the alanine residue, or may be located between the serine residue and the glycine residue.

The domain sequence preferably has an amino acid sequence corresponding to an amino acid sequence in which a cysteine residue is inserted at a position adjacent to a hydrophobic amino acid residue in an REP. In this case, the hydrophobic amino acid residues are immobilized by a hydrophobic interaction between molecules. The cysteine residue in the REP may be located adjacent to the hydrophobic amino acid residue, may be located between the hydrophobic amino acid residue and an amino acid residue other than the hydrophobic amino acid residue, may be located between the hydrophobic amino acid residue and the glycine residue, the serine residue, or the alanine residue, or may be located between the hydrophobic amino acid residue and the glycine residue. The hydrophobic amino acid residue may be one selected from the group consisting of an isoleucine residue, a valine residue, a leucine residue, a phenylalanine residue, a methionine residue, and an alanine residue.

The domain sequence may have an amino acid sequence corresponding to an amino acid sequence in which a cysteine residue is inserted into an REP located in the vicinity of an N-terminus and/or a C-terminus of the domain sequence, in comparison to naturally derived fibroin. In this case, the molecular chain can be lengthened. In the present specification, the REP located in the vicinity of the N-terminus of the domain sequence means an REP located at positions 1 to 3 from the N-terminus of the domain sequence. For example, the cysteine residue may be located in the REP located at positions 1 and 2 from the N-terminus of the domain sequence. In the present specification, the REP located in the vicinity of the C-terminus of the domain sequence means an REP located at positions 1 to 3 from the C-terminus of the domain sequence. For example, the cysteine residue may be located in the REP located at positions 1 and 2 from the C-terminus of the domain sequence. The cysteine residue is preferably located in the REP located closest to the N-terminal side and/or closest to the C-terminal side of the domain sequence.

The domain sequence may have an amino acid sequence corresponding to an amino acid sequence in which a cysteine residue is inserted into the center or the vicinity of the center thereof in an REP, in comparison to naturally derived fibroin. In the present specification, the vicinity of the center of the amino acid sequence in the REP indicates positions 1 to 5 from the amino acid residue located in the center of the REP (the amino acid residue on the N-terminal side when two amino acid residues located in the center are present) toward the N-terminal side, or positions 1 to 5 from the amino acid residue located in the center of the REP (the amino acid residue on the C-terminal side when two amino acid residues located in the center are present). For example, the cysteine residue may be located in the middle of the REP, or may be located at positions 1 to 3 or 1 and 2 from the amino acid residue located in the middle of the REP toward the N-terminal side or the C-terminal side. Here, for example, in a case where a first segment containing a polypeptide skeleton in which cysteine residues are inserted so as to be located one by one on the N-terminal side and the C-terminal side of the domain sequence is used, a polypeptide derivative in which a first segment and a second segment are alternately linked so as to be located one by one can be obtained. The toughness of the synthetic leather obtained using this polypeptide derivative can be further improved. In a case where a first segment containing a polypeptide skeleton in which a cysteine residue is inserted so as to be located more centrally than the N-terminal side and the C-terminal side of the domain sequence is used, a polypeptide derivative in which the second segment is linked to the first segment can be expected to have improved solubility in a solvent.

The modified fibroin preferably contains a GPGXX motif (G represents a glycine residue, P represents a proline residue, and X represents an amino acid residue other than the glycine residue) in the amino acid sequence of the REP. The motif is contained in the REP, such that elongation of the modified fibroin can be improved.

In a case where the modified fibroin contains the GPGXX motif in the REP, a content rate of the GPGXX motifs is generally 1% or more. The content rate of the GPGXX motifs may be 5% or more and is preferably 10% or more. In this case, the stress of the modified fibroin fiber is further increased. An upper limit of a content rate of the GPGXX motif is not particularly limited and may be 50% or less or 30% or less.

In the present specification, the "content rate of the GPGXX motif" is a value calculated by the following method. The content rate of the GPGXX motif is calculated as c/d in a case where in all REPs included in a sequence excluding the sequence from the (A)ₙ motif located at the most C-terminal side to the C-terminal of the domain sequence in fibroin having a domain sequence represented by Formula 1: [(A)ₙ motif-REP]ₘ or Formula 2: [(A)ₙ motif-REP]ₘ-(A)ₙ motif, a number three times the total number of GPGXX motifs included in this region is defined as c (that is, a number corresponding to the total number of G and P in the GPGXX motif), and the total number of amino acid residues in all REPs excluding the sequence excluding the sequence from the (A)ₙ motif located at the most C-terminal side to the C-terminal of the domain sequence from the domain sequence and further excluding the (A)ₙ motifs is defined as d.

In the calculation of the content rate of the GPGXX motif, the *"*sequence excluding the sequence from the (A)ₙ motif located at the most C-terminal side to the C-terminus of the domain sequence from the domain sequence*"* is used to exclude the effect occurring due to the fact that the *"*sequence from the (A)ₙ motif located at the most C-terminal side to the C-terminus of the domain sequence*"* (a sequence corresponding to REP) may include a sequence having a low correlation with the sequence characteristic of fibroin, which influences the calculation result of the content rate of the GPGXX motif in a case where m is small (that is, in a case where the domain sequence is short). In a case where the *"*GPGXX motif*"* is located at the C-terminus of an REP, even when *"*XX*"* is *"*AA*"*, for example, it is regarded as the *"*GPGXX motif*"*.

Fig. 9 is a schematic view illustrating a domain sequence of fibroin. The calculation method of the content rate of the GPGXX motif will be specifically described with reference to Fig. 9. First, in the domain sequence of the fibroin illustrated in Fig. 9 (which is the *"*[(A)ₙ motif-REP]ₘ-(A)ₙ motif*"* type), all REPs are included in the *"*sequence excluding the sequence from the (A)ₙ motif located at the most C-terminal side to the C-terminus of the domain sequence from the domain sequence*"* (in Fig. 9, the sequence indicated as a *"*region A*"*), and therefore, the number of the GPGXX motifs for calculating c is 7, and c is 7 × 3 = 21. Similarly, since all REPs are included in the *"*sequence excluding the sequence from the (A)ₙ motif located at the most C-terminal side to the C-terminus of the domain sequence from the domain sequence*"* (in Fig. 9, the sequence indicated as the *"*region A*"*), the total number d of the amino acid residues in all REPs when the (A)ₙ motifs are further excluded from the sequence is 50 + 40 + 10 + 20 + 30 = 150. Next, c/d (%) can be calculated by dividing c by d, and in the case of the fibroin of Fig. 9, c/d (%) is 21/150 = 14.0%.

A hydrophobic degree (hydropathy index : hydrophobicity index) of the REP in the modified fibroin may be, for example, -0.80, -0.70, -0.06 or more, -0.50 or more, -0.40 or more, -0.30 or more, -0.20 or more, -0.10 or more, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit of the hydrophobic degree of each REP is not particularly limited, and may be 1.0 or less or 0.7 or less.

Herein, the *"*hydrophobic degree of REP*"* is a value calculated by the following method. The hydrophobic degree of REP is calculated as e/f in a case where in all REPs included in a sequence excluding the sequence from the (A)ₙ motif located at the most C-terminal side to the C-terminal of the domain sequence from the domain sequence (the sequence corresponding to the *"*region A*"* in Fig. 9) in spider fibroin including the domain sequence represented by Formula 1: [(A)ₙ motif-REP]ₘ or Formula 2: [(A)ₙ motif - REP]ₘ-(A)ₙ motif, the total sum of the hydropathy indices of each of amino acid residues in this region is defined as e, and the total number of amino acid residues in all REPs excluding the sequence from the (A)ₙ motif located at the most C-terminal side to the C-terminal of the domain sequence from the domain sequence and further excluding the (A)ₙ motifs is defined as f. In the calculation of the hydrophobic degree of REP, the reason for targeting the *"*sequence excluding the sequence from the (A)ₙ motif located at the most C-terminal side to the C-terminus of the domain sequence from the domain sequence*"* is the same as the reason described above.

The domain sequence may have an amino acid sequence corresponding to an amino acid sequence in which 1 or more and less than 16 cysteine residues are inserted into an REP, in comparison to naturally derived fibroin. That is, the total number of cysteine residues corresponding to the cysteine residues inserted into the REP may be 1 or more and less than 16. The total number of cysteine residues corresponding to the cysteine residues inserted into the REP may be 1 or more and 12 or less, 1 or more and 10 or less, 1 or more and 8 or less, 1 or more and 6 or less, or 2 or more and 4 or less. The number of cysteine residues per REP in the domain sequence may be, for example, 1 to 3, 1 or 2, or 1.

The total number of cysteine residues in the modified fibroin according to the present embodiment may be 1 or more and less than 16, 1 or more and 12 or less, 1 or more and 10 or less, 1 or more and 8 or less, 1 or more and 6 or less, or 2 or more and 4 or less.

The modified fibroin according to the present embodiment may be obtained by, for example, modification of an amino acid sequence corresponding to substitution, deletion, insertion, and/or addition of one or a plurality of amino acid residues in addition to modification related to the cysteine residue in the REP described above, in comparison to naturally derived fibroin.

A molecular weight of the modified fibroin according to the present embodiment is not particularly limited, and may be, for example, 10 kDa or more and 700 kDa or less The molecular weight of the modified fibroin according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, or may be 600 kDa or less, 500 kDa or less, 400 kDa or less, less than 360 kDa, 300 kDa or less, or 200 kDa or less.

The modified fibroin according to the present embodiment preferably has an amino acid sequence corresponding to an amino acid sequence in which a cysteine residue is inserted, and more specific examples of the modified fibroin include (i) modified fibroin having an amino acid sequence set forth in SEQ ID NO: 46, SEQ ID NO: 47, SEQ ID NO: 48, SEQ ID NO: 49, SEQ ID NO: 50, or SEQ ID NO: 51, and (ii) modified fibroin having an amino acid sequence set forth in SEQ ID NO: 52, SEQ ID NO: 53, SEQ ID NO: 54, or SEQ ID NO: 55.

The amino acid sequence (PRT1) set forth in SEQ ID NO: 46 is obtained by inserting one cysteine residue into an REP located on the most N-terminal side of a domain sequence in the amino acid sequence (PRT11) set forth in SEQ ID NO: 56. That is, the total number of cysteine residues in the amino acid sequence (PRT1) set forth in SEQ ID NO: 46 is 1.

The amino acid sequence (PRT2) set forth in SEQ ID NO: 47 is obtained by inserting one cysteine residue into an REP located on the most N-terminal side of a domain sequence in the amino acid sequence (PRT12) set forth in SEQ ID NO: 57. That is, the total number of cysteine residues in the amino acid sequence (PRT2) set forth in SEQ ID NO: 47 is 1.

The amino acid sequence (PRT3) set forth in SEQ ID NO: 48 is obtained by inserting one cysteine residue into an REP located on each of the most N-terminal side and an REP located on the most C-terminal side of a domain sequence in the amino acid sequence (PRT12) set forth in SEQ ID NO: 57. That is, the total number of cysteine residues in the amino acid sequence (PRT3) set forth in SEQ ID NO: 48 is 2.

The amino acid sequence (PRT4) set forth in SEQ ID NO: 49 is obtained by inserting one cysteine residue into an REP located at positions 1 and 2 on each of the N-terminal side and the C-terminal side of a domain sequence in the amino acid sequence (PRT12) set forth in SEQ ID NO: 57. That is, the total number of cysteine residues in the amino acid sequence (PRT4) set forth in SEQ ID NO: 49 is 4.

The amino acid sequence (PRT5) set forth in SEQ ID NO: 50 is obtained by inserting one cysteine residue into an REP located at positions 1 to 4 on each of the N-terminal side and the C-terminal side of a domain sequence in the amino acid sequence (PRT12) set forth in SEQ ID NO: 57. That is, the total number of cysteine residues in the amino acid sequence (PRT5) set forth in SEQ ID NO: 50 is 8.

The amino acid sequence (PRT6) set forth in SEQ ID NO: 51 is obtained by inserting one cysteine residue into an REP located at positions 1 to 8 on each of the N-terminal side and the C-terminal side of a domain sequence in the amino acid sequence (PRT12) set forth in SEQ ID NO: 57. That is, the total number of cysteine residues in the amino acid sequence (PRT6) set forth in SEQ ID NO: 51 is 16.

The amino acid sequence (PRT7) set forth in SEQ ID NO: 52 is obtained by inserting one cysteine residue into an REP located on each of the most N-terminal side and the most C-terminal side of a domain sequence in the amino acid sequence (PRT13) set forth in SEQ ID NO: 58. That is, the total number of cysteine residues in the amino acid sequence (PRT7) set forth in SEQ ID NO: 52 is 2.

The amino acid sequence (PRT8) set forth in SEQ ID NO: 53 is obtained by inserting one cysteine residue into an REP located on each of the most N-terminal side and the most C-terminal side of a domain sequence in the amino acid sequence (PRT14) set forth in SEQ ID NO: 59. That is, the total number of cysteine residues in the amino acid sequence (PRT8) set forth in SEQ ID NO: 53 is 2.

The amino acid sequence (PRT9) set forth in SEQ ID NO: 54 is obtained by inserting one cysteine residue into an REP located at positions 1 to 4 on each of the N-terminal side and the C-terminal side of a domain sequence in the amino acid sequence (PRT14) set forth in SEQ ID NO: 59. That is, the total number of cysteine residues in the amino acid sequence (PRT9) set forth in SEQ ID NO: 54 is 8.

The amino acid sequence (PRT10) set forth in SEQ ID NO: 55 is obtained by inserting one cysteine residue into an REP located at positions 1 to 8 on each of the N-terminal side and the C-terminal side of a domain sequence in the amino acid sequence (PRT14) set forth in SEQ ID NO: 59. That is, the total number of cysteine residues in the amino acid sequence (PRT10) set forth in SEQ ID NO: 55 is 16.

The modified fibroin of (i) may have only an amino acid sequence set forth in SEQ ID NO: 46, SEQ ID NO: 47, SEQ ID NO: 48, SEQ ID NO: 49, SEQ ID NO: 50, or SEQ ID NO: 51. The modified fibroin of (ii) may have only an amino acid sequence set forth in SEQ ID NO: 52, SEQ ID NO: 53, SEQ ID NO: 54, or SEQ ID NO: 55.

The modified fibroin described above may have a tag sequence at either or both of the N-terminal and C-terminal. By having a tag sequence, isolation, immobilization, detection, visualization, and the like of the modified fibroin become possible.

Examples of the tag sequence include an affinity tag using specific affinity (binding properties or affinity) to another molecule. A specific example of the affinity tag includes a histidine tag (His tag). The His-tag is a short peptide in which about 4 to 10 histidine residues are lined up and can be used for isolating modified fibroin by chelating metal chromatography, since it has a property of specifically binding to metal ions such as nickel. A specific example of the tag sequence may be an amino acid sequence set forth in SEQ ID NO: 70 or SEQ ID NO: 71 (amino acid sequence including a His tag).

As the tag sequence, it is possible to employ a tag sequence such as glutathione-S-transferase (GST) that specifically binds to glutathione or a maltose binding protein (MBP) that specifically binds to maltose.

As the tag sequence, an *"*epitope tag*"* utilizing an antigen-antibody reaction can be employed. Adding a peptide (epitope) exhibiting antigenicity as a tag sequence allows binding of an antibody to the epitope. Examples of the epitope tag include an HA (a peptide sequence of influenza virus hemagglutinin) tag, a myc tag, a FLAG tag, and the like. The use of the epitope tag allows purification of the modified fibroin to be easily performed with high specificity.

It is possible to use a tag sequence which is cleaved with a specific protease as the tag sequence. By treating a protein adsorbed via the tag sequence with a protease, the modified fibroin from which the tag sequence is cleaved can be recovered.

More specific examples of the modified fibroin having a tag sequence include (iii) modified fibroin having an amino acid sequence set forth in SEQ ID NO: 60 (PRT15), SEQ ID NO: 61 (PRT16), SEQ ID NO: 62 (PRT17), SEQ ID NO: 63 (PRT18), SEQ ID NO: 64 (PRT19), or SEQ ID NO: 65 (PRT20), and (iv) modified fibroin having an amino acid sequence set forth in SEQ ID NO: 66 (PRT21), SEQ ID NO: 67 (PRT22), SEQ ID NO: 68 (PRT23), or SEQ ID NO: 69 (PRT24).

The amino acid sequences set forth in SEQ ID NO: 60 (PRT15), SEQ ID NO: 61 (PRT16), SEQ ID NO: 62 (PRT17), SEQ ID NO: 63 (PRT18), SEQ ID NO: 64 (PRT19), and SEQ ID NO: 65 (PRT20) are obtained by introducing tag sequences having an amino acid sequence set forth in SEQ ID NO: 70 into N-termini of the amino acid sequences set forth in SEQ ID NO: 46 (PRT1), SEQ ID NO: 47 (PRT2), SEQ ID NO: 48 (PRT3), SEQ ID NO: 49 (PRT4), SEQ ID NO: 50 (PRT5), and SEQ ID NO: 51 (PRT6), respectively. The amino acid sequences set forth in SEQ ID NO: 66 (PRT21), SEQ ID NO: 67 (PRT22), SEQ ID NO: 68 (PRT23), and SEQ ID NO: 69 (PRT24) are obtained by introducing tag sequences having an amino acid sequence set forth in SEQ ID NO: 70 into N-termini of the amino acid sequences set forth in SEQ ID NO: 52 (PRT7), SEQ ID NO: 53 (PRT8), SEQ ID NO: 54 (PRT9), and SEQ ID NO: 55 (PRT10), respectively.

The modified fibroin of (iii) may have only an amino acid sequence set forth in SEQ ID NO: 60, SEQ ID NO: 61, SEQ ID NO: 62, SEQ ID NO: 63, SEQ ID NO: 64, or SEQ ID NO: 65.

Content rates of GPGXX motifs of the modified fibroin having the amino acid sequences set forth in SEQ ID NO: 60 (PRT15), SEQ ID NO: 61 (PRT16), SEQ ID NO: 62 (PRT17), SEQ ID NO: 63 (PRT18), SEQ ID NO: 64 (PRT19), and SEQ ID NO: 65 (PRT20) are 40.2%, 39.9%, 39.9%, 39.7%, 39.3%, and 38.6%, respectively, or are all 10% or more.

The modified fibroin of (iv) may have only an amino acid sequence set forth in SEQ ID NO: 66, SEQ ID NO: 67, SEQ ID NO: 68, or SEQ ID NO: 69.

Content rates of GPGXX motifs of the modified fibroin having the amino acid sequences set forth in SEQ ID NO: 66 (PRT21), SEQ ID NO: 67 (PRT22), SEQ ID NO: 68 (PRT23), and SEQ ID NO: 69 (PRT24) are 39.9%, 39.9%, 39.3%, and 38.6%, respectively, or are all 10% or more.

The above-mentioned modified fibroin may include a secretory signal for releasing the protein produced in the recombinant protein production system to the outside of a host. A sequence of the secretory signal is designed appropriately depending on the type of the host.

The protein can be produced by a normal method using a nucleic acid encoding the protein. The nucleic acid encoding the protein may be chemically synthesized based on base sequence information or may be synthesized using a PCR method or the like.

### (Second Segment)

The second segment contains a molecular group having a plasticizing function for the polypeptide skeleton. The molecular group having the plasticizing function for the polypeptide skeleton refers to a molecular group in which an intermolecular force between the molecular groups is smaller than an intermolecular force between the polypeptide skeletons, and in a case where the molecular groups are mixed with each other, the flexibility of the material can be improved as compared with the polypeptide skeleton alone. The molecular group having the plasticizing function for the polypeptide skeleton can also be said to be a molecular group having a melting point or a glass transition temperature lower than that of the polypeptide skeleton. The molecular group having the plasticizing function for the polypeptide skeleton may be, for example, polyether, polyester, or polycarbonate. The polyether as the molecular group may be, for example, polyethylene glycol (PEG) or polytetramethylene glycol (PTMG). The plasticizing function can also be said to be a function of improving flexibility or a function of increasing an elongation at break in bending and/or pulling. As the molecular group having the plasticizing function for the polypeptide skeleton, a molecular group having biodegradability or a molecular group derived from biomass is suitably used. Therefore, it is sufficiently expected that biodegradability and a biovalue of the synthetic leather as a whole are further improved, and furthermore, the energy required for producing the synthetic leather can be further reduced.

The second segment may contain a plurality of molecular groups. The second segment may contain a plurality of molecular groups, and the plurality of molecular groups may be linked to each other. The linking between the molecular groups may partially have a branch. Since the linking partially has a branch, the second segment can have a branched structure by the plurality of molecular groups, and a plurality of bonds with the first segment can be formed for each branch. That is, a network structure of the first segment and the second segment can also be formed by introducing a branch into a part of the linking. As described above, the second segment contains the plurality of molecular groups, and these molecular groups are linked to each other, such that the designed width of the second segment can be further expanded, and for example, the flexibility of the synthetic leather can be more easily adjusted. In a case where the second segment contains only one molecular group, a plurality of second segments may be linked to each other, and at least one of the plurality of second segments may be bonded to the first segment. Alternatively, the plurality of second segments may be bonded to one first segment. In a case where the plurality of second segments are linked to each other, a branch may be introduced into a part of the linking between the plurality of second segments to form a network of the second segments.

The second segment may contain, for example, a skeleton derived from at least one selected from the group consisting of polyether, polyester, polycarbonate, polyamide, polyol (polyvinyl alcohol or the like), polyolefin, polyacetal, polyketal, poly(meth)acrylate, silicone, polyurethane, polyalkyleneimine, a phenolic resin, a urea resin, a melamine resin, and polysaccharides, and preferably, may contain at least one functional group selected from the group consisting of a polyether group, a polyester group, a polycarbonate group, a polyamide group, a polyol group (polyvinyl alcohol group or the like), and a modified polysaccharide group, and may include at least one functional group selected from the group consisting of an ether group, an ester group, a carbonate group, an amide group, and s modified polysaccharide group. The second segment may have, for example, at least one structural unit selected from the group consisting of structural units having an ether bond, an ester bond, a carbonate ester bond (carbonate bond), an amide group, a siloxane bond, a urethane bond, a urethane bond, and a urea bond, or may have at least one structural unit selected from the group consisting of structural groups having an alkylene group, a substituted alkylene group, an oxymethylene group, an alkyleneimine group, and a modified polysaccharide group.

Examples of the polyether group include functional groups derived from polyalkylene glycols such as polyethylene glycol, polypropylene glycol, an ethylene oxide/propylene oxide copolymer, and polybutylene glycol (polytetramethylene glycol (PTMG)). When these polyether groups are contained in the second segment, the polyether group may be directly linked to a hetero element (O, N, and S) in an ester group, a thioester group, or an amide group of a linker contained in the second segment described below as necessary. Alternatively, the polyether group may be directly linked to a hetero element (O, N, and S) in an ester group, a thioester group, or an amide group of the polypeptide skeleton contained in the first segment. In this case, the entire polyether group is easily separated from the linker and/or the first segment, and as a result, the biodegradation rate of the polyether group can be increased.

Examples of the polyester group include functional groups derived from polyesters such as polylactic acid, poly(3-hydroxybutanoic acid), a polyhydroxybutanoic acid/hydroxyvaleric acid copolymer, a polyhydroxybutanoic acid/4-hydroxybutanoic acid copolymer, a polyhydroxybutanoic acid/hydroxyhexanoic acid copolymer, polytrimethylene terephthalate, a butanediol/long-chain dicarboxylic acid copolymer, polyethylene terephthalate, polybutylene succinate, a polybutylene succinate adipate copolymer, a polybutylene adipate terephthalate copolymer, polycaprolactone, and poly(trimethylene furandicarboxylate) (PTF). Among the polyesters described above, the polyester group is preferably a functional group derived from being classified into a biomass plastic such as polycaprolactone or a biodegradable plastic.

Examples of the polycarbonate group include functional groups derived from polycarbonates having an aliphatic hydrocarbon chain as a main skeleton, such as 1,6-hexanediol polycarbonate, 1,5-pentanediol polycarbonate, and 1,10 decanediol carbonate.

Examples of the polyamide group include functional groups derived from polyamides such as nylon 3, nylon 4, nylon 5, nylon 6, nylon 11, and nylon 610 Among the polyamides described above, the polyamide group is preferably a functional group derived from being classified into a biomass plastic or a biodegradable plastic.

Examples of the polyol group (polyvinyl alcohol group or the like) include functional groups derived from polyols (polyvinyl alcohols and the like) such as polyvinyl alcohol and an ethylene-vinyl alcohol copolymer. Among the polyols described above, the polyol group is preferably a functional group derived from being classified into a biomass plastic or a biodegradable plastic.

Examples of the modified polysaccharide group include functional groups derived from compounds obtained by chemically modifying cellulose, starch, chitin, chitosan, and the like. Examples of the chemically modified compound include cellulose acetate, ethyl cellulose, starch acetate, hydroxypropylated starch, carboxymethyl chitin, and carboxymethyl chitosan.

The second segment may further contain a linker in addition to the molecular group having the plasticizing function for the polypeptide skeleton. In this case, the molecular group and the polypeptide skeleton may be bonded via the linker. The linker may include, for example, at least one selected from the group consisting of a structural unit represented by the following Formula (1), structural units represented by the following General Formulas (2a) to (6), a structural unit represented by the following Formula (7), structural units represented by the following General Formulas (8a) to (9), structural units represented by the following General Formulas (10) to (11b), and structural units represented by the following General Formulas (13) to (16), may include at least one selected from the group consisting of a structural unit represented by the following Formula (1), structural units represented by the following General Formulas (2a) to (6), a structural unit represented by the following Formula (7), and structural units represented by the following General Formulas (8a) to (9), and preferably, from the viewpoint of improving biodegradability, may include at least one selected from the group consisting of a structural unit represented by the following Formula (1) and structural units represented by the following General Formulas (2a), (3a), (4a), (4b), and (10). The linker may have a plurality of structural units described above. That is, the linker may have only the structural unit described above, or may have a plurality of structural units described above (a plurality of structural units described above may be covalently bonded to the main body portion of the linker). The main body portion of the linker preferably has a relatively small molecular weight. In a case where the linker has three or more structural units described above, for example, three first segments can be bonded to one second segment, and a network structure can also be introduced into the block copolymer contained in the polypeptide derivative by such selection. A polypeptide derivative containing a block copolymer in which some of the second segments that are plural are bonded to each other can be obtained by selecting a raw material used for producing the polypeptide derivative (adjusting a functional group of a compound containing a polypeptide skeleton and a compound containing a molecular group having a plasticizing function for a polypeptide skeleton). In a case where the linker has a plurality of structural units described above, at least one of the plurality of structural units may be bonded to the polypeptide skeleton, and at least one of the plurality of structural units may be bonded to the molecular group. In a case where the linker has only the structural unit described above, the peptide skeleton is bonded to one of the two bonds described in the formula representing the structural unit representing the linker, and the molecular group is bonded to the other. For example, in a case where the linker is represented by the following Formula (1), preferably, the molecular group is bonded to nitrogen (N), and the other is bonded to a polypeptide skeleton. In a case where the linker has a plurality of structural units described above, for example, in a case of a structural unit represented by the following Formula (1), nitrogen (N) is preferably bonded to the main body of the linker, and the other is bonded to the polypeptide skeleton and the molecular group.

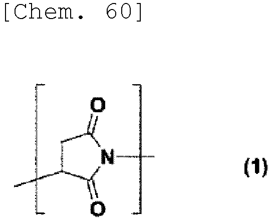

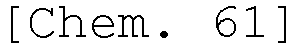

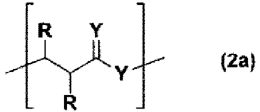

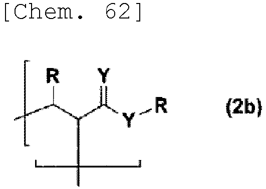

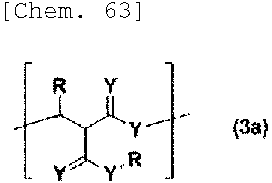

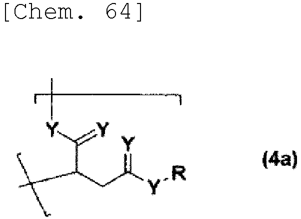

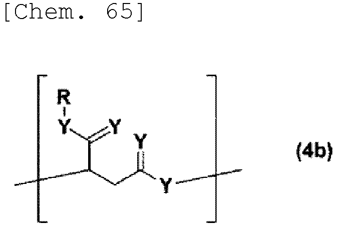

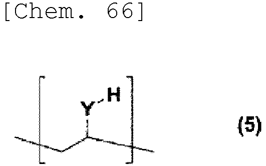

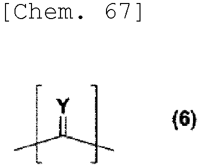

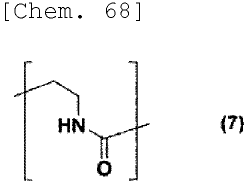

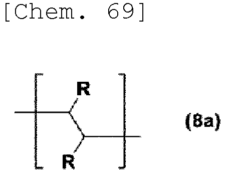

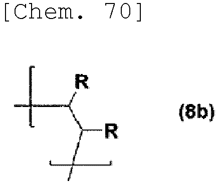

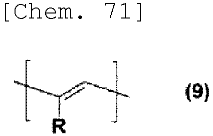

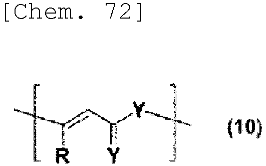

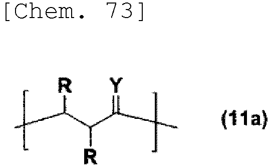

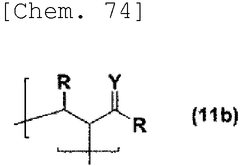

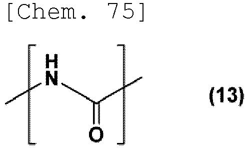

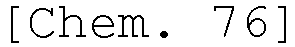

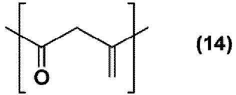

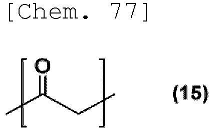

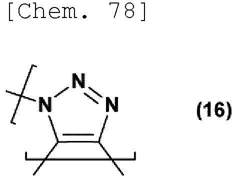

In General Formulas (2a), (2b), (3a), (4a), (4b), (5), (6), (10), (11a), and (11b), Y's each independently represent an oxygen atom, a sulfur atom, or NR¹. In NR¹, R¹ represents hydrogen, a hydrocarbon group, an aromatic group, a carbonyl group, or a sulfonyl group. In General Formulas (2a), (2b), (3a), (4a), (4b), (8a), (8b), (9), (10), (11a), and (11b), R's each independently represent hydrogen, a hydrocarbon group, or an aromatic group.

A molecular weight of the second segment (the molecular group having the plasticizing function for the polypeptide skeleton) may be, for example, 200 to 500,000, 300 to 400,000, 350 to 350,000, 400 to 300,000, 500 to 200,000, 600 to 1,000,000, 700 to 50,000, 800 to 10,000, 900 to 7,500, or 1,000 to 5,000.

When the molecular weight of the second segment is 200 or more, localization of the molecular group having the plasticizing function in the three-dimensional structure of the molecule can be easily suppressed, and a weight ratio of the molecular group having the plasticizing function that is required to be introduced to exhibit a certain level of function can be sufficiently reduced. As a result, there is a possibility that it is required to shorten a reaction time and lower a reaction temperature in the production of the polypeptide derivative.

When the molecular weight of the second segment is 500,000 or less, the molecular weight is within an appropriate range, and a decrease in binding reactivity of the second segment to the first segment is more easily suppressed.

The molecular weight of the second segment is a weight average molecular weight, and is generally determined by a known method using GPC.

The molecular weight of the second segment (the molecular group having the plasticizing function for the polypeptide skeleton) with respect to the molecular weight of the first segment (the polypeptide skeleton) can be appropriately adjusted according to the application of the polypeptide derivative and the like. The molecular weight of the second segment (when two or more second segments are bonded to one first segment, the total molecular weight) is, for example, preferably 1 to 10,000, more preferably 1.5 to 9,000, still more preferably 2 to 8,000, still more preferably 3 to 7,000, still more preferably 5 to 5,000, still more preferably 7 to 3,000, and further still more preferably 10 to 2,000, based on the molecular weight of the first segment of 100.

When the molecular weight of the second segment is 1 or more with respect to the molecular weight of the first segment, the flexibility of the synthetic leather obtained using the polypeptide derivative can be further enhanced. When the molecular weight of the second segment is 10,000 or less with respect to the molecular weight of the first segment, the synthetic leather can have sufficient plasticity (flexibility) and the rigidity of the material of the synthetic leather can be further improved. When the molecular weight of the second segment with respect to the molecular weight of the first segment is within the above range, it is possible to produce a synthetic leather having more excellent flexibility.

The molecular weight of the second segment and the molecular weight of the molecular group having the plasticizing function for the polypeptide skeleton may be, for example, 1.4 or more, 1.5 or more, 1.6 or more, 1.7 or more, 1.8 or more, 1.9 or more, 2.0 or more, 5.0 or more, 10 or more, 20 or more, 30 or more, or 40 or more, when the molecular weight of the first segment or the molecular weight of the polypeptide skeleton contained in the first segment is 100 (preferably, when the molecular weight of the polypeptide skeleton contained in the first segment is 100). An upper limit value thereof is not particularly limited, and may be, for example, 1,000 or less, 800 or less, 600 or less, 400 or less, 200 or less, 100 or less, 80 or less, 70 or less, 60 or less, or 50 or less. When the molecular weight of the first segment is 100, the molecular weight of the second segment is desirably within a range of 1 to 1,000, more desirably within a range of 1 to 800, still more desirably within a range of 1 to 600, still more desirably within a range of 1 to 400, still more desirably within a range of 1 to 200, further still more desirably within a range of 1 to 100, preferably within a range of 1 to 70, more preferably within a range of 1.5 to 60, still more preferably within a range of 1.5 to 50, and particularly preferably within a range of 2.0 to 50. In a case where a ratio of the molecular weight of the first segment (the polypeptide skeleton) to the molecular weight of the second segment (the molecular group having the plasticizing function for the polypeptide skeleton) is a value within the above range, for example, in a synthetic leather obtained using a polypeptide derivative, it can be expected to enhance the flexibility, stretchability, or the like while sufficiently maintaining the properties (for example, high mechanical strength) of the first segment due to containing of the polypeptide skeleton. A ratio of the molecular weight of the second segment when the molecular weight of the first segment is 100 is determined as a weight average molecular weight.

A content ratio of the first segment (the polypeptide skeleton) and the second segment (the molecular group having the plasticizing function) in the polypeptide derivative can be appropriately adjusted depending on the application of the synthetic leather and the like. Such a content ratio of the first segment when the second segment is 100 in terms of mass ratio may be, for example, 50 or more, 60 or more, 70 or more, 80 or more, 90 or more, 100 or more, 110 or more, 150 or more, 200 or more, 250 or more, 300 or more, 300 or more, 400 or more, 450 or more, 500 or more, 550 or more, or 600 or more. An upper limit value of such a value is not particularly limited, and may be 1,000 or less, 900 or less, 800 or less, or 700 or less. Since the content ratio of the first segment and the second segment in the polypeptide derivative is set to a value within the above range, wasteful use of the second segment is suppressed, and the production cost of the polypeptide derivative can be suppressed. A reduction of the content ratio of the second segment to the first segment in the polypeptide derivative can be advantageously realized by, for example, reducing the molecular weight of the first segment.

In the description of the above embodiment, the linker has been described as a constituent element of the second segment, and may be treated separately from the second segment. The linker can be treated as a constituent element of the first segment. A molecular weight of the linker moiety is smaller than those of the polypeptide skeleton and the molecular group, but in a case where the linker is treated separately from the second segment, as for the molecular weight of the second segment, a range obtained by subtracting the molecular weight of the linker moiety is treated as a preferred range of the molecular weight of the second segment. Similarly, in a case where the linker moiety is a constituent element of the first segment, as for the molecular weight of the first segment, a range obtained by adding the molecular weight of the linker moiety is treated as a preferred range of the molecular weight of the first segment.

### <Method for Producing Polypeptide Derivative>

The polypeptide derivative described above can be produced, for example, by introducing a functional group (for example, at least one functional group selected from the group consisting of a polyether group, a polyester group, a polycarbonate group, a polyamide group, a polyol group, and a modified polysaccharide group) including the molecular group into the first segment by generating a thioether group using a 1,4-addition reaction between a thiol group (also referred to as a mercapto group or a sulfhydryl group) of cysteine constituting a polypeptide skeleton and a carbon-carbon double bond of maleimide or a maleic acid derivative. The polypeptide derivative described above can also be produced by applying an example of the production method described above, for example, by forming a thioether bond using the 1,4-addition reaction of a maleimide group described above, with a compound containing a polypeptide skeleton having cysteine, a compound containing a molecular group having a plasticizing function for the polypeptide skeleton and having a thiol group, and a compound having two or more maleimide groups. The polypeptide derivative described above can also be produced, for example, by utilizing an addition reaction to an epoxy group or an isocyanate group, a substitution reaction to an α-halocarbonyl group, or a Huisgen cyclization reaction between an alkynyl group and an azide group.

More generally, an embodiment of the method for producing a polypeptide derivative includes a step of obtaining a polypeptide derivative by reacting a compound containing a polypeptide skeleton with at least one of compounds represented by the following General Formulas (1A) to (16A). The step may be a step of obtaining a polypeptide derivative by reacting a compound containing a polypeptide skeleton with a compound represented by any one of the following General Formulas (1A) to (12A). The polypeptide skeleton includes at least one selected from the group consisting of a thiol group, an amino group, a hydroxy group, a guanidino group, a carboxy group, a phenoxy group, an indole group, an amide group, an azide group, and an alkynyl group, and preferably includes at least one selected from the group consisting of a thiol group, an amino group, a hydroxy group, a guanidino group, a carboxy group, a phenoxy group, an indole group, and an amide group. The reaction between the compound containing the polypeptide skeleton and the compound represented by any one of the following General Formulas (1A) to (12A) and (14A) may be a Michael addition reaction. 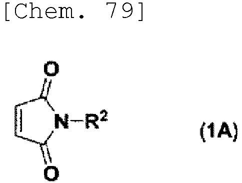 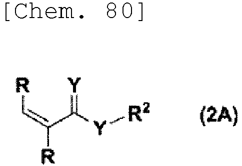 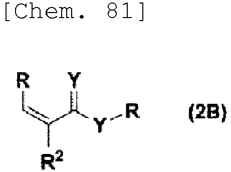 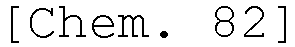 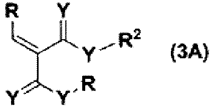 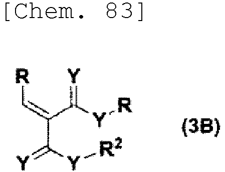 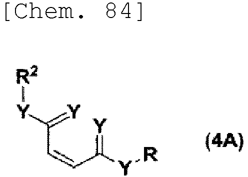 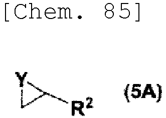 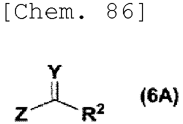 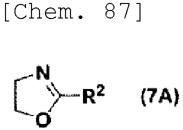 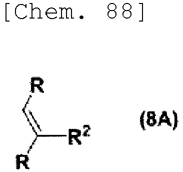 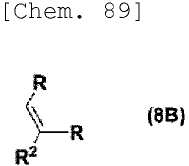 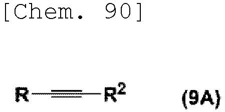 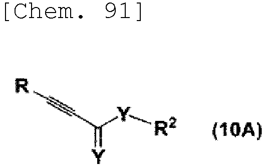 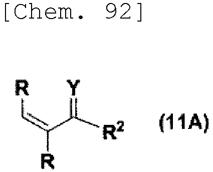 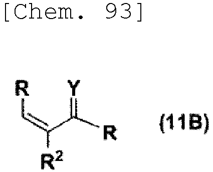 [Chem. 94]

**Z-R²** **(12A)**

[Chem. 95]

**OCN-R²** **(13A)**

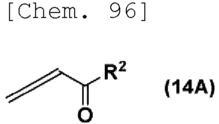

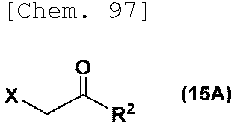

[Chem. 98]

**N₃-R²** **(16A)**

In General Formulas (1A), (2A), (2B), (3A), (3B), (4A), (5A), (6A), (7A), (8A), (8B), (9A), (10A), (11A), (11B), and (12A), R² represents a molecular group having a plasticizing function for the polypeptide skeleton. As the molecular group having the plasticizing function for the polypeptide skeleton, the examples described in the above description of the polypeptide derivative can be applied.

In General Formulas (2A), (2B), (3A), (3B), (4A), (5A), (6A), (8B), (9A), (10A), (11A), and (11B), Y's each independently represent an oxygen atom, a sulfur atom, or NR¹. In NR¹, R¹ represents hydrogen, a hydrocarbon group, an aromatic group, a carbonyl group, or a sulfonyl group. In General Formulas (2A), (2B), (3A), (3B), (4A), (5A), (8A), (8B), (9A), (10A), (11A), and (11B), R's each independently represent hydrogen, a hydrocarbon group, or an aromatic group. In General Formulas (6A) and (12A), Z represents a halogen atom, a sulfonic acid ester group, or a fluorine-containing carboxylic acid ester group. In General Formula (15A), X represents a halogen atom.

The number of molecular groups (second segments) having a plasticizing function for a polypeptide skeleton to be introduced into one compound containing a polypeptide skeleton (one first segment) is equal to or less than the total number of functional groups of a thiol group, an amino group, a hydroxy group, a guanidino group, a carboxy group, a phenoxy group, an indole group, an amide group, an azide group, and an alkynyl group (preferably, a thiol group, an amino group, a hydroxy group, a guanidino group, a carboxy group, a phenoxy group, an indole group, and an amide group) derived from an amino acid sequence constituting a polypeptide skeleton, and may be, for example, 1 or more, 2 or more, or 4 or more, and may be 30 or less, 25 or less, 20 or less, 15 or less, 10 or less, or 8 or less. The number of molecular groups (second segments) having a plasticizing function for a polypeptide skeleton to be introduced into one compound containing a polypeptide skeleton (one first segment) can be adjusted within the above range, and may be, for example, 1 to 30, 1 to 25, 1 to 20, 1 to 15, 1 to 10, 1 to 8, or 2 to 8.

In the method for producing a polypeptide derivative, for example, a compound containing a polypeptide skeleton and at least one of compounds represented by General Formulas (1A) to (16A) (preferably, compounds represented by General Formulas (1A) to (12A)) may be mixed in a solvent. In a case where a reaction is difficult to proceed, addition of an acid and a base, heating, and the like may be performed. The reaction temperature is usually 0 to 150°C, and may be, for example, 50 to 150°C, 70 to 120°C, 90 to 100°C, or may be 10 to 110°C, 20 to 100°C, or 25 to 90°C. The solvent to be used may be any solvent that can dissolve the compound containing the polypeptide skeleton and the compounds represented by General Formulas (1A) to (16A), and does not inhibit the progress of a desired reaction. Examples of the solvent include hexafluoroisopropyl alcohol (HFIP), dimethyl sulfoxide (DMSO), dimethylformamide (DMF), dimethylacetamide (DMA), acetic acid, and formic acid.

The product (crude product containing a polypeptide derivative) obtained by the above reaction may be purified by, for example, reprecipitation, normal phase column chromatography, reverse phase column chromatography, size exclusion chromatography, washing with a solvent, or the like.

An embodiment of the method for producing a polypeptide derivative described above includes a step of obtaining a polypeptide derivative obtained by reacting a compound containing a polypeptide skeleton with a compound containing a molecular group having a plasticizing function for the polypeptide skeleton, and a compound having at least two or more of at least one structural units selected from the group consisting of structural units represented by General Formulas (2-1A) to (2-16A). The step may be a step of obtaining a polypeptide derivative obtained by reacting a compound containing a polypeptide skeleton with a compound containing a molecular group having a plasticizing function for the polypeptide skeleton, and a compound having at least two or more of at least one functional groups selected from the group consisting of structural units represented by General Formulas (2-1A) to (2-12A).

The polypeptide skeleton includes at least one selected from the group consisting of a thiol group, an amino group, a hydroxy group, a guanidino group, a carboxy group, a phenoxy group, an indole group, an amide group, an azide group, and an alkynyl group, and preferably includes at least one selected from the group consisting of a thiol group, an amino group, a hydroxy group, a guanidino group, a carboxy group, a phenoxy group, an indole group, and an amide group.

The compound containing a molecular group may be a compound containing a reactive functional group capable of reacting with any one of the structural units represented by the following General Formulas (2-1A) to (2-12A) in addition to the molecular group. The reactive functional group may be at least one selected from the group consisting of a thiol group, an amino group, a hydroxy group, a guanidino group, a carboxy group, a phenoxy group, an indole group, an amide group, an azide group, and an alkynyl group, and is preferably at least one selected from the group consisting of a thiol group, an amino group, a hydroxy group, a guanidino group, a carboxy group, a phenoxy group, an indole group, and an amide group. The compound containing a molecular group may be a compound containing two or more reactive functional groups, or may be a compound containing two reactive functional groups.

The functional group of the polypeptide skeleton and the functional group of the compound containing the molecular group can be selected according to the type of the functional group of the compound having at least two or more of at least one functional groups selected from the group consisting of structural units represented by General Formulas (2-1A) to (2-16A), and may be the same as or different from each other. In a case where the functional group of the polypeptide skeleton is different from the functional group of the compound containing a molecular group, the molecular structure of the polypeptide derivative can be more easily controlled.

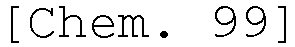

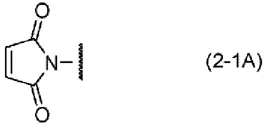

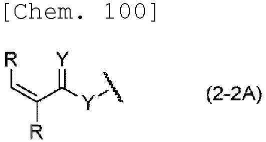

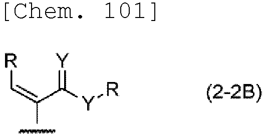

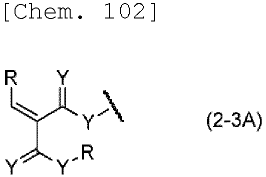

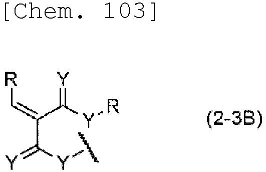

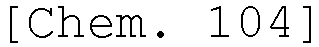

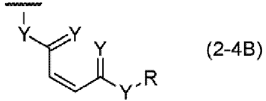

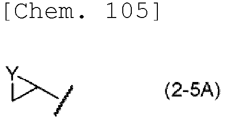

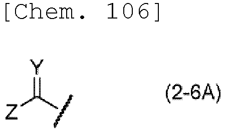

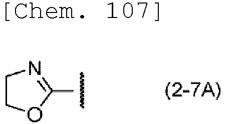

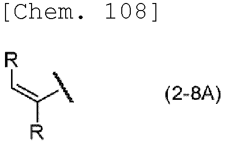

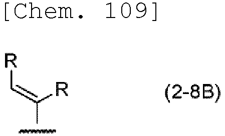

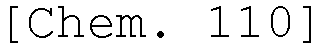

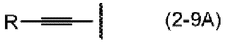

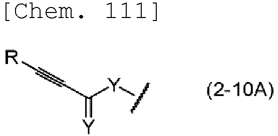

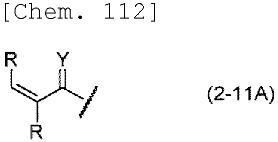

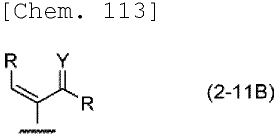

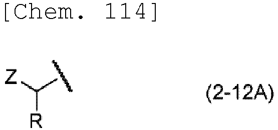

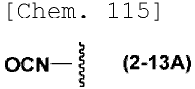

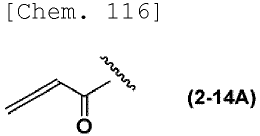

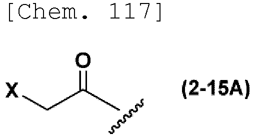

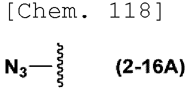

In General Formulas (2-2A), (2-2B), (2-3A), (2-3B), (2-4A), (2-5A), (2-6A), (2-10A), (2-11A), and (2-11B), Y's each independently represent an oxygen atom, a sulfur atom, or NR¹. R¹ represents hydrogen, a hydrocarbon group, an aromatic group, a carbonyl group, or a sulfonyl group. In General Formulas (2-2A), (2-2B), (2-3A), (2-3B), (2-4A), (2-8A), (2-8B), (2-9A), (2-10A), (2-11A), (2-11B), and (2-12A), R's each independently represent hydrogen, a hydrocarbon group, or an aromatic group. In General Formulas (2-6A) and (2-12A), Z represents a halogen atom, a sulfonic acid ester group, or a fluorine-containing carboxylic acid ester group. In General Formula (2-15A), X represents a halogen atom.

The reaction between the compound containing the polypeptide skeleton and the compound containing a molecular group having a plasticizing function for the polypeptide skeleton and the compound containing two or more specific functional groups described above may be, for example, a Michael addition reaction or the like.

The second segment may preferably contain a skeleton derived from an addition reaction (or addition polymerization product) of the compound containing a molecular group and the compound containing at least two or more of at least one functional groups selected from the group consisting of structural units represented by General Formulas (2-1A) to (2-12A), or may contain a skeleton derived from an addition reaction (or addition polymerization product) of the compound containing a reactive functional group and a polyether group and the compound containing at least two or more functional groups selected from the group consisting of structural units represented by General Formulas (2-1A) to (2-12A) and a polyether group.

In the step of obtaining a polypeptide derivative, a compound containing a polypeptide skeleton containing a specific functional group, and a functional group that has a plasticizing function for a polypeptide skeleton and reacts with a compound containing a molecular group having a specific functional group are separated as compounds having two or more specific structural units. By adopting such a method, a reaction solution can be prepared without proceeding a reaction between a compound containing a polypeptide skeleton containing a specific functional group and a compound having a plasticizing function for a polypeptide skeleton and containing a molecular group containing a specific functional group. Since the reaction does not proceed even when mixing is performed, it is possible to take time, for example, until a sufficiently uniform state is obtained. By blending a compound having two or more specific structural units after preparing a uniform reaction solution, the reaction system can be more easily controlled by starting the reaction, and a more uniform polypeptide derivative can be prepared. Such a method is particularly useful when solubility in a solvent and the like are low depending on the type of polypeptide skeleton or molecular group.

### Examples

Hereinafter, the present disclosure will be described more specifically based on Examples and the like. However, the present disclosure is not limited to the following Examples.

### <Production of Modified Fibroin>

### (1) Preparation of Expression Vector

Modified fibroin having an amino acid sequence (PRT27) set forth in SEQ ID NO: 72, modified fibroin having an amino acid sequence (PRT17) set forth in SEQ ID NO: 62, and modified fibroin having an amino acid sequence (PRT18) set forth in SEQ ID NO: 63 were designed. A value of an average hydropathy index of the modified fibroin having the amino acid sequence (PRT27) set forth in SEQ ID NO: 72 is 0.44, a value of an average hydropathy index of the modified fibroin having the amino acid sequence (PRT17) set forth in SEQ ID NO: 62 is 0.45, and a value of an average hydropathy index of the modified fibroin having the amino acid sequence (PRT18) set forth in SEQ ID NO: 63 is 0.46.

The amino acid sequence (PRT17) set forth in SEQ ID NO: 62 is obtained by inserting one cysteine residue into an REP located at position 1 on each of the N-terminal side and the C-terminal side of a domain sequence in the amino acid sequence (PRT27) set forth in SEQ ID NO: 72. The total number of cysteine residues in the modified fibroin having the amino acid sequence set forth in SEQ ID NO: 62 is 2. The amino acid sequence (PRT18) set forth in SEQ ID NO: 63 is obtained by inserting one cysteine residue into an REP located at positions 1 and 2 on each of the N-terminal side and the C-terminal side of a domain sequence in the amino acid sequence (PRT27) set forth in SEQ ID NO: 72. The total number of cysteine residues in the modified fibroin having the amino acid sequence set forth in SEQ ID NO: 63 is 4.

Each of nucleic acids encoding the modified fibroins having the amino acid sequences set forth in SEQ ID NO: 62, SEQ ID NO: 63, and SEQ ID NO: 72 was synthesized. In the nucleic acid, an NdeI site was added to the 5' end and an EcoRI site was added downstream of the stop codon. The nucleic acid was cloned into a cloning vector (pUC118). Thereafter, the nucleic acid was enzymatically cleaved by treatment with NdeI and EcoRI, and then, recombined into the protein expression vector pET-22b(+), thereby obtaining an expression vector.

### (2) Production of Protein

*Escherichia coli* BLR (DE3) was transformed with the obtained expression vector. The transformed *Escherichia coli* was cultured in 2 mL of an LB culture medium containing ampicillin for 15 hours. The culture solution was added to a 100 mL seed culture medium containing ampicillin (Table 4) so that OD₆₀₀ was 0.005. The culture solution was maintained at a temperature of 30°C, and put in a culture flask (for about 15 hours) until OD₆₀₀ reached 5, thereby obtaining a seed culture solution.

**[Table 4]**

| Seed culture medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

The seed culture solution was added to a jar fermenter to which a 500 mL production medium (Table 5) was added so that OD₆₀₀ was 0.05. Culture was performed while maintaining the culture solution temperature at 37°C and controlling the pH to be constant at 6.9. The culture solution was maintained to have a 20% dissolved oxygen concentration of the saturated dissolved oxygen concentration.

**[Table 5]**

| Production medium | |
|---|---|
| Reagent | Concentration (g/L) |
| Glucose | 12.0 |
| KH₂PO₄ | 9.0 |
| MgSO₄·7H₂O | 2.4 |
| Yeast Extract | 15 |
| FeSO₄·7H₂O | 0.04 |
| MnSO₄·5H₂O | 0.04 |
| CaCl₂·2H₂O | 0.04 |
| ADECANOL (ADEKA Corporation, LG-295S) | 0.1 (mL/L) |

Immediately after glucose in the production medium was completely consumed, a feed solution (455 g/1 L of glucose, 120 g/1 L of Yeast Extract) was added at a speed of 1 mL/min. Culture was performed while maintaining the culture solution temperature at 37°C and controlling the pH to be constant at 6.9. The culturing was performed for 20 hours while a dissolved oxygen concentration in the culture solution was maintained at 20% of the saturated dissolved oxygen concentration. The expression of the modified fibroin was then induced by adding 1 M isopropyl-β-thiogalactopyranoside (IPTG) to the culture solution so that the final concentration was 1 mM. When 20 hours had elapsed after the addition of IPTG, the culture solution was centrifuged, and bacterial cells were collected. SDS-PAGE was conducted using the bacterial cells prepared from the culture solutions obtained before the addition of IPTG and after the addition of IPTG. The expression of the target modified fibroin which depended on the addition of IPTG was confirmed by the appearance of a band of the size of the target modified fibroin.

### (3) Purification of Protein

Bacterial cells that were collected 2 hours after the addition of IPTG were washed with 20 mM Tris-HCl buffer (pH 7.4). The washed bacterial cells were suspended in 20 mM Tris-HCl buffer (pH 7.4) containing about 1 mM PMSF, and the cells were disrupted with a high-pressure homogenizer (manufactured by GEA Niro Soavi). The disrupted cells were centrifuged to obtain a precipitate. The obtained precipitate was washed with a 20 mM Tris-HCl buffer (pH 7.4) until the precipitate reached a high level of purity. The washed precipitate was suspended in a 8 M guanidine buffer (8 M guanidine hydrochloride, 10 mM sodium dihydrogen phosphate, 20 mM NaCl, 1 mM Tris-HCl, pH 7.0) so that the precipitate reached a concentration of 100 mg/mL, and then, the precipitate was dissolved by being stirred with a stirrer at 60°C for 30 minutes. After the precipitate was dissolved, the resulting solution was dialyzed with water using a dialysis tube (cellulose tube 36/32 manufactured by Sanko Junyaku Co., Ltd.). A white coagulation protein obtained after dialysis was collected by centrifugation, and water was removed in a freeze dryer to recover freeze-dried powder, thereby obtaining powdered modified fibroins (PRT17, PRT18, and PRT27).

### <Production of Synthetic Leather of Example A>

Configuration of Synthetic Leather of Example A
- Base fabric (fiber base material): cotton woven fabric
- Skin layer: polypeptide derivative film
- Adhesive layer: polypeptide derivative DMSO solution

### (Preparation of Base Fabric)

A cotton woven fabric was cut to a size of 105 mm × 70 mm to produce a base fabric.

### (Preparation of Skin Layer (Polypeptide Derivative Film))

After the following a), b), and c) were put into 4.65 g (4.23 mL) of dimethyl sulfoxide (DMSO), replacement with nitrogen was performed, stirring (15 min) was performed at 100°C and 300 rpm, and then the solution was allowed to stand at room temperature for 10 min. Thus, a polypeptide derivative was synthesized in DMSO to prepare a polypeptide derivative solution (polypeptide derivative concentration: 7 wt%) for forming a skin layer.
a) Modified fibroin (PRT17, number average molecular weight of 50,000): 213 mg
b) mal-PEG-mal (number average molecular weight of 20,000): 129 mg (BioChempeg Scientific Inc. HO022022-20K)
c) HS-PEG-SH (number average molecular weight of 2,000): 8.1 mg (BioChempeg Scientific Inc. HO003003-2K)

The polypeptide derivative solution was dropped on release paper attached to a stainless steel plate, smoothed with a doctor blade, and then cast with a width of 80 mm and a thickness of 400 um.

Thereafter, the cast polypeptide derivative solution was dried in an air blowing oven at 60°C for 30 min. Thus, a polypeptide derivative film for a skin layer was prepared.

### (Preparation of Adhesive (Polypeptide Derivative Solution))

After the following a), b), and c) were put into 5.58 g (5.07 mL) of DMSO, replacement with nitrogen was performed, stirring (15 min) was performed at 100°C and 300 rpm, and then the solution was allowed to stand at room temperature for 10 min. Thus, a polypeptide derivative was synthesized in DMSO to prepare a polypeptide derivative solution (polypeptide derivative concentration: 7 wt%) for an adhesive.
a) Modified fibroin (PRT17, number average molecular weight of 50,000): 256 mg
b) mal-PEG-mal (number average molecular weight of 20,000): 155 mg (BioChempeg Scientific Inc. HO022022-20K)
c) HS-PEG-SH (number average molecular weight of 2,000): 9.7 mg (BioChempeg Scientific Inc. HO003003-2K)

### (Preparation of Synthetic Leather)

A frame-shaped silicone rubber sheet having a thickness of 500 um was attached onto an outer peripheral portion of one surface of a polypeptide derivative film for a skin layer, a polypeptide derivative solution for an adhesive was applied to the inside of the frame-shaped sheet, and then a base fabric was placed thereon. Next, the base fabric was pre-dried in an oven at 60°C for 60 min, and then dried in a vacuum oven at 80°C for 12 hours. Thus, a target synthetic leather (Example A) in which a skin layer was formed on a base fabric with an adhesive layer interposed therebetween was obtained.

### <Production of Synthetic Leather of Example B>

Configuration of Synthetic Leather of Example B
- Base fabric (fiber base material): rayon knitted fabric
- Impregnation solution: polypeptide derivative DMSO solution
- Skin layer: polypeptide derivative film
- Adhesive layer: polypeptide derivative DMSO solution

### (Preparation of Impregnated Base Fabric (Porous Base Material Layer))

A rayon knitted fabric was cut to a size of 105 mm × 70 mm to produce a base fabric.

After the following a), b), and c) were put into 10.45 g (9.5 mL) of DMSO, replacement with nitrogen was performed, and stirring (15 min) was performed at 100°C and 300 rpm, thereby synthesizing the polypeptide derivative in DMSO, and at the same time, preparing a polypeptide derivative solution (polypeptide derivative concentration: 5 wt%) as an impregnation solution.
a) Modified fibroin (PRT17, number average molecular weight of 50,000): 335 mg
b) mal-PEG-mal (number average molecular weight of 20,000): 203 mg (BioChempeg Scientific Inc. HO022022-20K)
c) HS-PEG-SH (number average molecular weight of 2,000): 12.7 mg (BioChempeg Scientific Inc. HO003003-2K)

A rectangular frame-shaped silicone rubber sheet (thickness: 2 mm) slightly larger than the base fabric was bonded onto a stainless steel plate on which a Teflon sheet was laid, and then the base fabric (rayon) was placed inside the frame-shaped rubber sheet. After the polypeptide derivative solution was dropped on the base fabric, the base fabric was covered with a Teflon sheet, and then leveled to spread the polypeptide derivative solution throughout (air was removed). Thus, an impregnated base fabric was obtained. Thereafter, the impregnated base fabric was placed in a chamber at 4°C and gelled (overnight). Ethanol was placed in a container, the gel was put into the container together with the sheet (ethanol replacement), the container was allowed to stand for 3 hours to peel off the Teflon sheet, and DMSO was replaced with ethanol. Thereafter, the ethanol in the container was discarded, RO water was added, and the container was allowed to stand for 2 hours to replace the ethanol with water. Next, the gel that had been completely replaced with RO water was frozen in a freezer at -20°C and then dried using a freeze dryer overnight, thereby making the impregnated body of the impregnated base fabric porous (sponged). Thus, a base fabric (impregnated base fabric) including a sponge layer was prepared.

### (Preparation of Skin Layer (Polypeptide Derivative Film))

A skin layer was prepared in the same manner as in Example A.

### (Preparation of Adhesive (Polypeptide Derivative Solution))

A skin layer was prepared in the same manner as in Example A.

### (Preparation of Synthetic Leather)

A frame-shaped sheet having a thickness of 500 um was attached onto an outer peripheral portion of one surface of the prepared film for a skin layer, a polypeptide derivative solution for an adhesive was applied to the inside of the frame-shaped sheet, and then an impregnated base fabric was placed thereon. Next, the impregnated base fabric was pre-dried in an oven at 60°C for 60 min, and then dried in a vacuum oven at 80°C for 12 hours. Thus, a target synthetic leather (Example B) in which a skin layer was formed on a base fabric including a sponge layer (impregnated body) with an adhesive layer interposed therebetween was obtained.

### <Production of Synthetic Leather of Example C>

Configuration of Synthetic Leather of Example C
· Base fabric: rayon knitted fabric
· Impregnation solution: polypeptide derivative DMSO solution
· Skin layer: polypeptide derivative film

### (Preparation of Skin Layer (Polypeptide Derivative Film))

A skin layer was prepared in the same manner as in Example A.

### (Preparation of Synthetic Leather)

A base fabric was prepared in the same manner as in Example B. A polypeptide derivative solution as an impregnation solution was prepared in the same manner as in Example B. A frame-shaped silicone rubber sheet having a thickness of 2 mm was attached onto an outer peripheral portion of one surface of a polypeptide derivative film for a skin layer, a polypeptide derivative solution was applied to the inside of the frame-shaped sheet, a base fabric was placed thereon, and then the entire base fabric was covered with a Teflon sheet. Thereafter, the base fabric was placed in a refrigerator at 4°C and gelled (overnight). Ethanol was placed in a container, the gel was put into the container together with the sheet (ethanol replacement), the container was allowed to stand for 3 hours to peel off the Teflon sheet, and DMSO was replaced with ethanol.
Next, the ethanol in the container was discarded, RO water was added, and the container was allowed to stand for 2 hours to replace the ethanol with water. Thereafter, the gel that had been completely replaced with RO water was frozen in a freezer at -20°C and then dried using a freeze dryer overnight, thereby making the impregnated body of the impregnated base fabric porous (sponged). Thus, a base fabric including a sponge layer was prepared, and the base fabric was bonded to a skin layer, thereby obtaining a target synthetic leather (Example C).

### <Production of Synthetic Leather of Example D>

Configuration of Synthetic Leather of Example D
· Base fabric (fiber base material): rayon knitted fabric
· Impregnation solution: polypeptide derivative DMSO solution
· Skin layer: polypeptide derivative film
· Adhesive layer: polypeptide derivative DMSO solution

### (Preparation of Impregnated Base Fabric (Porous Base Material Layer))

A rayon knitted fabric was cut to a size of 105 mm × 70 mm to produce a base fabric.

After the following a) and b) were put into 9.5 g (8.64 mL) of DMSO, replacement with nitrogen was performed, and stirring (15 min) was performed at 100°C and 300 rpm, thereby synthesizing the polypeptide derivative in DMSO, and at the same time, preparing a polypeptide derivative solution (polypeptide derivative concentration: 5 wt%) as an impregnation solution.
a) Modified fibroin (PRT17, number average molecular weight of 50,000): 363 mg
b) mal-PEG-mal (number average molecular weight of 20,000): 137 mg (BioChempeg Scientific Inc. HO022022-20K)

A rectangular frame-shaped silicone rubber sheet (thickness: 2 mm) slightly larger than the base fabric was bonded onto a stainless steel plate on which a Teflon sheet was laid, and then the base fabric (rayon) was placed inside the frame-shaped rubber sheet. After the polypeptide derivative solution was dropped on the base fabric, the base fabric was covered with a Teflon sheet, and then leveled to spread the polypeptide derivative solution throughout (air was removed). Thus, an impregnated base fabric was obtained. Thereafter, the impregnated base fabric was placed in a refrigerator at 4°C and gelled (overnight). Ethanol was placed in a container, the gel was put into the container together with the sheet (ethanol replacement), the container was allowed to stand for 3 hours to peel off the silicone sheet, and DMSO was replaced with ethanol. Thereafter, the ethanol in the container was discarded, RO water was added, and the container was allowed to stand for 2 hours to replace the ethanol with water. Next, the gel that had been completely replaced with RO water was frozen in a freezer at -20°C and then dried using a freeze dryer overnight, thereby making the impregnated body of the impregnated base fabric porous (sponged). Thus, a base fabric (impregnated base fabric) including a sponge layer was prepared.

### (Preparation of Skin Layer (Polypeptide Derivative Film))

After the following a) and b) were put into 3.72 g (3.38 mL) of DMSO, replacement with nitrogen was performed, stirring (15 min) was performed at 100°C and 300 rpm, and then the solution was allowed to stand at room temperature for 10 min. Thus, a polypeptide derivative was synthesized in DMSO to prepare a polypeptide derivative solution (polypeptide derivative concentration: 7 wt%) for forming a skin layer.
a) Modified fibroin (PRT17, number average molecular weight of 50,000): 203 mgb) mal-PEG-mal (number average molecular weight of 20,000): 77 mg (BioChempeg Scientific Inc. HO022022-20K)

### (Preparation of Adhesive (Polypeptide Derivative Solution))

After the following a) and b) were put into 3.72 g (3.38 mL) of DMSO, replacement with nitrogen was performed, stirring (15 min) was performed at 100°C and 300 rpm, and then the solution was allowed to stand at room temperature for 10 min. Thus, a polypeptide derivative was synthesized in DMSO to prepare a polypeptide derivative solution (polypeptide derivative concentration: 7 wt%) for forming a skin layer.
a) Modified fibroin (PRT17, number average molecular weight of 50,000): 203 mgb) mal-PEG-mal (number average molecular weight of 20,000): 77 mg (BioChempeg Scientific Inc. HO022022-20K)

### (Preparation of Synthetic Leather)

A target synthetic leather (Example D) was obtained by performing preparation in the same manner as in Example B.

### <Production of Synthetic Leather of Comparative Example A>

Configuration of Synthetic Leather of Comparative Example A
· Base fabric: cotton woven fabric
· Skin layer: modified fibroin film
· Adhesive layer: modified fibroin DMSO solution

### (Preparation of Base Fabric)

A cotton woven fabric was cut to a size of 105 mm × 70 mm to produce a base fabric.

### (Preparation of Skin Layer (Modified Fibroin Film))

After the following a) was put into 4.65 g (4.23 mL) of DMSO, replacement with nitrogen was performed, stirring (15 min) was performed at 100°C and 300 rpm, and then the solution was allowed to stand at room temperature for 10 min. Thus, a modified fibroin DMSO solution (modified fibroin concentration: 7 wt%) for forming a skin layer was prepared.
a) Modified fibroin (PRT17, number average molecular weight of 50,000): 350 mg

The modified fibroin DMSO solution was dropped on release paper attached to a stainless steel plate, smoothed with a doctor blade, and then cast with a width of 80 mm and a thickness of 400 um. The cast modified fibroin DMSO solution was dried in an air blowing oven at 60°C for 30 min. Thus, a modified fibroin film for a skin layer was prepared.

### (Preparation of Adhesive (Modified Fibroin DMSO Solution))

After the following a) was put into 5.58 g (5.07 mL) of DMSO, replacement with nitrogen was performed, stirring (15 min) was performed at 100°C and 300 rpm, and then the solution was allowed to stand at room temperature for 10 min. Thus, a modified fibroin DMSO solution (modified fibroin concentration: 7 wt%) for forming an adhesive layer was prepared.
a) Modified fibroin (PRT17, number average molecular weight of 50,000): 420 mg

### (Preparation of Synthetic Leather)

A frame-shaped silicone rubber sheet having a thickness of 500 um was attached onto an outer peripheral portion of one surface of a film for a skin layer, an adhesive was applied to the inside of the frame-shaped sheet, and then a base fabric was placed thereon. Next, the base fabric was bone dried in an oven at 60°C for 60 min, and then dried in a vacuum oven at 80°C for 12 hours. Thus, a target synthetic leather (Comparative Example A) in which a skin layer was formed on a base fabric with an adhesive layer interposed therebetween was obtained.

### <Evaluation of Flexibility>

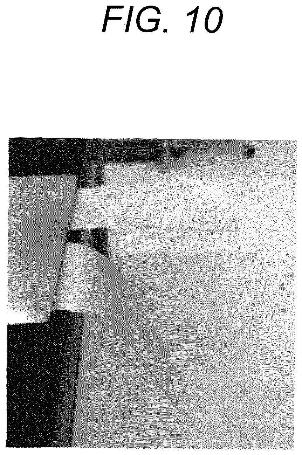

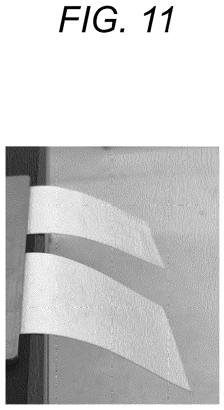

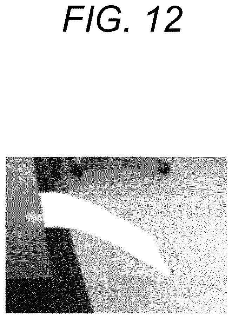

The synthetic leathers of Examples A to D and Comparative Example A were used, each of the synthetic leathers was supported in a cantilever state only at one end in a length direction, and a bending amount due to the own weight at this time was observed. The results are illustrated in Figs. 10, 11, and 12. The upper part of the photograph in Fig. 10 shows Comparative Example A, and the lower part of the photograph shows Example A. The upper part of the photograph in Fig. 11 shows Example D, and the lower part of the photograph shows Example B. Fig. 12 illustrates Example C.

As is clear from Figs. 10, 11, and 12, it is recognized that the bending amount was the largest in Example A, followed by Example C, Example B, and Example D, and the bending amount was the smallest in Comparative Example A. This indicates that the synthetic leather containing the polypeptide derivative has excellent flexibility as compared with the case of not containing the polypeptide derivative. In Examples B, C, and D in which the base fabric was a porous base material layer, a soft feel was obtained when the base fabric was pressed with fingers as compared with Example A and Comparative Example A in which the base fabric was not a porous base material layer. Further, in Example B in which the second segment of the polypeptide derivative contained in the base fabric, the skin layer, and the adhesive layer was composed of mal-PEG-mal and HS-PEG-SH, the flexibility was higher than Example D in which the second segment was composed of only mal-PEG-mal. From this, it can be recognized that higher flexibility is exhibited in the synthetic leather containing a polypeptide derivative composed of the second segment at a higher weight ratio than that of the first segment.

### <Evaluation of Appearance and Bleed-Out During Molding>

### (Example 1)

A polypeptide derivative was prepared using methoxy polyethylene glycol maleimide (manufactured by Sigma-Aldrich Inc.: second segment) having a number average molecular weight of 5,000. Specifically, in a glass container, 2,500 mg of modified fibroin (first segment) having the amino acid sequence (PRT18) set forth in SEQ ID NO: 63 prepared as described above and having a number average molecular weight of 50,000 and 500 mg of the methoxy polyethylene glycol maleimide were weighed, 50 mL of hexafluoroisopropyl alcohol (manufactured by Central Glass Co., Ltd., HFIP) was further added as a solvent, and the mixture was stirred to dissolve the modified fibroin, thereby obtaining a reaction solution. Thereafter, the reaction was performed by stirring the reaction solution at room temperature (25°C) for 20 hours.

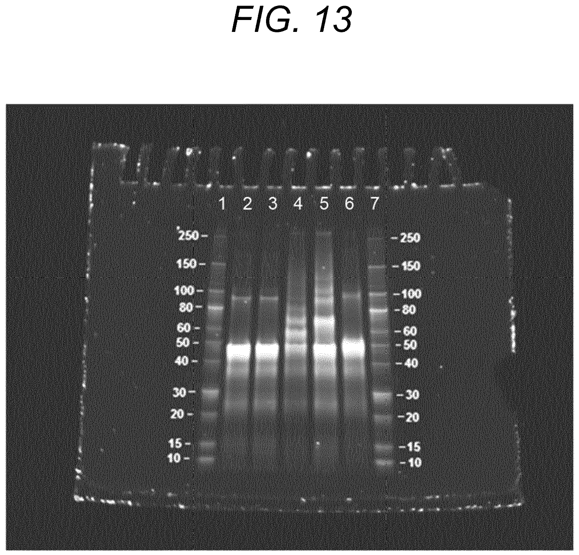

When 20 hours had elapsed, the solvent was distilled off, and the crude product was washed three times with methanol (manufactured by Nacalai Tesque Inc.). 50 mL of methanol was used for one washing. Unreacted products such as methoxy polyethylene glycol maleimide were removed by a washing operation. After the washing operation, drying was performed under reduced pressure to obtain a product (polypeptide derivative). An increase in molecular weight was confirmed by SDS-PAGE for the product, and it was confirmed that a polyethylene glycol chain was introduced into modified fibroin. It was confirmed that 1 to 4 second segments were bonded to one first segment in the obtained polypeptide derivative. Fig. 13 illustrates results of SDS-PAGE. In Fig. 13, Lanes 1 and 7 are standard samples for specifying the molecular weight, Lanes 2 and 3 are modified fibroins having the amino acid sequence (PRT18) set forth in SEQ ID NO: 63, and Lane 4 is the polypeptide derivative prepared in Example 1.

### (Example 2)

A polypeptide derivative was prepared in the same manner as in Example 1, except that methoxy polyethylene glycol maleimide (manufactured by Sigma-Aldrich Inc.) having a number average molecular weight of 10,000 was used, the blending amount of methoxy polyethylene glycol maleimide was 20 mg, the amount of modified fibroin was 50 mg, 1 mL of hexafluoroisopropyl alcohol (manufactured by Central Glass Co., Ltd., HFIP) was added as a solvent, and 1 mL of methanol was used for one washing of the crude product. An increase in molecular weight was confirmed by SDS-PAGE for the product, and it was confirmed that a polyethylene glycol chain was introduced into modified fibroin. It was confirmed that 1 to 4 second segments were bonded to one first segment in the obtained polypeptide derivative. In Fig. 13, Lane 5 is the polypeptide derivative prepared in Example 2.

### (Example 3)

A polypeptide derivative was prepared in the same manner as in Example 2, except that methoxy polyethylene glycol maleimide (manufactured by Sigma-Aldrich Inc.) having a number average molecular weight of 750 was used, and the blending amount of methoxy polyethylene glycol maleimide was 1.5 mg. An increase in molecular weight was confirmed by SDS-PAGE for the product, and it was confirmed that a polyethylene glycol chain was introduced into modified fibroin. It was confirmed that 1 to 4 second segments were bonded to one first segment in the obtained polypeptide derivative. In Fig. 13, Lane 6 is the polypeptide derivative prepared in Example 3.

### (Comparative Example 1)

For comparison, the same experiment was performed using modified fibroin without cysteine. That is, in a glass container, 20 mg of modified fibroin (number average molecular weight: 100,000) having the amino acid sequence (PRT27) set forth in SEQ ID NO: 72 prepared as described above and 4 mg of methoxy polyethylene glycol maleimide (manufactured by Central Glass Co., Ltd., HFIP) having a number average molecular weight of 5,000 were weighed, 1 mL of hexafluoroisopropanol (manufactured by Sigma-Aldrich Inc.) was further added as a solvent, and the mixture was stirred to dissolve the modified fibroin, thereby obtaining a reaction solution. Thereafter, the reaction was performed by stirring the reaction solution at room temperature (25°C) for 20 hours.

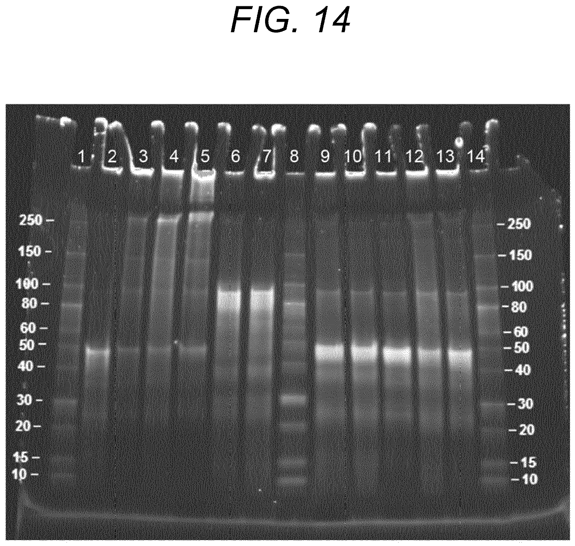

When 20 hours had elapsed, the solvent was distilled off, and the crude product was washed three times with methanol (manufactured by Nacalai Tesque Inc.). 1 mL of methanol was used for one washing. Unreacted products such as methoxy polyethylene glycol maleimide were removed by a washing operation. After the washing operation, drying was performed under reduced pressure to obtain a product. It was confirmed by SDS-PAGE for the product that there was no change in molecular weight, and it was confirmed that the reaction between modified fibroin and methoxy polyethylene glycol maleimide did not proceed. Fig. 14 illustrates results of SDS-PAGE. In Fig. 14, Lane 1 is a standard sample for specifying the molecular weight, Lane 6 is a product, and Lane 7 is the modified fibroin having the amino acid sequence (PRT27) set forth in SEQ ID NO: 72.

### (Comparative Example 2)

A mixture of modified fibroin (number average molecular weight: 100,000) having the amino acid sequence (PRT27) set forth in SEQ ID NO: 72 prepared as described above and polyethylene oxide was prepared. Specifically, 10 g of the modified fibroin and polyethylene oxide (number average molecular weight: 4,000) were blended in a container so as to have a weight of 5 mass% with respect to 100 mass% of the total amount of the modified fibroin, and dry-mixing was performed under a condition of a rotation speed of 1,000 times/min using a bead mill (manufactured by NIPPON COKE & ENG. COMPANY, LIMITD, Attritor) for 10 minutes, thereby preparing a mixture.

### (Comparative Example 3)

A mixture was prepared in the same manner as in Comparative Example 2, except that polyethylene oxide having a number average molecular weight of 20,000 was used as the polyethylene oxide.

### (Comparative Example 4)

A mixture of modified fibroin (number average molecular weight: 100,000) having the amino acid sequence (PRT27) set forth in SEQ ID NO: 72 prepared as described above and polyethylene oxide monostearate (40E.O., number average molecular weight: 2,000) was prepared. Polyethylene oxide monostearate was blended so as to have a weight of 10 mass% with respect to 100 mass% of the total amount of the modified fibroin, mixing was performed in ethanol (99.5%) for 15 hours, and drying was performed in an oven at 60°C for 5 hours, thereby preparing a mixture.

### (Evaluation)

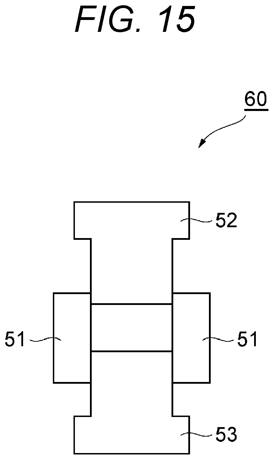

Molded bodies were prepared using the polypeptide derivatives prepared in Examples 1 to 3 and the mixtures prepared in Comparative Examples 2, 3, and 4, respectively, by heat and pressure molding under the following conditions. A pressure molding machine 60 illustrated in Fig. 15 was used for molding. Fig. 15 is a schematic cross-sectional view of the pressure molding machine. The pressure molding machine 60 illustrated in Fig. 15 includes a mold 51 capable of heating in which a through-hole is formed, and an upper pin 52 and a lower pin 53 capable of moving up and down within the through-hole of the mold 51. A sample is introduced into a gap formed by inserting the upper pin 52 or the lower pin 53 into the mold 51, and the composition is compressed by the upper pin 52 and the lower pin 53 while the mold 51 is heated, such that a molded body can be obtained.

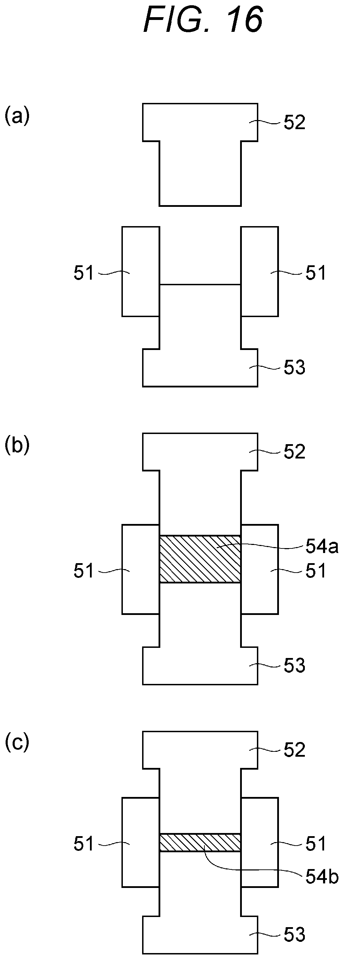

A step of obtaining a molded body will be described with reference to Fig. 16. Fig. 16(a), 16(b), and 16(c) are schematic cross-sectional views of the pressure molding machine before introduction of a composition, after the introduction of the composition, and in a state where the composition is heated and pressurized, respectively. As illustrated in Fig. 16(a), the composition is introduced into the through-hole in a state where only the lower pin 53 is inserted into the through-hole of the mold 51, and as illustrated in Fig. 16(b), the upper pin 52 is inserted into the through-hole of the mold 51 and lowered to start heating the mold 51, and a sample 54a before heating and pressurization is heated and pressurized in the through-hole. The upper pin 52 is lowered until the pressurizing force reaches a predetermined pressurizing force, and heating and pressurization are continued until the sample reaches a predetermined temperature in the state illustrated in Fig. 16(c) to obtain a molded body 54b after heating and pressurization. In some cases, the temperature of the mold 51 may be lowered using a cooler (for example, a spot cooler), and when the molded body 54b reaches a predetermined temperature, the upper pin 52 or the lower pin 53 may be removed from the mold 51 to obtain a molded body. Regarding the pressurization, the upper pin 52 may be lowered in a state where the lower pin 53 is fixed, but both the lowering of the upper pin 52 and the lifting of the lower pin 53 may be performed.

Specifically, first, 0.6 g of the polypeptide derivative or the mixture was weighed and introduced into the through-hole of the mold 51 (in Example 1, a cylindrical mold having a circular through-hole having a diameter of 14 mm was used, and in Comparative Examples 2, 3, and 4, a cylindrical mold having a rectangular through-hole with a cross section of 35 mm × 25 mm was used) of the pressure molding machine 60 illustrated in Fig. 15. At this time, the sample was added so that the thickness was uniform. After all the samples were introduced, heating of the mold 51 was started, and the samples were pressurized by inserting the upper pin 52 and the lower pin 53 into the through-hole using a hand press machine (manufactured by NPa SYSTEM CO., LTD, trade name: NT-100H-V09). At this time, the pressurization condition of the sample was controlled to be 50 MPa. When 5 minutes had elapsed after the temperature of the sample reached 150°C, the heating was stopped, the sample was cooled with a spot cooler (manufactured by TRUSCO NAKAYAMA CORPORATION, trade name: TS-25EP-1), and the sample was taken out when the temperature of the sample reached 50°C, and deburring was performed. Thereafter, a disk-shaped molded body (in Comparative Examples 2, 3, and 4, a rectangular parallelepiped molded body having a length of 35 mm × a width of 25 mm × a thickness of 3.0 mm) having a diameter of 14 mm × a thickness of 3.0 mm was obtained. All the moisture contents in the molding material and the mixture used for molding were 7 to 9 mass% when measured with a heat-drying type moisture meter.

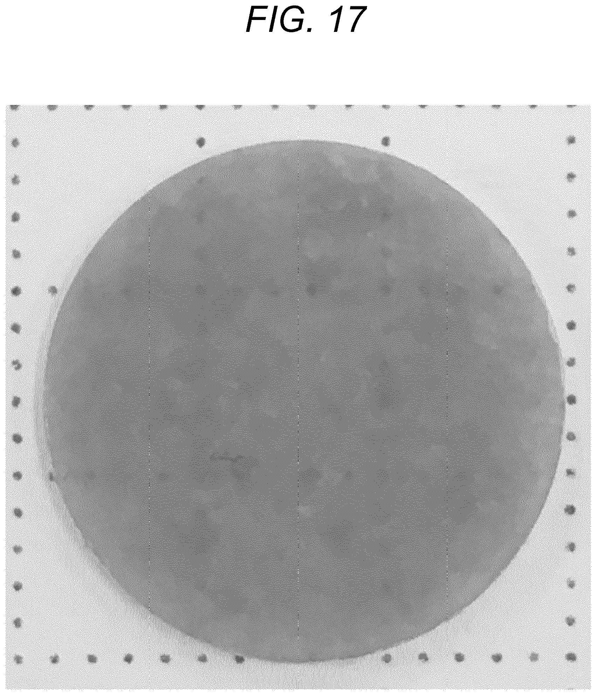

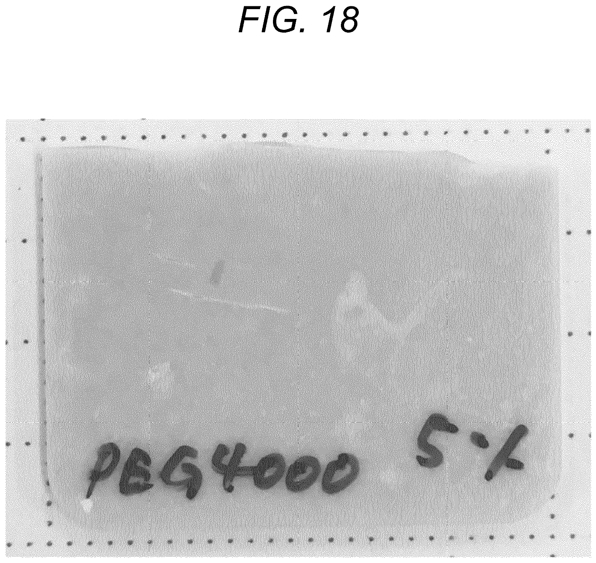

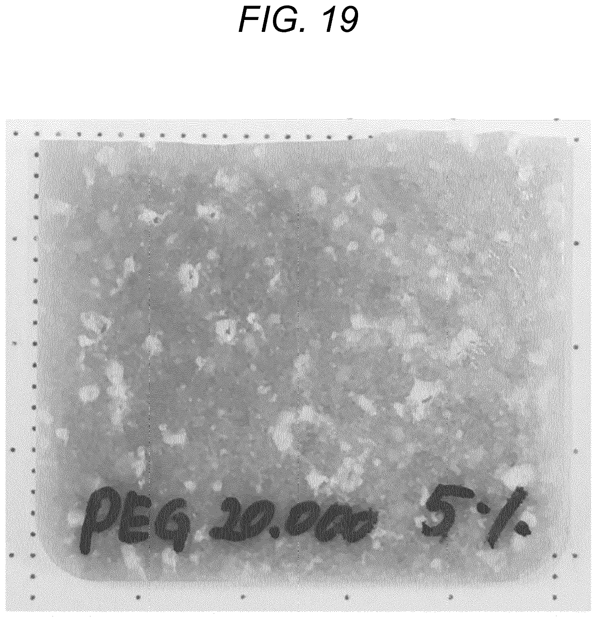

The obtained molded body was subjected to visual observation for appearance and finger touch observation for bleed-out, and was evaluated according to the following criteria. The results are shown in Table 6. The appearance of the molded body is illustrated in each of Figs. 17, 18, and 19.

### [Evaluation Criteria of Appearance]

A: The appearance is uniform.
B: The appearance is uniform, but turbidity is observed.
C: The appearance is not uniform.

### [Evaluation Criteria of Bleed-Out]

A: There is no stickiness.
B: There is stickiness immediately after molding, but there is no stickiness after wiping off.
C: Stickiness remains even after wiping off, or stickiness occurs again.

**[Table 6]**

| | Appearance | Bleed-out |
|---|---|---|
| Example 1 | A | A |
| Example 2 | A | - |
| Example 3 | A | - |
| Comparative Example 2 | B | A |
| Comparative Example 3 | C | A |
| Comparative Example 4 | B | C |

In Table 6, "-" means that measurement is not performed. As can be seen from the results shown in Table 6 and illustrated in Figs. 17, 18, and 19, since the first segment and the second segment were bonded directly to each other, phase separation or the like was not confirmed in Examples 1 to 3. On the other hand, in Comparative Examples 1 to 4, it was confirmed that phase separation and/or bleed-out occurred in the mixture of the modified fibroin and the flexible polymer.

### (Example 4)

A film containing the synthetic polymer prepared in Example 1 and the artificial fibroin (number average molecular weight: 100,000) having the amino acid sequence (PRT27) set forth in SEQ ID NO: 72 prepared as described above was molded. Specifically, 4.4 g of dimethyl sulfoxide was weighed in a container, 0.15 g of the artificial fibroin was weighed therein, and the mixture was stirred under nitrogen under a condition of 120°C for 15 minutes to dissolve the artificial fibroin. 0.15 of the artificial fibroin was further added, and the mixture was stirred under nitrogen under a condition of 120°C for 30 minutes, thereby preparing a solution in which a total of 0.30 g of the artificial fibroin was dissolved. In the solution, 0.15 g of the synthetic polymer was weighed and stirred under nitrogen under a condition of 90°C for 15 minutes to dissolve the synthetic polymer. 0.15 g of the synthetic polymer was further added, and the mixture was stirred under nitrogen under a condition of 90°C for 30 minutes, thereby preparing a dope solution in which 0.30 g of each of the artificial fibroin and the synthetic polymer was dissolved. In the dope solution, a mixing ratio was adjusted so that synthetic polymer : artificial fibroin : dimethyl sulfoxide was 6 mass% : 6 mass% : 88 mass%.

The dope solution was allowed to stand at 80°C for 30 minutes to defoam the dope solution. A film was molded from the dope solution after defoamation using a coater (manufactured by Imoto machinery Co., LTD.). First, a PET film (150 mm × 200 mm) as a release member was placed on a coater, and a liquid film of the dope solution was provided in the form of the PET film under the conditions of an applicator thickness of 0.5 mm, a coating speed of 20 m/min, and a temperature of 40°C. Thereafter, the liquid film of the dope solution was allowed to stand together with the PET film in an air blowing oven, drying was performed under a condition of 100°C for 15 minutes, transfer to a vacuum oven was further performed, vacuum-drying was performed under a condition of 80°C for 15 hours, and absolute drying was performed, thereby preparing a film-shaped molded body (film) formed of a synthetic polymer and artificial fibroin.

The prepared film was clear and colorless, and flexibility was observed. It was confirmed that the film was not cracked even when a bending stress was applied.

### (Example 5)

2,300 mg of the artificial fibroin (number average molecular weight: 50,000, first segment) having the amino acid sequence (PRT17) set forth in SEQ ID NO: 62 prepared as described above and 900 mg of polyethylene glycol bismaleimide (manufactured by BioChempeg Scientific Inc., HO022022-20K, second segment) having a number average molecular weight of 20,000 were dissolved in 115 mL of a solvent (DMSO, manufactured by Kishida Chemical Co., Ltd.) in an eggplant flask, 12 µL of triethylamine (manufactured by Nacalai Tesque Inc.) was added, and a reaction was performed at 80°C for 17 hours while stirring the mixture. 300 mL of ethyl acetate (manufactured by Kanto Chemical Co.) and 300 mL of hexane (manufactured by Kanto Chemical Co.) were added, and the mixture was allowed to stand for 2 hours to precipitate a product. Further, centrifugation (1,000 g × 10 minutes) was performed to remove the supernatant. In order to remove unreacted polyethylene glycol bismaleimide, ethyl acetate was added again, and centrifugation and supernatant removal were repeated three times. Further, drying was performed under reduced pressure to obtain a product. The prepared synthetic polymer has a structure in which a plurality of blocks in which an N-substituted maleimide having a polyethylene glycol chain (corresponding to a second segment) is bonded to a molecular chain terminal of artificial fibroin (corresponding to a first segment) are repeated. That is, the synthetic polymer is a molecule in which three segments are bonded in the order of the second segment, the first segment, and the second segment.

A film containing the synthetic polymer prepared as described above was molded. Specifically, 4.55 g of dimethyl sulfoxide was weighed in a container, 0.45 g of the synthetic polymer was added thereto, and the mixture was stirred under nitrogen under a condition of 90°C for 30 minutes, thereby preparing a dope solution in which the synthetic polymer was dissolved. The stirring rate was set to 100 rpm. In the dope solution, a mixing ratio was adjusted so that synthetic polymer : dimethyl sulfoxide was 9 mass% : 91 mass%.

A film-shaped molded body (film) was prepared in the same manner as in Example 4 using the dope solution. It was confirmed that the prepared film was clear and colorless, flexibility thereof was superior to that of the film of Example 4. It was confirmed that the film was not cracked even when a bending stress was applied.

### (Example 6)

1,424 mg of the artificial fibroin (number average molecular weight: 50,000, first segment) having the amino acid sequence (PRT17) set forth in SEQ ID NO: 62 prepared as described above and 1,076 mg of methoxy polyethylene glycol maleimide (manufactured by BioChempeg Scientific Inc., MF001022-20K, second segment) having a number average molecular weight of 20,000 were dissolved in 71 mL of a solvent (DMSO, manufactured by Kishida Chemical Co., Ltd.) in an eggplant flask, 7.5 µL of triethylamine (manufactured by Nacalai Tesque Inc.) was added, and a reaction was performed at 80°C for 17 hours while stirring the mixture. 210 mL of ethyl acetate (manufactured by Kanto Chemical Co.) and 210 mL of hexane (manufactured by Kanto Chemical Co.) were added, and the mixture was allowed to stand for 17 hours to precipitate a product. Further, centrifugation (1,000 g × 10 minutes) was performed to remove the supernatant. In order to remove unreacted methoxy polyethylene glycol maleimide, ethyl acetate was added again, and centrifugation and supernatant removal were repeated three times. Further, drying was performed under reduced pressure to obtain a synthetic polymer. The prepared synthetic polymer has a structure in which an N-substituted maleimide having a polyethylene glycol chain (corresponding to a second segment) is bonded to both ends of the molecular chain of artificial fibroin (corresponding to a first segment). That is, the synthetic polymer is a molecule in which three segments are bonded in the order of the second segment, the first segment, and the second segment.

A film containing the synthetic polymer prepared as described above was molded. Specifically, 4.30 g of dimethyl sulfoxide was weighed in a container, 0.70 g of the synthetic polymer was added thereto, and the mixture was stirred under nitrogen under a condition of 90°C for 30 minutes, thereby preparing a dope solution in which the synthetic polymer was dissolved. The stirring rate was set to 100 rpm. In the dope solution, a mixing ratio was adjusted so that synthetic polymer : dimethyl sulfoxide was 14 mass% : 86 mass%.

A film-shaped molded body (film) was prepared in the same manner as in Example 4 using the dope solution. It was confirmed that the prepared film was clear and colorless, flexibility thereof was superior to that of the film of Example 4. It was confirmed that the film was not cracked even when a bending stress was applied.

### (Example 7)

Polyethylene glycol (manufactured by BioChempeg scientific Inc., number average molecular weight: 20,000) having maleimide groups at both ends was reacted with artificial fibroin having two cysteine residues to prepare a synthetic polymer. Specifically, 4.022 g of artificial fibroin (number average molecular weight: 50,000) having the amino acid sequence (PRT17) set forth in SEQ ID NO: 62 prepared as described above, 1.564 g of polyethylene glycol having maleimide groups at both ends, and 201 mL of dimethyl sulfoxide as a solvent were weighed in an eggplant flask, and the mixture was stirred at 80°C for 48 hours to be dissolved and react, thereby obtaining a solution of a synthetic polymer. To the obtained solution, 603 mL of ethyl acetate and 603 mL of hexane were added, the mixture was stirred for 2 hours, centrifugation was performed, and then washing was further performed three times with ethyl acetate. Thereafter, drying was performed with an evaporator, and finally, vacuum drying was performed at room temperature to obtain a powder of a synthetic polymer.

### (Example 8)

Polyethylene glycol (manufactured by BioChempeg scientific Inc., number average molecular weight: 20,000) having maleimide groups at both ends was reacted with artificial fibroin having two cysteine residues to prepare a synthetic polymer. Specifically, 348 mg of artificial fibroin (number average molecular weight: 50,000) having the amino acid sequence (PRT17) set forth in SEQ ID NO: 62 prepared as described above was weighed in a stainless steel container, 2.11 mL of dimethyl sulfoxide was added as a solvent, and the mixture was stirred and dissolved, thereby obtaining a fibroin solution. 132 mg of polyethylene glycol having maleimide groups at both ends was weighed in a glass container, 1.09 mL of dimethyl sulfoxide was added as a solvent, and the mixture was stirred and dissolved, thereby obtaining a polyethylene glycol solution. The obtained fibroin solution and polyethylene glycol solution were mixed while stirring the mixture, the mixture was reacted at 100°C for 5 minutes while stirring the mixture, and the mixture was further allowed to stand and react at 100°C for 10 minutes, thereby preparing a synthetic polymer. In Example 8, the synthetic polymer was subjected to film molding described below without being dried in a state in which the synthetic polymer was dissolved in dimethyl sulfoxide.

### (Example 9)

Polyethylene glycol (manufactured by BioChempeg scientific Inc., number average molecular weight: 20,000) having maleimide groups at both ends was reacted with artificial fibroin having two cysteine residues and artificial fibroin having four cysteine residues to prepare a synthetic polymer. Specifically, 336 mg of artificial fibroin (number average molecular weight: 50,000, the total number of cysteine residues: 2) having the amino acid sequence (PRT17) set forth in SEQ ID NO: 62 prepared as described above, 44 mg of artificial fibroin (number average molecular weight: 53,100, the total number of cysteine residues: 4) having the amino acid sequence (PRT18) set forth in SEQ ID NO: 63 prepared as described above, and 160 mg of polyethylene glycol having maleimide groups at both ends were weighed in a glass container, 4.42 mL of dimethyl sulfoxide was added as a solvent, and the components described above were dissolved under stirring, thereby obtaining a reaction solution. The obtained reaction solution was allowed to react under stirring at 100°C for 10 minutes, and then was allowed to stand and react at 100°C for 5 minutes, thereby preparing a synthetic polymer. In Example 9, the synthetic polymer was subjected to film molding described below without being dried in a state in which the synthetic polymer was dissolved in dimethyl sulfoxide.

### (Example 10)

Polyethylene glycol (manufactured by BioChempeg scientific Inc., number average molecular weight: 20,000) having maleimide groups at both ends was reacted with 2,2'-(ethylenedioxy)diethanethiol having two thiol groups and artificial fibroin having two cysteine residues to prepare a synthetic polymer. Specifically, 300 mg of artificial fibroin (number average molecular weight: 50,000) having the amino acid sequence (PRT17) set forth in SEQ ID NO: 62 prepared as described above and 284 mg of polyethylene glycol having maleimide groups at both ends were weighed in a glass container, 3.42 mL of dimethyl sulfoxide was added as a solvent, and the mixture was stirred to dissolve the respective components, thereby obtaining a reaction solution. The obtained reaction solution was stirred and allowed to react at 100°C for 15 minutes, 1.55 mg of 2,2'-(ethylenedioxy)diethanethiol was further added, and the mixture was stirred at 100°C for 10 minutes to react, thereby preparing a synthetic polymer. In Example 10, the synthetic polymer was subjected to film molding described below without being dried in a state in which the synthetic polymer was dissolved in dimethyl sulfoxide.

### (Example 11)

Polyethylene glycol (manufactured by BioChempeg scientific Inc., number average molecular weight: 1,000) having maleimide groups at both ends was reacted with polyethylene glycol (manufactured by BioChempeg scientific Inc., number average molecular weight: 2,000) (that is, having thiol groups at both ends) having cysteine residues at both ends and artificial fibroin having two cysteine residues, thereby preparing a synthetic polymer. Specifically, 378 mg of artificial fibroin (number average molecular weight: 50,000) having the amino acid sequence (PRT17) set forth in SEQ ID NO: 62 prepared as described above, 79 mg of polyethylene glycol having maleimide groups at both terminals, and 143 mg of polyethylene glycol having cysteine residues at both ends were weighed in a vial bottle, 4.40 mL of dimethyl sulfoxide was added as a solvent, and the mixture was stirred and dissolved, thereby obtaining a reaction solution. The reaction solution was allowed to react under stirring at 100°C for 10 minutes, and was further allowed to stand and react at 100°C for 10 minutes, thereby preparing a synthetic polymer. In Example 11, the synthetic polymer was subjected to film molding described below without being dried in a state in which the synthetic polymer was dissolved in dimethyl sulfoxide.

### (Comparative Example 5)

For comparison with the synthetic polymers prepared in Examples 7 to 11, a similar operation was performed using artificial fibroin (molecular weight: 100,000) having the amino acid sequence (PRT27) set forth in SEQ ID NO: 72 prepared as described above. Specifically, 650 mg of artificial fibroin (molecular weight: 100,000) having the amino acid sequence (PRT27) set forth in SEQ ID NO: 72 prepared as described above was weighed in a stainless steel container, 3.955 mL of dimethyl sulfoxide was added as a solvent, and the mixture was stirred, thereby obtaining a dispersion. The obtained dispersion was stirred and dissolved at 120°C for 30 minutes, and then allowed to stand at 100°C for 10 minutes. In Comparative Example 5, the artificial fibroin dissolved in dimethyl sulfoxide was subjected to film molding as described below without drying.

### <Evaluation of Molded Body>

Film-shaped molded bodies were prepared under the following conditions using the synthetic polymers prepared in Examples 7 to 11, respectively. As for the synthetic polymer prepared in Example 7, first, 530 mg of a powder of the synthetic polymer was added to 3.53 mL of dimethyl sulfoxide, and the mixture was stirred at 90°C for 40 minutes to dissolve the powder, thereby preparing a dope solution. Next, the dope solution was allowed to stand at 80°C for 30 minutes, and the defoamed solution was subjected to film molding. The synthetic polymers prepared in Examples 8 to 11 were subjected to film molding while being dissolved in dimethyl sulfoxide as dope solutions without drying. The artificial fibroin of Comparative Example 5 was similarly subjected to film molding as a dope solution while being dissolved in dimethyl sulfoxide without drying.

First, a PET film (150 mm × 200 mm) was prepared as a release member, and a liquid film of the dope solution was provided on the PET film so as to have a thickness of 0.4 mm with an applicator. Thereafter, the liquid film was allowed to stand together with the PET film in an air blowing oven, drying was performed under a condition of 60°C for 30 minutes, transfer to a vacuum oven was further performed, vacuum-drying was performed under a condition of 80°C for 15 hours, and absolute drying was performed, thereby preparing a film-shaped molded body (film) formed of a synthetic polymer or artificial fibroin.

Each of the prepared films was subjected to a tensile test to evaluate the elastic modulus, tensile strength, elongation at break, and toughness. The results are shown in Table 7. Table 7 also shows the thickness of the film in the absolute dry state. The elongation at break shown in Table 7 was expressed as a relative value based on the results obtained in Comparative Example 5.

**[Table 7]**

| | Film thickness [µm] | Elastic modulus | Elongation at break | Toughness |
|---|---|---|---|---|
| Example 7 | 26 | 73 | 1292 | 639 |
| Example 8 | 27 | 76 | 5400 | 2927 |
| Example 9 | 22 | 63 | 8520 | 3120 |
| Example 10 | 28 | 24 | 22332 | 7190 |
| Example 11 | 59 | 38 | 436 | 217 |
| Comparative Example 5 | 49 | 100 | 100 | 100 |

As shown in Table 7, it can be seen that in the films obtained in Examples 7 to 11, the elongation at break was extremely large and the elastic modulus was suppressed to be low in comparison to the film obtained in Comparative Example 5. It can be confirmed from this that the films obtained in Examples 7 to 11 have excellent flexibility. In addition, the films obtained in Examples 7 to 11 have sufficiently excellent toughness.

From the results of Examples 7 and 8, it was confirmed that a film having an excellent elastic modulus, elongation at break, and toughness was prepared by directly molding a film from a solution after reaction rather than preparing a film after once isolating a synthetic polymer as a powder and dissolving the synthetic polymer again. From the results of Examples 8, 10, and 11, it was confirmed that a film having flexibility to be obtained was prepared by preparing a second segment having flexibility, and the tendency of the flexibility became remarkable by lengthening the second segment.

### (Example 12)

2,177 mg of the artificial fibroin (number average molecular weight: 50,000, first segment) having the amino acid sequence (PRT17) set forth in SEQ ID NO: 62 prepared as described above and 823 mg of polyethylene glycol bismaleimide (manufactured by BioChempeg Scientific Inc., HO022022-20K: second segment) having a number average molecular weight of 20,000 were dissolved in 12,000 mg of a dimethyl sulfoxide solution (DMSO, manufactured by Kishida Chemical Co., Ltd.) having a lithium chloride concentration of 2 M, and the mixture was stirred under nitrogen at 100°C for 4 hours to react, thereby preparing a dope solution in which the synthetic polymer was dissolved. The stirring rate was set to 100 rpm. In the dope solution, a mixing ratio was adjusted so that synthetic polymer : dimethyl sulfoxide was 20 mass% : 80 mass%. A viscosity of the obtained dope at 100°C was 10,900 mPa·s. The synthetic polymer in the prepared dope solution has a structure in which a plurality of blocks in which an N-substituted maleimide having a polyethylene glycol chain (corresponding to a second segment) is bonded to a molecular chain terminal of artificial fibroin (corresponding to a first segment) are repeated.

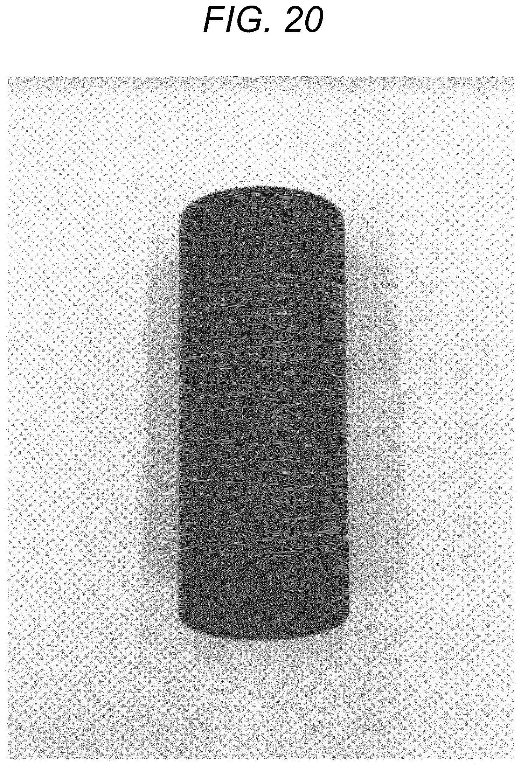

Fibers were prepared by dry-wet spinning under the following conditions using the dope solution. The dope solution at 100°C was extruded together with nitrogen gas from a nozzle (nozzle diameter: 0.1 mm) by a gear pump, passed through a first coagulation bath (first bath), a second coagulation bath (second bath), and a stretching bath (third bath), and then wound up by a heating type roller set at 60°C. A stretch ratio in the stretching bath was set to 8.5 times. The first bath was a methanol bath at 0°C, the second bath was a methanol bath at 25°C, and the third bath was a water bath at 54°C. It was confirmed that the obtained fibers had sufficient stretchability. The appearance of the obtained fibers is illustrated in Fig. 20.

### <Reference Example 1: Evaluation of Hydrophobicity (Solubility) of Structural Protein>

The solubility in a 9 M lithium bromide aqueous solution of each of the modified fibroin and silk fibroin (value of average hydropathy index: 0.21) was evaluated. As the silk fibroin, "Silk Powder IM" manufactured by KB SEIREN, LTD. was used. As the modified fibroin, modified fibroin (number average molecular weight: 50,000) having the amino acid sequence (PRT17) set forth in SEQ ID NO: 62 prepared as described above and modified fibroin (number average molecular weight: 100,000) having the amino acid sequence (PRT27) set forth in SEQ ID NO: 72 prepared as described above were used. Specifically, the solubility was evaluated by adding predetermined amounts of silk fibroin and modified fibroin to a 9 M lithium bromide aqueous solution, respectively, and stirring the mixture at 60°C for 30 minutes, and then the appearance of the aqueous solution was observed. The results are shown in Table 7. In Table 7, "A" indicates that the added fibroin was completely dissolved and a solution having a set concentration could be prepared, and "B" indicates that a residue of fibroin was observed. That is, it was confirmed that modified fibroin was highly hydrophobic fibroin that was not dissolved in an amount equivalent to 5 mass% even in a 9 M lithium bromide aqueous solution at 60°C.

**[Table 8]**

| | | Set value of fibroin concentration [mass%] | | | | | | | | |
|---|---|---|---|---|---|---|---|---|---|---|
| | | 1 | 5 | 20 | 30 | 31 | 32 | 33 | 34 | 35 |
| Silk fibroin | | - | A | A | A | B | B | B | B | B |
| Artificial fibroin | PRT17 | B | B | - | B | - | - | - | - | - |
| | PRT27 | - | B | - | B | - | - | - | - | - |

### <Reference Example 2: Evaluation of Hydrophobicity (Hot Water Resistance) of Structural Protein>

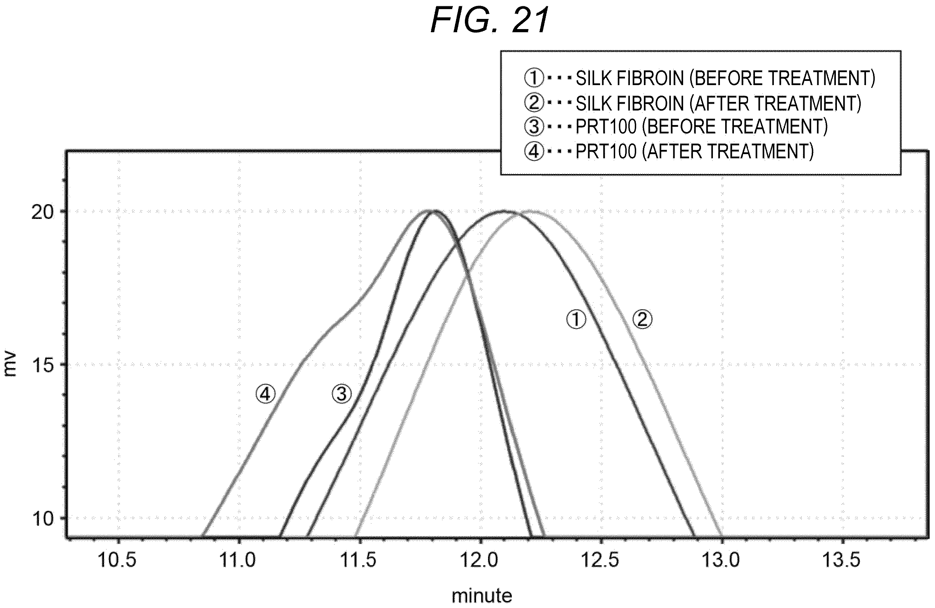

The modified fibroin and silk fibroin were subjected to a hot water resistance test to evaluate the hot water resistance. As the silk fibroin, "Silk Powder IM" manufactured by KB SEIREN, LTD. was used. As the modified fibroin, modified fibroin (number average molecular weight: 50,000) having the amino acid sequence (PRT17) set forth in SEQ ID NO: 62 prepared as described above was used. A dispersion was prepared by adding 50 mg of fibroin and 950 mg of RO water as a solvent to a glass container so that the content of fibroin was 5 mass%. The dispersion was stirred at 100°C for 5 hours. After 5 hours had elapsed, the solvent was removed by centrifugation. The solvent was removed and then washed twice with acetone, and the acetone was removed by centrifugation. After the washing operation, drying was performed under reduced pressure to obtain a processed powder. The degree of decomposition was confirmed by GPC measurement for the processed powder. The results of GPC are illustrated in Fig. 21. As is apparent from Fig. 21, a decrease in molecular weight of silk fibroin was confirmed as compared with that before processing, and such a decrease in molecular weight was not observed in the modified fibroin. From this result, it is presumed that the modified fibroin had lower hydrophilicity to RO water than the silk fibroin. In other words, it can be said that the modified fibroin has high hydrophobicity. In Fig. 21, the results of the modified fibroin (number average molecular weight: 50,000) having the amino acid sequence (PRT17) set forth in SEQ ID NO: 62 are referred to as PRT100.

### <Reference Example 3: Evaluation of Hydrophobicity (Contact Angle) of Structural Protein>

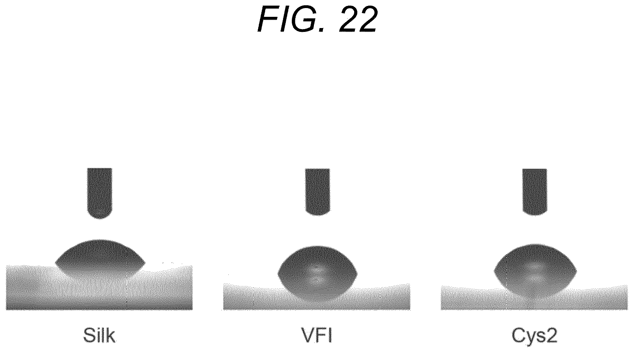

For modified fibroin and silk fibroin, the contact angle with respect to water was measured and evaluated. As the silk fibroin, "Silk Powder IM" manufactured by KB SEIREN, LTD. was used. As the modified fibroin, modified fibroin (number average molecular weight: 50,000) having the amino acid sequence (PRT17) set forth in SEQ ID NO: 62 prepared as described above and modified fibroin (number average molecular weight: 100,000) having the amino acid sequence (PRT27) set forth in SEQ ID NO: 72 prepared as described above were used. Specifically, for the measurement of the contact angle, a dispersion was prepared by first adding 30 mg of fibroin and 970 mg of dimethyl sulfoxide as a solvent to a glass container so that a content of the fibroin was 3 mass%. The dispersion was stirred at 100°C for 15 minutes to prepare a dimethyl sulfoxide solution. Next, a masking tape having a thickness of 100 um was attached onto the slide glass to form a shim, and the dimethyl sulfoxide solution prepared as described above was cast to form a liquid film. Thereafter, each slide glass was transferred into a vacuum oven (manufactured by TOKYO RIKAKIKI CO, LTD., product name: VOS-210C), allowed to stand, and dried under reduced pressure at 80°C to form a fibroin film. 2 µL of RO water was added dropwise to the film, and the contact angle of water after 5 seconds was measured using a contact angle meter (manufactured by Kyowa Interface Science Co., Ltd., product name: DMs-061). The measurement was performed on 6 samples for each fibroin, and the average value thereof was used for evaluation. The results are shown in Table 8 and are illustrated in Fig. 22. As shown in Table 8, it was confirmed that the modified fibroin had a larger water contact angle and higher hydrophobicity than the silk fibroin. In Fig. 22, the results for silk fibroin are shown on the left, the results for modified fibroin having the amino acid sequence (PRT17) set forth in SEQ ID NO: 62 are shown on the right, and the results for modified fibroin having the amino acid sequence (PRT27) set forth in SEQ ID NO: 72 are shown in the center.

**[Table 9]**

| | | Water contact angle [°] (average value) |
|---|---|---|
| Silk fibroin | | 54.9 |
| Artificial fibroin | PRT17 | 70.0 |
| | PRT27 | 72.4 |

### <Reference Example 4: Structural Evaluation of Polypeptide Derivative>

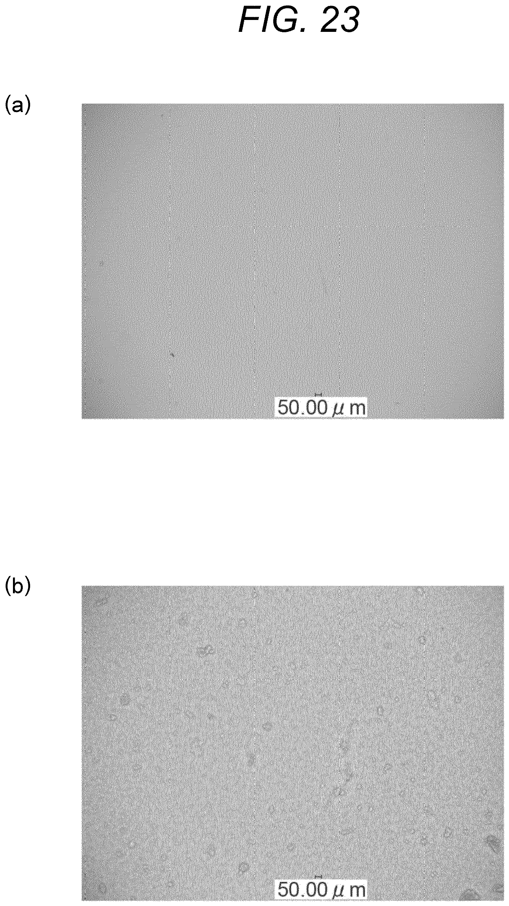

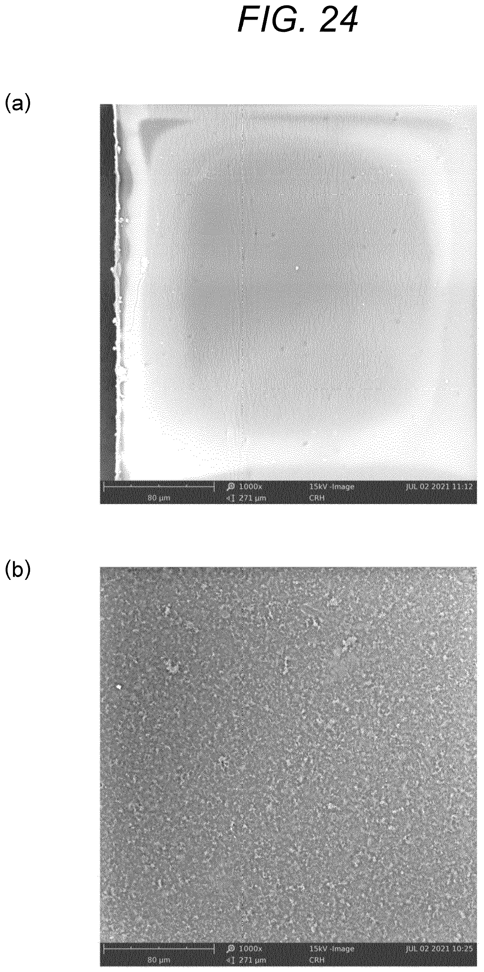

The following two types of cast films (polypeptide derivative film and modified fibroin + PEG mixture film) were prepared. Figs. 23 and 24 illustrate the results of 100-fold observation with an optical microscope and the results of 1,000-fold observation with a scanning electron microscope for the two types of cast films, respectively.

Figs. 23(a) and 23(b) are optical micrographs showing a polypeptide derivative film and a modified fibroin + PEG mixture film enlarged at a magnification of 100 times, respectively. Figs. 24(a) and 24(b) are scanning electron micrographs showing a polypeptide derivative film and a modified fibroin + PEG mixture film enlarged at a magnification of 1,000 times, respectively.

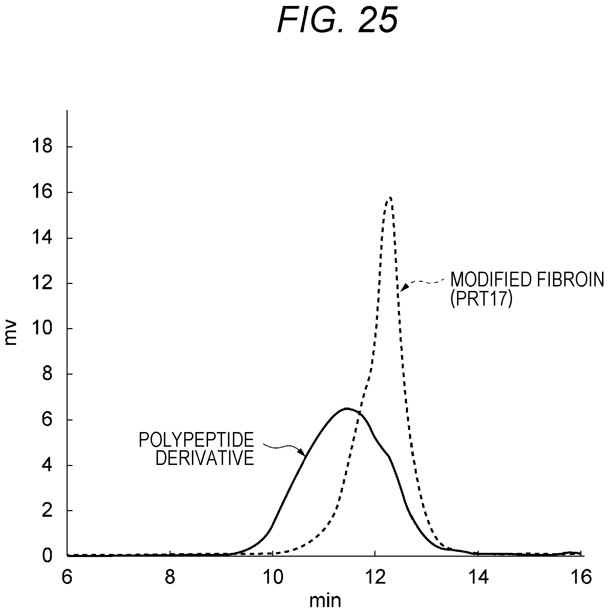

The GPC charts of the polypeptide derivative and the modified fibroin (PRT17, number average molecular weight of 50,000) as a raw material thereof are illustrated in Fig. 25.

From Figs. 23 to 25, it was confirmed that a clear phase separation structure was not confirmed in the polypeptide derivative film by observation with the microscope, and as a result of chemical bonding between fibroin and PEG, an increase in molecular weight was observed.

### (Preparation of Polypeptide Derivative Film)

After the following a) and b) were put into 0.92 g (0.84 mL) of dimethyl sulfoxide (DMSO), replacement with nitrogen was performed, stirring (15 minutes) was performed at 100°C and 300 rpm, and then the solution was allowed to stand at room temperature for 10 minutes. Thus, a polypeptide derivative was synthesized in DMSO to prepare a polypeptide derivative solution (polypeptide derivative concentration: 8 wt%).
a) Modified fibroin (PRT17, number average molecular weight of 50,000): 58 mg
b) mal-PEG-mal (number average molecular weight of 20,000): 22 mg (BioChempeg Scientific Inc. HO022022-20K)

The polypeptide derivative solution was dropped on release paper attached to a stainless steel plate, smoothed with a doctor blade, and then cast with a width of 80 mm and a thickness of 400 um. Thereafter, the cast polypeptide derivative solution was dried in an air blowing oven at 60°C for 30 minutes, and then vacuum drying was performed at 80°C for 15 hours. Thus, a polypeptide derivative film was prepared.

### (Preparation of Modified Fibroin + PEG Mixture Film)

A modified fibroin + PEG mixture film was prepared in the same manner as in the polypeptide derivative film described above, except that the following a) and b) were put into 0.92 g (0.84 mL) of dimethyl sulfoxide (DMSO) to adjust a mixed solution.
a) Modified fibroin (PRT27, number average molecular weight of 100,000): 58 mg
b) PEG (number average molecular weight 20,000): 22 mg (Wako Pure Chemical Industries, Ltd.)

### Industrial Applicability

According to the present disclosure, it is possible to provide a synthetic leather having excellent flexibility while containing a material containing a polypeptide skeleton. According to the present disclosure, it is possible to adjust the physical properties and function of the polypeptide derivative by selecting the second segment to be introduced into the first segment. As a result, the physical properties and function of the synthetic leather prepared using the polypeptide derivative can be adjusted.

### Reference Signs List

- 1: Skin layer
- 2: Base material layer
- 3: Adhesive layer
- 4: Porous layer
- 10, 20, 30, 40: Synthetic leather
- 51: Mold
- 52: Upper pin
- 53: Lower pin
- 54a: Sample
- 54b: Molded body
- 60: Pressure molding machine

### [Sequence Listing]

International application FP22-0822-Synthetic leather and method for producing the same JP22029657 20220802-01530476352201772639 Normal
20220802141929202207261716390140_P1AP101_FP_127.xml under the International Patent Cooperation Treaty

## Claims

1. A synthetic leather comprising a base material layer containing a fiber base material,
wherein at least a part of the synthetic leather contains a polypeptide derivative,
the polypeptide derivative contains a block copolymer containing
a first segment containing a polypeptide skeleton, and
one or a plurality of second segments that are bonded to the first segment,
the polypeptide skeleton is a recombinant polypeptide skeleton, and
the second segment contains a molecular group having a plasticizing function for the polypeptide skeleton.

2. A synthetic leather comprising a base material layer containing a fiber base material,
wherein at least a part of the synthetic leather contains a polypeptide derivative,
the polypeptide derivative contains a block copolymer containing
a first segment containing a polypeptide skeleton, and
one or a plurality of second segments that are bonded to the first segment,
the polypeptide skeleton is a hydrophobic polypeptide skeleton, and
the second segment contains a molecular group having a plasticizing function for the polypeptide skeleton.

3. The synthetic leather according to claim 1 or 2, wherein a molecular weight of the molecular group having the plasticizing function for the polypeptide skeleton is 10 or more when a molecular weight of the polypeptide skeleton is 100.

4. The synthetic leather according to claim 3, wherein the molecular weight of the molecular group having the plasticizing function for the polypeptide skeleton is 100 or less when the molecular weight of the polypeptide skeleton is 100.

5. The synthetic leather according to claim 1 or 2, wherein a total molecular weight of the second segment is 10 or more based on a molecular weight of the first segment being 100.

6. The synthetic leather according to claim 5, wherein the total molecular weight of the second segment is 2,000 or less based on the molecular weight of the first segment being 100.

7. The synthetic leather according to any one of claims 1 to 6, wherein the molecular group having the plasticizing function for the polypeptide skeleton is polyether, polyester, or polycarbonate.

8. The synthetic leather according to claim 7, wherein the polyether is polyethylene glycol.

9. The synthetic leather according to any one of claims 1 to 8, wherein the polypeptide skeleton is a hydrophobic recombinant polypeptide skeleton.

10. The synthetic leather according to any one of claims 1 to 9, wherein an average hydropathy index of the polypeptide skeleton is 0.22 or more.

11. The synthetic leather according to any one of claims 1 to 10, wherein the polypeptide skeleton includes a skeleton derived from a recombinant protein.

12. The synthetic leather according to claim 11, wherein the recombinant protein includes a recombinant structural protein.

13. The synthetic leather according to claim 12, wherein the recombinant structural protein includes modified fibroin.

14. The synthetic leather according to claim 13, wherein the modified fibroin includes modified spider silk fibroin.

15. The synthetic leather according to any one of claims 1 to 14, wherein the synthetic leather includes at least one of a skin layer bonded to the base material layer, an adhesive layer bonded to the base material layer and the skin layer, and a polymeric substance contained in the base material layer and impregnated into the fiber base material, and
at least one of the fiber base material, the skin layer, the adhesive layer, and the polymeric substance contains the polypeptide derivative.

16. The synthetic leather according to any one of claims 1 to 15, wherein the synthetic leather includes a porous layer.

17. The synthetic leather according to claim 16, wherein the porous layer contains the polypeptide derivative.

18. The synthetic leather according to claim 17, wherein the base material layer is the porous layer.

19. The synthetic leather according to any one of claims 1 to 18, wherein the first segment is bonded to the second segment by at least one functional group selected from the group consisting of a thiol group, an amino group, and a hydroxy group in the polypeptide skeleton.

20. A method for producing a synthetic leather including a base material layer containing a fiber base material, the method comprising:
(1) a step of forming a skin layer on the base material layer with an adhesive layer interposed therebetween, in which at least one of the base material layer, the adhesive layer, and the skin layer contains a polypeptide derivative;
(2) a step of forming a skin layer on the base material layer without interposing an adhesive layer, in which at least one of the base material layer and the skin layer contains a polypeptide derivative; or
(3) a step of forming the base material layer containing the fiber base material and the polymeric substance impregnated into the fiber base material by impregnating the fiber base material with an impregnation solution containing a polymeric substance and then solidifying the polymeric substance, in which at least one of the fiber base material and the polymeric substance contains a polypeptide derivative,
wherein the polypeptide derivative contains a block copolymer containing
a first segment containing a polypeptide skeleton, and
one or a plurality of second segments that are bonded to the first segment,
the polypeptide skeleton is a recombinant polypeptide skeleton, and
the second segment contains a molecular group having a plasticizing function for the polypeptide skeleton.

21. A method for producing a synthetic leather including a base material layer containing a fiber base material, the method comprising:
(1) a step of forming a skin layer on the base material layer with an adhesive layer interposed therebetween, in which at least one of the base material layer, the adhesive layer, and the skin layer contains a polypeptide derivative;
(2) a step of forming a skin layer on the base material layer without interposing an adhesive layer, in which at least one of the base material layer and the skin layer contains a polypeptide derivative; or
(3) a step of forming the base material layer containing the fiber base material and the polymeric substance impregnated into the fiber base material by impregnating the fiber base material with an impregnation solution containing a polymeric substance and then solidifying the polymeric substance, in which at least one of the fiber base material and the polymeric substance contains a polypeptide derivative,
wherein the polypeptide derivative contains a block copolymer containing
a first segment containing a polypeptide skeleton, and
one or a plurality of second segments that are bonded to the first segment,
the polypeptide skeleton is a hydrophobic polypeptide skeleton, and
the second segment contains a molecular group having a plasticizing function for the polypeptide skeleton.

22. A synthetic leather comprising:
a base material layer containing a fiber base material; and
at least one of a skin layer bonded to the base material layer, an adhesive layer bonded to the base material layer and the skin layer, and a polymeric substance contained in the base material layer and impregnated into the fiber base material,
wherein at least one of the fiber base material, the skin layer, the adhesive layer, and the polymeric substance contains a recombinant polypeptide derivative.

23. A synthetic leather comprising:
a base material layer containing a fiber base material; and
at least one of a skin layer bonded to the base material layer, an adhesive layer bonded to the base material layer and the skin layer, and a polymeric substance contained in the base material layer and impregnated into the fiber base material,
wherein at least one of the fiber base material, the skin layer, the adhesive layer, and the polymeric substance contains a hydrophobic polypeptide derivative.
